(19) Europäisches Patentamt
European Patent Office
Office européen des brevets

(11) EP 0 883 624 B1

(12) **EUROPÄISCHE PATENTSCHRIFT**

(45) Veröffentlichungstag und Bekanntmachung des Hinweises auf die Patenterteilung:
**06.11.2002 Patentblatt 2002/45**

(21) Anmeldenummer: **97905135.6**

(22) Anmeldetag: **28.02.1997**

(51) Int Cl.[7]: **C07H 15/04**, C07H 15/08, C07H 15/26, C07F 9/10, A61K 9/127

(86) Internationale Anmeldenummer:
**PCT/EP97/01011**

(87) Internationale Veröffentlichungsnummer:
**WO 97/031927 (04.09.1997 Gazette 1997/38)**

(54) **TETRAETHERLIPIDE UND DIESE ENTHALTENDE LIPOSOMEN SOWIE DEREN VERWENDUNG**

TETRAETHER LIPIDS AND LIPOSOMES CONTAINING SAID LIPIDS, AND USE OF THE SAME

TETRAETHER LIPIDES ET LIPOSOMES LES CONTENANT, ET UTILISATION DE CEUX-CI

(84) Benannte Vertragsstaaten:
**AT DE FR GB IT NL**

(30) Priorität: **29.02.1996 DE 19607722**

(43) Veröffentlichungstag der Anmeldung:
**16.12.1998 Patentblatt 1998/51**

(73) Patentinhaber: **Humatrix AG**
**60488 Frankfurt/Main (DE)**

(72) Erfinder:
- **FREISLEBEN, H.-J.**
  **D-64390 Erzhausen (DE)**
- **ANTONOPOULOS, Emmanouil**
  **D-63263 Neu-Isenburg (DE)**
- **BAKOWSKY, Udo**
  **D-99817 Eisenach (DE)**
- **ROTHE, Ulrich**
  **D-06193 Frössnitz (DE)**

(74) Vertreter:
**Meyer-Dulheuer, Karl-Hermann, Dr. et al**
**Patentanwalt,**
**Metzlerstrasse 27**
**60594 Frankfurt am Main (DE)**

(56) Entgegenhaltungen:
**WO-A-93/08202**

- **Biochimica et Biophysica Acta, Band 778, 1984, Detlef Blöcher et al, "Physicochemical Characterization of Tetraether Lipids from Thermoplastma Acidophilum"**
- **Biochimica et Biophysica Acta, Band 664, 1981, S.C. Kushwaha et al, "Novel Polar Lipids from the Methanogen Methanospirillum Hungatei GP1"**
- **Biochimica et Biophysica Acta, Band 487, 1977, Thomas A. Langworthy, "Long-Chain Diglycerol Tetraethers from Thermoplasma Acidophilum"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Tetraetherlipid, ein Verfahren zum Herstellen desselben und seine Verwendung sowie das erfindungsgemäße Tetraetherlipid enthaltende Liposomen und deren Verwendung.

Liposomen sind normalerweise aus Doppelschicht-bildenden Phospholipiden zusammengesetzte Lipidvesikel, die einen wäßrigen Innenraum umschließen. Sie haben in den letzten Jahren zum Beispiel als Modell für Untersuchungen zur Membranstruktur und -funktion gesteigertes Interesse hervorgerufen. Ihr Aufbau aus wäßrigem Innenraum und äußerer Lipidschicht macht sie außerdem als Transportvehikel für hydrophile und lipophile Substanzen interessant. Dementsprechend wird die Verwendung von Liposomen als Träger für Wirkstoffe, z.B. Pharmazeutika, diskutiert und teilweise auch schon praktiziert.

Zur Herstellung von Liposomen werden hauptsächlich natürliche Lecithine aus Sojabohnen oder Eigelb bzw. definierte natürliche oder künstliche Phospholipide wie Cardiolipin, Sphingomyelin, Lysolecithin und andere verwendet. Durch Variation der polaren Kopfgruppen (Cholin, Ethanolamin, Serin, Glycerin, Inosit), der Länge und der Sättigungsgrade der Kohlenwasserstoffketten werden Größe, Stabilität und Fähigkeit zur Aufnahme und Freisetzung von Arzneistoffen beeinflußt.

Ein wesentlicher Nachteil herkömmlicher Liposomen ist jedoch ihre Instabilität. Aus normalen Doppelschicht-bildenden Phospholipiden gebildete Liposomen sind auch im gekühlten Zustand nur kurze Zeit haltbar. Ihre Lagerstabilität läßt sich zwar z.B. durch Einbeziehen von Phosphatidsäure erhöhen, jedoch ist die somit verbesserte Stabilität für viele Zwecke immer noch unzureichend. Weiter sind herkömmliche Liposomen nicht säurestabil und daher nicht für den Transport pharmazeutischer Wirkstoffe geeignet, die nach oraler Verabreichung den Magen passieren und erst im Darm resorbiert werden sollen.

Die Literaturstelle, "Biochimica et Biophysica Acta", Band 778, 1984, Seite 74-80, Blöcher et al, "PHYSICOCHEMICAL CHARACTERIZATION OF TETRAETHER LIPIDS FROM THERMOPLASMA ACIDOPHILUM" - "DIFFERENTIAL SCANNING CALORIMETRY STUDIES ON GLYCOLIPIDS AND GLYCOPHOSPHOLIPIDS", befäßt sich eingehend mit Thermoplasma acidophilum, unter anderem mit dem Organismus und den Kulturbedingungen sowie der differentiellen Abtastcalorimetrie (DSC). Auch ist die Basisstruktur von DialkyldiglycerolTetraethern aus Thermoplasma acidophilum in der allgemeinen Form beschrieben mit jeweiligen Resten $R_1$ und $R_2$, wobei $R_1$ und $R_2$ ein Zucker und ein Phosphoglycerylester sein können.

Die WO 93/08202, beschreibt die Bildung von stabilen Liposomen aus Lipidextrakten von Archaebakterien (Archaea). Unter anderem werden Polyetherlipide in ihrer Strukturformel angegeben. Auch befaßt sich diese Schrift mit einem Verfahren zum Herstellen unilamellarer Liposomen aus insgesamt polaren Lipidextrakten verschiedener Archaeobakterien.

Biochemica et Biophysica Acta, 478, (1977) 37-50, Thomas A. Langworthy et al. "long chain Diglycerol Tetraethers from Thermoplasma Acidophilum" beschreibt die Verwendung einer DEAE-Zellulose Acetat Chromatography.

Aufgabe der Erfindung ist es daher, die Herstellung mechanisch und chemisch stabilerer Liposomen mit verbesserter Lagerstabilität zu ermöglichen.

Diese Aufgabe wird erfindungsgemäß gelöst durch die Bereitstellung von Tetraetherlipiden (TEL) mit der allgemeinen Formel

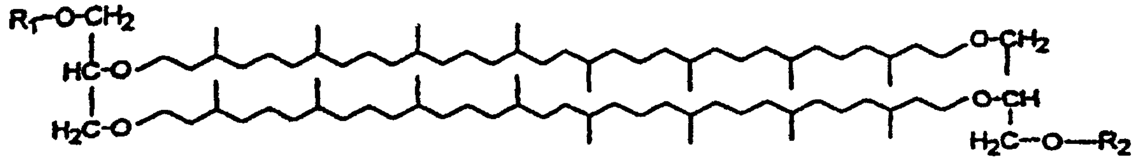

dadurch gekennzeichnet,
daß $R_1$ aus der die Reste $R_1A$-$R_1J$ umfassenden Gruppe ausgewählt ist, in der

$R_1A$:

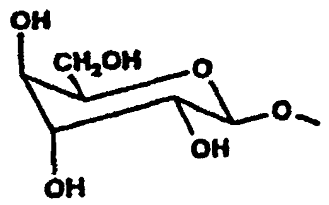

$R_1B$:

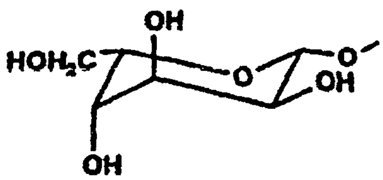

$R_1C$:

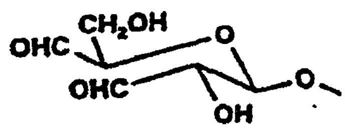

$R_1D$:

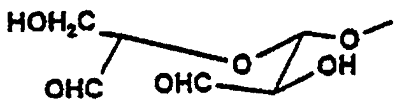

$R_1E$:

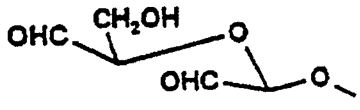

$R_1F$:

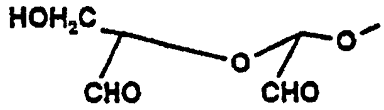

$R_1G$:

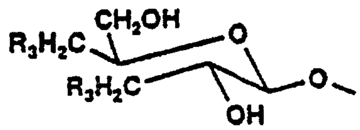

$R_1H$:

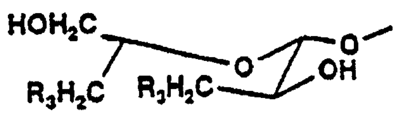

$R_1I$:

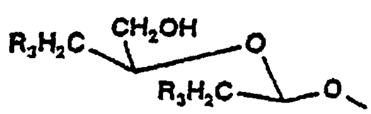

$R_1J$:

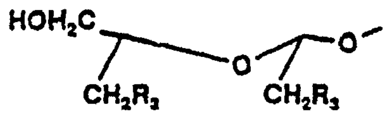

bedeutet, und wobei $R_2$ aus der die Reste $R_2K$, $R_2L$, $R_2M$, und Wasserstoff umfassenden Gruppe ausgewählt ist, worin

$R_2K$:

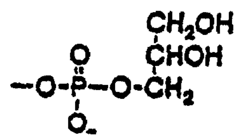

$R_2L$:

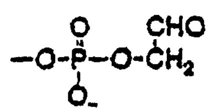

$R_2M$:

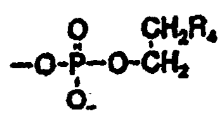

bedeutet und wobei

$R_3$ und $R_4$ sind aus der Wasserstoff, Peptide, Proteine, Polyamine, Cholin oder Polysaccharide, Polyethylenglykole (PEG) und Thiole umfassenden Gruppe ausgewählt sind.

Das Lipidgerüst des erfindungsgemäßen TEL besteht aus einem 72-gliedrigen Makrotetraethertyklus, in dem die $\bar{\omega}$-Kohlenstoffatome der Phytanylketten zweier Diethermoleküle kovalent miteinander verbunden sind. Tetraetherlipide diesen Typs sind bereits bekannt und bisher ausschließlich in Archaebakterien nachgewiesen worden. Eine Zusammenfassung aller bisher bekannten Basisstrukturen archaebakterieller Lipide ist in Langworthy und Pond (1986) enthalten.

Das Lipidgerüst des erfindungsgemäßen TEL enthält keine Doppelbindungen und ist daher unempfindlich gegen Oxidation. Weiter enthält es statt der in Lipiden aus Eubakterien und Eukaryonten enthaltenen Lipidesterbindung nur Lipidetherbindungen, die auch bei hohen Protonenkonzentrationen, wie sie zum Beispiel im Magen auftreten, nicht angegriffen werden. Schließlich enthält es in der ursprünglich isolierten Form überraschenderweise den Zucker Gulose,

der sich erfindungsgemäß leicht in Oxidationsprodukte umwandeln läßt. Weder Gulose noch eines seiner Oxidationsprodukte sind bisher in einem archaebakteriellen Lipid nachgewiesen worden.

In einer bevorzugten Ausführungsform enthält das erfindungsgemäße TEL als einen Zuckerrest ($R_1$) ein modifiziertes oder unmodifiziertes β-D-Gulosid ($R_1A$) und einen modifizierten oder unmodifizierten Rest $R_2$, bevorzugt $R_2K$. In weiteren Ausführungsformen ist $R_1$ ein durch Oxidation erhaltenes β-Gulosidderivat mit einer der oben als $R_1B$ bis $R_1J$ bezeichneten Formeln. $R_2$ kann Wasserstoff sein oder einer der Formeln $R_2K$, $R_2L$, $R_2M$, entsprechen. Durch die Modifikation ($R_3$, $R_4$) werden beispielsweise LDL-Rezeptoren-Liganden, z.B. ApoE oder β-Amyloid, Antikörper oder Teile dieser Proteine an die jeweiligen Reste angeheftet. Dabei erlaubt die Verbindung des erfindungsgemäßen TEL beispielsweise mit einem Protein die stabile Verankerung des Proteines unter Verwendung des TEL als Membrananker in einer herkömmlichen Lipiddoppelschicht.

Ein besonders bevorzugtes Tetraetherlipid mit $R_1$ = β-D-Gulosid und $R_2$ = Phosphoglycerin kann aus dem Archaebakterium Thermoplasma acidophilum (DSM 10217) gewonnen werden. Thermoplasmen wachsen zwischen pH 1,0 und 4,0 und bei Temperaturen von 33 bis 67°C. Die optimalen Kultivierungsbedingungen für Thermoplasma acidophilum DSM 10217 sind in Beispiel 1 wiedergegeben. Eine bis zu einer $OPD_{578}$ von ca. 0,6 gezüchtete Kultur des Mikroorganismus wird geerntet und die geernteten Mikroorganismen beispielsweise gefriergetrocknet. Das getrocknete Zellmaterial wird sodann mit organischen Lösungsmitteln extrahiert und die organische Phase von wäßrigen oder festen Bestandteilen getrennt. Anschließend wird das Lösungsmittel verdampft und der getrocknete Rückstand erneut in organischem Lösungsmittel aufgenommen. Die Probe wird auf DEAE-Zelluloseacetat chromatographiert und die TEL-haltigen Fraktionen anschließend einer Kieselgelchromatographie unterworfen. Die hierbei gewonnenen TEL-haltigen Fraktionen werden eingetrocknet und ergeben so das erfindungsgemäße TEL.

In einer bevorzugten Ausführungsform wird das getrocknete Zellmaterial mit Petrolether/Isopropanol (77:23; v/v) in einer Soxhlet-Apparatur extrahiert und nach dem Abdampfen des Lösungsmittels in Chloroform/Methanol (7:3; v/v) aufgenommen. Anschließend wird eine DEAE-Celluloseacetatchromatographie durchgeführt, um eine TEL-haltige Phospholipidfraktion von neutralen Lipiden, Glykolipiden und Pigmenten zu befreien. Aus den TEL-haltigen Phospholipiden wird anschließend TEL durch Chromatographie an Kieselgel isoliert. Die Durchführung des erfindungsgemäßen Verfahrens zur Gewinnung von TEL, die im einzelnen auch in Beispiel 2 beschrieben ist, ergibt bei einem Fermenteransatz von 50 Litern Thermoplasma acidophilum eine Ausbeute von ca.
10 mg TEL/1.

Für das erfindungsgemäße Tetraetherlipid gibt es eine Reihe möglicher Anwendungen, bei denen von seinen außergewöhnlichen Eigenschaften, vor allem bezüglich der Stabilisierung von Membranen, Gebrauch gemacht wird. So kann das erfindungsgemäße TEL beispielsweise zur Beschichtung von planaren Membranen, z.B. Polymermembranen verwendet werden. Dazu wird ein kondensierter Langmuir-Blodgett-Film, bestehend aus unterschiedlichen Phospholipiden einschließlich des erfindungsgemäßen TEL, bevorzugt TEL aus Thermoplasma acidophilum, auf eine präaktivierte Polymermembran übertragen und über die polaren Kopfgruppen kovalent an diese gebunden. Das bipolare membrandurchspannende TEL stabilisiert dabei den Lipidfilm. Durch die Verwendung dieses Lipides ist es möglich, die Polymeroberfläche in einem Einschrittverfahren mit einer Doppelschicht-artigen Anordnung zu bedecken.

Eine Vielzahl möglicher Anwendungen des erfindungsgemäßen Tetraetherlipides erfordern seine vorherige Modifikation. Beispiele solcher Reaktionen sind in Beispiel 4 gezeigt.

So ist z.B. eine orientierte Fixierung des Tetraetherlipids auf einer Oberfläche durch eine selektive Oxidation des Glycerinrestes möglich. Für eine selektive Oxidation des TEL aus Thermoplasma acidophilum wird dieses, wie in Beispiel 4 beschrieben, mit Periodat bei -20°C behandelt. Weiterhin kann ein flexibler, hydrophiler Abstandshalter für Abstände von bis zu 5 nm zwischen Polymeroberfläche und Lipidschicht eingefügt werden. Somit ist es möglich, einerseits Oberflächeninhomogenitäten auszugleichen und andererseits membrandurchspannende, zur zum Polymer gerichteten Seite der Lipidschicht orientierte Proteine zu inkorporieren. Solcher Art lipidbeschichtete planare Membranen können für die selektive Permeation und Separation von Protonen, Ionen oder kleinen hydrophilen Molekülen eingesetzt werden. Der Vorteil von bipolaren Tetraetherlipidmembranen im Vergleich zu solchen aus Doppelschicht-bildenden Esterlipiden besteht in ihrer erhöhten chemischen und mechanischen Stabilität sowie einer verminderten Ionenpermeabilität. So können z.B. Protonenpumpen, Trägerproteine und Ionophore in die planare Lipidschicht eingebaut werden, um Filtrationsprozesse durch äußere Signale, wie z.B. Licht, auszulösen oder zu modifizieren und damit zu steuern.

Das erfindungsgemäße TEL kann weiterhin zur Herstellung von Trennmaterialien verwendet werden. Dazu wird bevorzugt das aus Thermoplasma acidophilum isolierte TEL zunächst oxidiert, bevorzugt selektiv am Phosphoglycerinrest. Das oxidierte TEL kann sodann mit freien Aminogruppen von Trennmaterialteilchen (z.B. aminierten Silicabeads) Schiff'sche Basen ausbilden, beispielsweise durch Reaktion in Chloroform-Methanol-Lösung bei Raumtemperatur in Anwesenheit von Aktivatoren. Die entstandenen Schiff'sehen Basen werden mit Borhydrid im Dunkeln zum sekundären Amin reduziert und führen zu einem mit dem erfindungsgemäßen TEL bedeckten Trennmaterialteilchen. Eine zweite Möglichkeit, das erfindungsgemäße TEL (oder andere Tetraetherlipide) kovalent z.B. an Silica- oder Melamin-beads zu binden, besteht in der Verwendung von Cyanurchlorid ($C_3H_6N_3Cl_3$) als Aktivator. Hierbei kann auf die

Oxidation vicinaler Hydroxylgruppen verzichtet werden, die Bindung erfolgt direkt zwischen jeweils einer Hydroxylgruppe an der Teilchenoberfläche und am Lipid über das Cyanurchlorid.

Lipidbeschichtete sphärische Trennmaterialien, z.B. Kieselgel, aminiertes Kieselgel oder Melamin, lassen sich zur Füllung von Chromatographiesäulen und zu Affinitätstrennungen von (lipophilen) Molekülen, z.B. Steroiden, oder von membranständigen Proteinen verwenden. Außerdem ist es möglich, in die Lipidschicht Rezeptorstrukturen oder immunologisch aktive Proteine zu integrieren und damit die Selektivität und die Trennleistung zu optimieren.

In einer weiteren Ausführungsform dient das erfindungsgemäße TEL als solches, in bevorzugten Ausführungsformen aber auch in Verbindung mit üblichen Doppelschicht-bildenden Phospholipiden, als Modellmembran für Untersuchungen mit biochemischer Fragestellung.

In einer besonders bevorzugten Ausführungsform wird das erfindungsgemäße TEL zum Herstellen von Liposomen verwendet. Liposomen, deren Phospholipidanteil zu 100% aus erfindungsgemäßem TEL besteht, haben sich dabei als außergewöhnlich stabil, nahezu unbegrenzt lagerfähig und protonenundurchlässig erwiesen. Für viele Anwendungen, darunter die Formulierung von Pharmazeutika mit säurelabilen Wirkstoffen, stellen reine TEL-Liposomen daher das Mittel der Wahl dar.

Als vorteilhaft hat sich auch die Herstellung von Liposomen erwiesen, die neben einem Anteil von 0,1 bis 20 Gew.-% TEL übliche Doppelschicht-bildende Phospholipide enthalten (Mischliposomen), wobei dieser Gewichtsprozentanteil auf das Gesamtlipid bezogen ist. Bei diesen Doppelschicht-bildenden Phospholipiden kann es sich beispielsweise handeln um Sphingomyeline, Cephaline, Lecithine und Cardiolipin. Darüber hinaus kann jedes weitere für die Bildung von Liposomen geeignete Lipid herangezogen werden. Der besondere Vorteil der erfindungsgemäßen Mischliposomen ist ihre durch den TEL-Anteil verbesserte (erhöhte) Stabilität, verglichen mit Liposomen ohne TEL-Anteil.

Im folgenden beziehen sich die Ausdrücke "TEL-Liposom", "Liposom aus reinem TEL" etc. auf Liposomen, deren Phospholipidanteil zu 100% aus erfindungsgemäßen TEL, bevorzugt TEL aus Thermoplasma acidophilum besteht. "Mischliposomen" umfassen zusätzlich herkömmliche Phospholipide, und der Ausdruck "Liposom" umfaßt sowohl Liposomen aus reinem TEL als auch Mischliposomen.

In bevorzugten Ausführungsformen umfaßt das erfindungsgemäße Mischliposom 1 bis 15 Gew.-% TEL, besonders bevorzugt 5 bis 10 Gew.-% TEL, bezogen auf den Gesamtlipid.

Die erfindungsgemäßen Liposomen einschließlich der Mischliposomen können als wirkstoffträger für kosmetische und/oder pharmazeutische Wirkstoffe dienen. Sie werden bevorzugt bei solchen Wirkstoffen als Transportvehikel eingesetzt, bei denen eine orale Applikation nicht möglich ist, weil diese Wirkstoffe in der sauren Magenflüssigkeit inaktiviert oder durch Lipasen oder Peptidasen im Dünndarm abgebaut werden würden. Die erfindungsgemäßen Liposomen sind jedoch säurestabil und können deshalb ungehindert durch den Magen in den Dünndarm gelangen und eingeschlossene Wirkstoffe, die nach oraler Applikation normalerweise nicht in die Blutbahn gelangen können, dort zur Resorption bringen.

Die Wirkstoffe können im wäßrigen Lumen des Liposoms enthalten sein oder in die Liposomenmembran integriert werden. In einer weiteren Ausführungsform ist der Wirkstoff kovalent an das im Liposom enthaltene TEL gebunden.

Bevorzugte Wirkstoffe sind z.B. Cytostatika, Immunsuppressiva, Imunstimulanzien oder Impfstoffe sowie Hormone. Die Lokalisierung des Wirkstoffes im Lumen oder in der Membran ist abhängig von seiner Wasser- bwz. Lipidlöslichkeit und wird vom Öl/Wasser-Verteilungskoeffizienten bestimmt. Viele Wirkstoffe haben eine intermediäre Verteilung, d.h. sowohl eine gewisse Wasser- als auch Lipidlöslichkeit, gemäß derer sie sich innerhalb eines Liposomens auf Lumen und Membran verteilen.

Beispiele für im Lumen gelöste Wirkstoffe sind Impfstoffe, Hormone und wasserlösliche Anteile bzw. Derivate von Daunorubicin und Doxorubicin bzw. von Methylprednisolon. Ein bevorzugtes Methylprednisolonderivat ist dabei das Natriumsalz des Methylprednisolon-Hydrogensuccinats.

In der Membran lokalisierte Wirkstoffe müssen ausreichend lipidlöslich sein. In bevorzugten Ausführungsformen werden Liposomen verwendet, deren Membran die durch den Öl/Wasser-Verteilungskoeffizienten bestimmten lipidlöslichen Anteile bzw. Derivate von Daunorubicin und Doxorubicin bzw. von Methylprednisolon enthalten.

Bevorzugt kovalent gebundene Wirkstoffe sind dagegen beispielsweise Antikörper, Hormone, Lectine und Interleukine oder aktive Fragmente dieser Stoffgruppen.

Es konnte gezeigt werden, daß die erfindungsgemäßen Liposomen besonders gut mit Zellmembranen wechselwirken können. Nachgewiesen wurde die rasche Übertragung lipophiler Fluoreszenz-Farbstoffe (Bromobimane und Nilrot) auf die isolierte Erythrozytenmembran (Intermembranaustausch) und die starke Anreicherung Carboxyfluorescein-gefüllter TEL-Liposomen an der Oberfläche von Tumorzellen bzw. von Tumorzellkulturen. Die das erfindungsgemäße TEL aus Thermoplasma acidophilum enthaltenden Liposomen scheinen eine besondere Affinität für Tumorzellen aus dem Magen-Darm-Trakt sowie der Harnblase zu haben. Sie ermöglichen daher die zielgerichtete Behandlung von gut- oder bösartigen Tumoren dieser Organe, indem sie durch Konjugation oder Einschluß von Chemotherapeutika mit gegen Tumoren wirksamen Wirkstoffen beladen werden. Jedoch können auch andere Tumoren gezielt

bekämpft werden, indem z.B. tumorspezifische Antikörper bzw. Antikörperfragmente in die Liposomenmembran eingebaut werden. Eine chemotherapeutische Behandlung mit zielgerichtet wirkenden Chemotherapeutika trägt dazu bei, unerwünschte Nebenwirkungen drastisch zu reduzieren.

Die erfindungsgemäßen Liposomen ermöglichen darüber hinaus einen zielgerichteten Gentransfer. Dazu werden Nukleinsäuren, z.B. DNA- oder RNA-Sequenzen, die Gene oder Genfragmente enthalten und in linearer Form oder in Form von circulär geschlossenen Vektoren, die weiteres genetisches Material enthalten können, vorliegen, in reine TEL-Liposomen oder Mischliposomen verpackt und den zu transfizierenden Zellen in vitro oder in vivo zugesetzt. Wenn die Liposomenmembran darüber hinaus Proteine oder Peptide enthält, für die entsprechende Antikörper auf den mit der Gentherapie zu erreichenden Zellen vorhanden sind, so wird durch den Kontakt zwischen Antikörper auf der Zielzelle und Antigen in der Membran des erfindungsgemäßen Liposomes eine Konjugation zwischen Liposom und Zielzelle gefördert.

In einer weiteren erfindungsgemäßen Ausführungsform dient das erfindungsgemäße Tetraetherlipid in reiner Form oder als Bestandteil von reinen oder gemischten Liposomen als Grundlage für die Herstellung medizinischer Salben oder Hautcremes. In die Liposomen können ebenfalls zusätzlich Therapeutika verpackt werden.

Ein weiteres mögliches Anwendungsgebiet für das erfindungsgemäße TEL, für Liposomen aus reinem TEL aus Thermoplasma acidophilum oder die dieses TEL enthaltenden Mischliposomen ist die Verwendung dieser Substanzen in der medizinischen Diagnostik. Dabei können z.B. Mikrotiterplatten mit TEL, TEL-Liposomen oder TEL-Mischliposomen beschichtet werden, die durch Konjugation mit spezifischen Antikörpern oder Antigenen erhalten worden sind. Solcher Art beschichtete Träger können somit für die immunometrische Bestimmung von Proteinen, Peptiden oder Stoffwechselprodukten eingesetzt werden.

Die folgenden Abbildungen und die Beispiele erläutern die Erfindung.

**Beschreibung der Abbildungen**

Abbildung 1 zeigt das $^1$H-NMR-Spektrum von peracetyliertem Glykolipid in Benzol.

Abbildung 2 zeigt das $^1$H-NMR-Spektrum von peracetyliertem Glykolipid in Aceton.

Abbildung 3 ist ein Dünnschichtchromatogramm (5x5 cm; HPTLC, Silica-60, 5 µm) von methanolysiertem und solvolysiertem TEL. Die Bahnen zeigen von links nach rechts:

Abb. 3a)

Bahn 1: Methanolyse unter Rückfluß, 18 h
Bahn 2: Methanolyse bei RT, 80 h
Bahn 3: Methanolyse bei 4°C, 120 h

Abb. 3b)

Bahn 1: Vergleich TEL
Bahn 2: Solvolyse bei 4°C, 172 h
Bahn 3: Vergleich Gesamtglykolipidfraktion
Bahn 4: Methanolyse unter Rückfluß, 18 h (als Referenz für den Tetraether = TELoK)
Bahn 5: Solvolyse bei RT, 172 h

Das Chromatogramm wurde in Chloroform/Methanol 9:1 (v/v) entwickelt und der Nachweis der Produkte erfolgte in Methanol/Schwefelsäure 9:1 (v/v). Das erfindungsgemäße Tetraetherlipid aus Thermoplasma acidophilum bleibt am Startpunkt liegen. Das Glykolipid hat einen Rf-Wert von 0,5 und der Tetraether ohne polare Kopfgruppen hat einen Rf-Wert von 0,7.

Abbildung 4 zeigt das $^1$H-NMR-Spektrum von TEL in Methanol.

Abbildung 5a ist ein Massenspektrum eines TEL-Natriumadduktes und Abbildung Sb das eines Lithiumadduktes. Aus den beiden Massenspektren errechnet sich eine Molekülmasse (M) von 1617,4.

| | | | |
|---|---|---|---|
| $(M + Na)^+$ | = 1640,4 | (-23) | M = 1617,4 |
| $(M + 2Li - H)^+$ | = 1630,4 | (-13) | M = 1617,4 |

Unter den Konditionen der Massenanalyse wird ein Natrium, jedoch 2 Lithium - eines zur Neutralisation - dem sauren Lipidmolekül assoziiert. Bei den Korrekturen der experimentellen Daten wurden alle Massengewichte auf ganze Zahlen gerundet.

Abbildung 6 zeigt das $^1$H-NMR-Spektrum von Glykolipid (GL) in Methanol.

Abbildung 7a zeigt das Massenspektrum des GL-Natriumadduktes und Abbildung 7b das des GL-Lithiumadduktes. Aus beiden Massenspektren errechnet sich eine Molekülmasse (M) von 1463,6.

| | | | |
|---|---|---|---|
| $(M + Na)^+$ | = 1486,6 | (-23) | M = 1463,6 |
| $(M + Li)^+$ | = 1470,6 | (- 7) | M = 1463,6 |

Unter den Konditionen der Massenanalyse wird ein Natrium bzw. ein Lithium dem durch Abspaltung des Phosphoglycerins nicht mehr sauren Lipidmolekül assoziiert. Die Differenz von 1617,4 - 1463,6 entspricht dem Verlust des Phosphoglycerins.

Abbildung 8 zeigt das Formelschema der Hydrolyse von TEL aus Thermoplasma acidophilum.

Abbildung 9 zeigt den Vergleich der Aufnahme von in TEL-Liposomen bzw. PC-Liposomen eingeschlossenem Carboxyfluorescein in verschiedene Zellinien.

Abbildung 9a: HT-29-Zellen

Abbildung 9b: HepG2-Zellen

Abbildung 9c: CHO-Zellen

Abbildung 9d: CF-Pac-Zellen

**Beispiel 1**

**Kultivierung und Konservierung von Thermoplasma acidophilum**

1.1 Medium

Das Kulturmedium für Thermoplasma acidophilum setzt sich aus Freundt'schem Medium (Christiansen et al., (1975)), einer 20%igen (w/v) Glucoselösung und einer 20%igen (w/v) Hefeextraktlösung (Difco) zusammen. Das Freundt'sche Medium wird *in situ* bei 120°C sterilisiert, der 10 l-Fermenter 25 min und der 50 l-Fermenter 45 min. Die Glucose- und Hefelösungen werden separat 10 min bei 110°C sterilisiert und erst unmittelbar vor der Inokulation dem Medium beigefügt.

1.1.1 Für die Inokulation mit Gefrierzellen werden 94 vol.-% Freundt'schem Medium 5 Vol.-% Glucoselösung und 1 Vol.-% Hefeextraktlösung zugefügt.

1.1.2 Für die Beimpfung mit einer Suspensionskultur werden 84 Vol.-% Freundt'sches Medium, 5% Glukoselösung und 1% Difcolösung wie oben vereint.

1.2 Inokulation

2.1 Im Fall der Kultur aus Gefrierzellen werden dem in 1.1 beschriebenen Medium 1 ml/l Gefrierzellen zugesetzt. Die lag-Phase beträgt 2 bis 3 Tage.

2.2 Für Kulturen mit Suspensionskultur-Inokulum werden dem in 1.2 beschriebenen Medium 10 Vol.-% Suspensionskultur zugesetzt. Die lag-Phase beträgt in diesem Fall nur wenige Stunden.

1.3 Kultivierung von Thermoplasma acidophilum

Thermoplasma acidophilum wird bevorzugt in 10 l- oder 50 l-Fermentern gezüchtet. Alle Fermenterteile müssen aus Schwefelsäure-festem Material sein. Der Anmelder verwendet die folgenden Fermenter: Braun Biostat S (10 l, Glaskorpus), Braun Biostat 50 D (50 l, Edelstahlkorpus).

Ausgehend von Gefrierzellen, die wie unten beschrieben konserviert worden sind, kann ein 10 l-Fermenter ohne vorherige Flaschenzucht direkt angeimpft werden. Die Normalbedingungen bei der Fermenterzucht sind 59°C und pH 2. Nach Erreichen einer optischen Dichte von 0,4 (bei 578 nm) wird das erste Mal 1 Vol.-% Hefeextraktlösung nachgeführt, ein weiteres Mal nach weiteren 8 Stunden. Die Zugabe von Hefeextrakt läßt den pH im Medium ansteigen und wird durch Zugabe entsprechender Mengen von 1 M Schwefelsäure kompensiert. Nach Erreichen einer optischen Dichte (578 nm) von 0,6 bis 0,7 werden 5 l der 10 l-Fermenterkultur als Inokulum für einen 50 l-Fermenter entnommen. Dieser wird wie üblich kultiviert, wobei nach 20 und 28 Stunden jeweils 1 Vol.-% Hefeextrakt zugeführt wird. Nach

Erreichen der stationären Phase wird 1 Liter als Inokulum für einen weiteren 10 l-Fermenter verwendet.

Thermoplasma acidophilum wächst obligat aerob, hohe Sauerstoffkonzentrationen sind für das Wachstum jedoch nicht vorteilhaft. Die Sauerstoffregulation wird auf 0,02 bis 0,03 vvm (Volumen Belüftung pro Volumen Medium pro Minute) im 10 l-Fermenter und auf 0,04 vvm im 50 l-Fermenter eingestellt. Während des Wachstums nimmt der Sauerstoffgehalt des Mediums konstant und rapide bis unter die Grenze der Meßbarkeit ab, muß aber nicht gegenreguliert werden. Der pH wird während der Kultivierung laufend gemessen und bei pH-Veränderungen reguliert.

1.4 Zellernte

Das Wachstum von Thermoplasma acidophilum erreicht die stationäre Phase nach ca. 40 Stunden ($OD_{578}$ von ca. 0,6). Die Ernte sollte daher nach ca. 40 Stunden bei einer $OD_{578}$ von 0,6, maximal 0,7 erfolgen.

Die Zellkultur aus einem 10 l-Fermenter wird in einer Heraeus-Stockzentrifuge 15 Minuten bei 3000 x g geerntet, der Überstand wird verworfen, der Niederschlag wird in Freundt'scher Lösung resuspendiert. Die Suspension wird 10 Minuten in einer Christzentrifuge bei maximaler Umdrehungszahl (4500 Upm, entsprechend in etwa 3000 x g) zentrifugiert, der Vorgang noch mindestens zweimal wiederholt, wobei Freundt'sche Lösung durch schwefelsaures Aquabidest, pH 2, ersetzt wird, bis das Pellet weiß ist. Schließlich wird die weiße Zellnaßmasse in Aguabidest suspendiert, in Methanol/Trockeneis eingefroren und bis zur Gewichtskonstanz gefriergetrocknet.

Die Zellkultur des 50 l-Fermenters wird mittels eines Pelicon-Tangentialfluß-Filtersystems zur Ernte auf ein Volumen von 2 l konzentriert. Dieses Konzentrat wird mit schwefelsaurem destilliertem Wasser, pH 2, gewaschen, bis das Filtrat klar und ungefärbt ist. Nun wird die konzentrierte Zellsuspension zentrifugiert (s.o.), das Pellet in Aquabidest resuspendiert, rezentrifugiert, resuspendiert, eingefroren und bis zur Gewichtskonstanz gefriergetrocknet.

1.5 Optische Kontrolle der Reinheit der Thermoplasma acidophilum-Kultur

Im Lichtmikroskop erscheint Thermoplasma acidophilum nach obiger Kultur mit einer Zelldichte von $10^7$ Zellen/ml in meist isodiametrischer Form mit einem Durchmesser zwischen 1 und 3 µm, einige Zellen haben pleiomorphes Aussehen. Nach Laserlicht-Streumessungen im Malvern particle sizer liegt das Maximum der Größenverteilung bei 2,3 µm. Gefrierbruchelektronenmikroskopisch erscheinen die Zellen meist rund, pleiomorphe Zellen weisen Längsachsen zwischen 1,1 und 2,7 µm und Querachsen um 0,6 µm auf. Die Zellen zeigen ein typisches Bruchverhalten, das sie von den meisten anderen Zellen unterscheidet: sie brechen senkrecht (quer) zur Membranebene, entlang der Kohlenwasserstoffketten und nicht entlang der inneren Grenzfläche der Doppelschicht (tangential).

Bei den gegebenen Wachstumsbedingungen (59°C, pH 2) können nur wenige Organismen in die Kultur einwachsen, z.B. Bacillus acidocaldarius. Züchtung bei pH 1,5 verhindert auch dies, ist allerdings mit einem Ausbeuteverlust hinsichtlich der gewünschten Lipidkomponente verbunden. Somit liegt das Züchtungsoptimum bei pH 2 unter Einhaltung möglichst steriler ("keimreduzierter") Bedingungen.

Eine Kontamination der Thermoplasma acidophilum-Kultur kann schon während des Wachstums erkannt werden: die exponentielle Phase verläuft wesentlich steiler, d.h. die OD nimmt stärker zu als bei einer reinen Thermoplasma acidophilum-Kultur. Außerdem ändert sich der Geruch der Kultur in typischer Weise. Im Lichtmikroskop erscheinen bei Kontamination erheblich größere Zellen und elektronenmikroskopisch haben diese Zellen ein anderes Gefrierbruchverhalten als Thermoplasma acidophilum.

1.6 Konservierung

Die Konservierung von Thermoplasma acidophilum erfolgt bei -80°C oder in flüssigem $N_2$. Zur Konservierung wird eine aktive Kultur (8-10 l) durch Zugabe von Calciumcarbonat auf pH 5,0-5,5 eingestellt. Nach Sedimentation von Calciumsulfat und überschüssigem Calciumcarbonat (mindestens 30 min ruhig stehenlassen) wird der Überstand abgehoben und unter sterilen Bedingungen 15 min bei 3000 x g zentrifugiert. Der Überstand wird verworfen, und die Thermoplasma acidophilum-Zellen des Pellets werden in frisch zubereiteten 10 mM Natriumcitratpuffer, pH 5,5, resuspendiert. Die Suspension wird auf Eis in Cryocups auf 0,5 bis 3,0 ml portioniert, die Cryocups werden eine Stunde in flüssigem Stickstoff eingefroren und anschließend darin oder bei -80°C aufbewahrt.

Zur Kultivierung werden die Cryocups mit den Zellen im Wasserbad bei 37°C aufgetaut.

Es ist unbedingt notwendig, daß die Konservierungsschritte steril durchgeführt werden, da der pH von 1-2, der bei der Kultivierung das Einwachsen der meisten Mikroorganismen verhindert, nicht eingehalten wird.

**Beispiel 2**

**Extraktion und Reinigung des TEL aus Thermoplasma acidophilum-DSM 10217**

2.1 Extraktion von Gesamtlipid

Die Extraktion von Gesamt-Lipid wird mit gefriergetrocknetem Material durchgeführt. Dazu werden 8 bis 10 g gefriergetrocknete Zellmasse mit 300 ml eines Petrolether(Fraktion 60 bis 80°C)/2-Propanol-Gemisches (77:23 v/v) in einer Soxhletapparatur (rekurrierendes, geschlossenes System) 40 Stunden unter Rückfluß kontinuierlich extrahiert. Die beschriebene Extraktion ist nahezu quantitativ und führt zu einer reinen Gesamtlipidfraktion.

2.2 Anreicherung der Phospholipidfraktion

Das Gesamtlipid einschließlich der Phospholipide wird mittels einer Büchi 680 MPLC-Anlage, bestehend aus einer Pumpe (max. Fördervolumen 30 ml/min), 6-Wege-Probenaufgabeventil, Fraktionssammler und Brechungsindex-Detektor (Refraktometer 188,00, Knauer, Berlin), fraktioniert.

Vorbereitung der Säule: DEAE-Zellulose (Serval Cellulose Ionenaustauscher p.a. Typ DEAE 32, Serva, München) wird nach der Methode von Rouser et al. (1986) mit Essigsäure in das Acetat überführt. Mit diesem DEAE-Zellulose-Acetat wird eine Büchi MPLC-Stempelsäule (Durchmesser 2,5 cm, Füllhöhe ca. 20 cm) im Slurry-Verfahren gepackt und mit Chloroform/Methanol (7:3 v/v) konditioniert.

500 mg Lipidgesamtextrakt werden in 10 ml Chloroform/Methanol (7:3 v/v) gelöst und auf die DEAE-Zellulose-Acetat-Säule aufgetragen.

Neutrale Lipide und Glykolipide werden mit Chloroform/Methanol (7:3; 4 Säulenvolumina) eluiert, Pigmente mit Chloroform/Eisessig (3:1; 4 Säulenvolumina). Anschließend wird die Essigsäure mit 4 Säulenvolumina Methanol von der Säule gespült und diese mit Chloroform/Methanol (7:3; 3 Säulenvolumina) wieder konditioniert. Schließlich werden die Phospholipide mit 5 Säulenvolumina folgenden Elutionsgemisches mit einem Fluß von 6 ml/min zu 20 ml Fraktionen fraktioniert: Chloroform/Methanol/Ammoniaklösung 25 %/Ammoniumacetat (63:27:10:1; v/v/v/w).

Alle Fraktionen werden dünnschichtchromatographisch auf ihren TEL-Gehalt überprüft, TEL-haltige Fraktionen werden vereint und mit wäßriger 0,1 M KCl mehrmals gewaschen. Die organische Phase wird nach folgendem Schema evaporiert (Stufe 1 bis 4: Rotationsverdampfer Büchi):

Evaporations-Schema:

| Stufe | Wasserbadtemperatur | Vakuum |
|---|---|---|
| 1 | 50°C | 600 mbar |
| 2 | 50°C | 250 mbar |
| 3 | 50°C | 20 mbar |
| 4 | Eventuell vorhandene Wasserreste werden durch Zugabe kleiner Mengen Chloroform/Methanol 3:1 und Wiederholung der Stufen 1-3 azeotrop entfernt. | |
| 5 | RT | <1 mbar (12h) |

Ausbeute:

500 mg aufgegebene Lipidmenge ergibt 250-300 mg TEL-haltige Phospholipidfraktionen.

2.3 Fraktionierung der TEL-haltigen Phospholipide

Die TEL-haltigen Phospholipide werden weiter über eine Silika-Säule (23 x 2,5 cm) fraktioniert. Die Säule wird mit Silikagel (Europrep. 60-20, Eurochrom/Knauer, Berlin) im Trokkenfüllverfahren mit Stickstoff bei einem Druck von 10 bar gepackt und anschließend mit dem Elutionsmittel Chloroform/Methanol/Wasser (70:25:4 v/v/v) oder Petrolether/2-Propanol/Wasser (70:80:10 v/v/v) konditioniert. 100 mg Phospholipid werden in 5 ml Elutionsgemisch gelöst und über *das* Probenventil auf die Säule gegeben. Die Phospholipide werden in 6 ml Fraktionen bei einem Fluß von 10 ml/min eluiert.

Ausbeute: 100 mg aufgegebener Phospholipide ergeben ca. 80 mg TEL.

2.4 Ausbeuten des Verfahrens

Ein Züchtungsansatz im 50 l-Fermenter ergibt 30 g Zelltrokkenmasse und 1 g Gesamtlipid. Daraus lassen sich 500 bis 600 mg TEL-haltige Phospholipidfraktionen isolieren, und aus diesen wiederum 400 bis 500 mg TEL.

2.5 Überprüfung der Reinheit

Die Reinheit der angereicherten TEL-Fraktion wird mit HPLC und HPTLC überprüft. Für die Durchführung der Analysen werden die folgenden Geräte und Chemikalien verwendet:

2.5.1 HPLC:

Anlage: Waters Ass., Eschborn: Pumpen Modell 510, Injektor Model U6K, Probenschleife 100 µl, Photodetektor LC-Spektrometer Lambda-Max 481.
On-line-Entgaser ERC, Pulsationsdämpfer ERC und Refraktometer ERC 7512 (ERMA Optical Works, Tokio), temperiert bei 35°C.
2-Kanalschreiber BD 41, Kipp und Zonen, Delft, Holland. Analytische Säule: 250 x 4 mm gepackt mit LiChrosorb Si 100, 5 µm (Merck, Darmstadt).
Elutionsmittel: n-Hexan/2-Propanol/Wasser (60:80:11 v/v/v).
Probenaufgabe: 200 µg TEL.
Flußrate: 1 ml/min.

2.5.2 HPTLC:

Desaga, Heidelberg: Horizontale Entwicklungskammer für HPTLC-Platten 5 x 5 cm, Silika 60, 5 µm (Merck, Darmstadt); Fließmittel:

a) für apolare Lipide: Chloroform/Diethylether 6:1 (v/v);

b) für Glykolipide: Chloroform/Methanol 9:1 (v/v);

c) für Phospholipide: Chloroform/Methanol/Wasser 80:25:4 (v/v/v) und Chloroform/Methanol/Wasser 65:25:4 (v/v/v); Lipid-Detektion mit Schwefelsäure/Methanol 1:9 (v/v), Erhitzen auf 140°C;

Detektion der Phospholipide mit Dittmers Reagens (Dittmer & Lester, 1964);
Detektion der Glykolipide mit Orcinolreagens (Karlsson & Martensson, 1968), Erhitzen auf 100°C, 10-15 min.

**Beispiel 3**

**Charakterisierung des TEL-Moleküles aus Thermoplasma acidophilum**

3.1 Identifizierung des Zuckerrestes

Zur Identifizierung des Zuckerrestes wurde zunächst das TEL aus Thermoplasma acidophilum durch Abspalten der polaren Phosphoglycerinkopfgruppe zum Glykolipid (GL) hydrolysiert (s.u.) und mittels $^1$H-NMR untersucht. Die nach Zuordnung der Signale im $^1$H-NMR-Spektrum analysierten $^1$H-$^1$H-Koppelkonstanten lassen eine Bestimmung der relativen Konfiguration der Zentren C-1, C-2 und C-3 zu. Die Konfiguration an C-4 und C-5 wurde durch ΔNOE-Messungen ermittelt. Diese Messungen wurden zur besseren Signaltrennung an peracetyliertem Glykolipid durchgeführt. Untersuchungen einer Lösung dieser Substanz in Benzol-$D_6$ (Abb. 1) zeigte anhand der nahezu identischen $^1$H-$^1$H-Koppelkonstanten im Vergleich zum Glykolipid, daß die Peracetylierung keine Veränderung von Konstitution, Konfiguration oder Konformation des Zuckerrestes zur Folge hatte.
Unter Verwendung von Aceton-$D_6$ (Abb. 2) als Lösungsmittel konnte ein NOE zwischen H-5 und H-1 nachgewiesen werden. Daraus folgt, daß sowohl H-1 als auch H-5 axial angeordnet sind. Über die kleine Kopplung (1,5 Hz) von H-5 zu H-4 folgt für H-4 eine equatoriale Anordnung. Somit sind die relativen Konfigurationen an C-1 bis C-5 eindeutig bestimmt.

Tabelle 1

| Spektroskopische Daten für peracetyliertes Glykolipid in Benzol | | | | |
|---|---|---|---|---|
| Zuordnung | Bezeichnung im Spektrum | δ/ppm | Multiplizität, Koppelkonstanten/Hz | |
| H-3 eq | a | 5,81 | t | 2 x 3,5 |
| H-2 ax | b | 5,53 | dd | 8, 3,5 |
| H-4 eq | c | 5,21 | dd | 3,5, 1,5 |
| H-1 ax | d | 4,98 | d | 8 |
| H-7' | e | 4,36 | dd | 11,5, 4,5 |
| H-6 | f | 4,34 | dd | 11, 6,5 |
| H-6 | g | 4,28 | entartet | |
| H-7' | h | 4,25 | dd | 11, 5,5 |
| H-5 ax | i | 4,24 | entartet | |
| H-7' | j | 4,12 | dd | 10,5 |
| H-7' | k | 3,81 | m | |
| H-8 | l | 3,73 | m | |

500 Mhz, 5 mg in Benzol

Verknüpfungsmatrix über Koppelkonstanten:

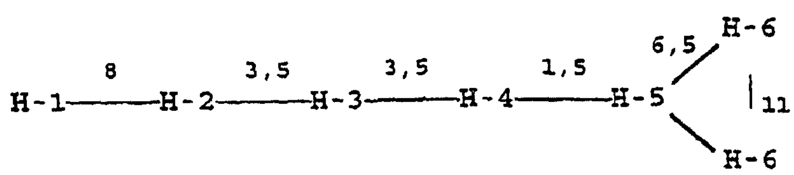


ΔNOE's:

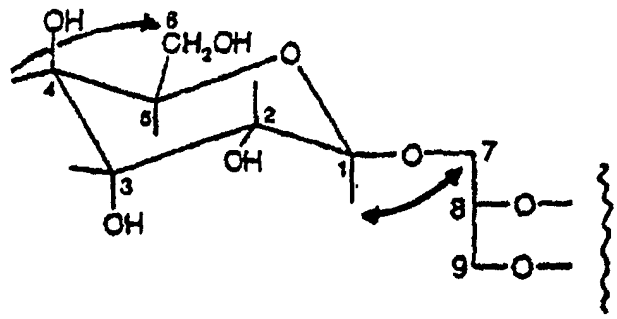

Tabelle 2

| Spektroskopische Daten für peracetyliertes Glykolipid in Aceton | | | | |
|---|---|---|---|---|
| Zuordnung | Bezeichnung im Spektrum | δ/ppm | Multiplizität, Koppelkonstanten/Hz | |
| H-3 eq | a | 5,34 | d | 3,5 |
| H-4 eq | b | 4,97 | dd | 3,5, 1,5 |
| H-1 ax | c | 4,94 | d | 8 |
| H-2 ax | d | 4,91 | dd | 8, 3 |
| H-5 ax | e | 4,30 | td | 2 x 5,5, 1,5 |

Tabelle 2 (fortgesetzt)

| Spektroskopische Daten für peracetyliertes Glykolipid in Aceton | | | | |
|---|---|---|---|---|
| Zuordnung | Bezeichnung im Spektrum | δ/ppm | Multiplizität, Koppelkonstanten/Hz | |
| H-6 | f | 4,18 | dd | 11,5, 5,5 |
| H-7' | g | 4,13 | dd | 11,5, 4,5 |
| H-6 | h | 4,12 | dd | 11,5, 5,5 |
| H-7' | i | 4,05 | dd | 11,5, 6 |
| H-7 | j | 3,84 | dd | 10,5, 5 |

Verknüpfungsmatrix über Koppelkonstanten:

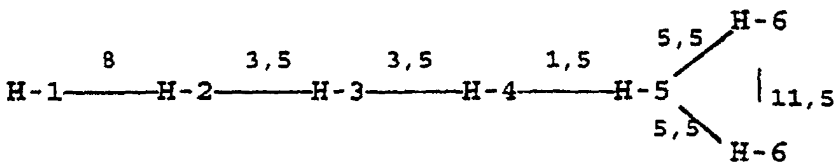


ΔNOE's:

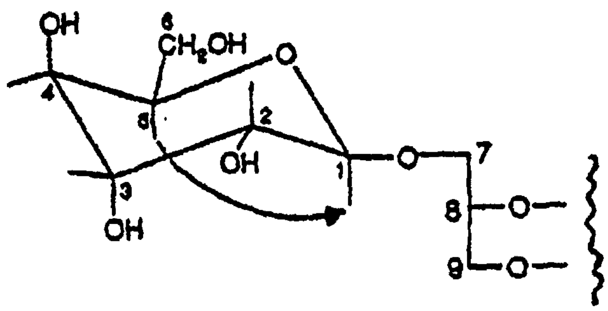

Ergebnis der NMR-Spektroskopie: Der Zuckerrest des TEL aus Thermoplasma acidophilum DSM 10217 ist β-Gulose.

3.2 Summenformel und Strukturvorschlag

Für das erfindungsgemäße TEL ist mittels FAB-MS ein Molekulargewicht von 1617,4 festgestellt worden. Dieser Wert korreliert gut mit der vorgeschlagenen Struktur eines 2,3,2',3'-Tetra-O-dibiphytanyl-di-sn-glycerol-1'-β-gulosyl-1-phosphoryl-3"-sn-glycerols, für das im undissoziierten Zustand ein MG von 1617,4 berechnet wird. Das Grundgerüst des TEL ist ein 72-gliedriger Dibiphytanyl-diglyceryl-tetraetherheterozyklus mit der Summenformel $C_{86}H_{156-172}O_6$. Die Anzahl der Wasserstoffatome in der Summenformel variiert, da sich in Abhängigkeit z.B. von der Züchtungstemperatur innerhalb der Dibiphytanyl-Ketten Pentazyklen ausbilden, die dem Lipid seinen spezifischen physikochemischen Charakter geben. Bei jeder Pentazyklisierung werden 2 Wasserstoffatome abgespalten. Die Molekulargewichte der verschiedenen dabei entstehenden Tetraethergrundgerüste sowie ihr jeweiliger Anteil bei den verschiedenen Züchtungstemperaturen sind in der nachfolgenden Tabelle angegeben.

Tabelle 3

| Verteilung der Pentazyklisierung bei 39°C und 59°C Züch- tungstemperatur | | | |
|---|---|---|---|
| Anzahl der Pentazyklen | Molekulargewicht | %-Anteil bei Züchtungstemperatur von 39°C | %-Anteil bei Züchtungstemperatur von 59°C |
| 0 | 1301,3 | 5,5 ± 0,5 | 6,6 ± 0,5 |
| 1 | 1299,3 | 17,3 ± 0,4 | 9,2 ± 1,2 |
| 2 | 1297,3 | 23,0 ± 4,0 | 9,4 ± 0,05 |
| 3 | 1295,3 | 23,4 ± 0,5 | 17,1 ± 1,4 |
| 4 | 1293,3 | 14,8 ± 1,3 | 19,0 ± 1,3 |
| 5 | 1291,2 | 10,8 ± 1,0 | 21,6 ± 0,3 |
| 6 | 1289,2 | 3,6 ± 0,9 | 11,7 ± 0,2 |
| 7 | 1287,2 | 1,2 ± 0,5 | 4,7 ± 0,3 |
| 8 | 1285,2 | 0,5 ± 0,4 | 0,8 ± 0,3 |

**Beispiel 4**

**Chemische Modifikationen des TEL aus Thermoplasma acidophilum**

4.1 Hydrolyse

Für die Hydrolyse des TEL wurden die beiden Verfahren der HF-Spaltung (Solvolyse) und der Methanolyse eingesetzt.

4.1.1 Solvolyse mittels Flußsäure

TEL-Lösungen (ca. 15 mg/ml) in Petrolbenzin/2-Propanol (3:2 v/v) werden in Szintillationsröhrchen überführt und das Lösungsmittel mit Stickstoff verblasen. Lösungsmittelreste werden durch Trocknen der Proben 5 Stunden lang im Hochvakuum entfernt, und anschließend wird das genaue Gewicht der Probe ermittelt.

Zu diesen TEL-Proben wird wäßrige 48%-ige Flußsäure zugegeben, bis die Flüssigkeit den Lipidfilm vollständig bedeckt, und die so vorbereiteten Reaktionsansätze alternativ ca. 7 Tage bei folgenden Temperaturen inkubiert:

a) 4°C
b) RT

Nach dem Reaktionsende werden die Proben zunächst in einem Exsikkator über KOH, bei Wasserstrahlpumpen-Vakuum und anschließend im Hochvakuum getrocknet. Das Lipid wird in Petrolbenzin (Endkonzentration 1 mg/ml) aufgenommen und dreimal mit jeweils der Hälfte des Volumens der organischen Phase einer 0,58%-igen NaCl-Lösung gewaschen. Die organische Phase wird mit $Na_2SO_4$ getrocknet, im Rotationsverdampfer evaporiert und im Hochvakuum restlos entfernt.

Der Reaktionsfortgang wird wie folgt chromatographisch kontrolliert:

Auf einer 5 x 5 cm HPTLC Si-Platte werden jeweils 15-20 µg der Proben aufgetragen. Als Referenzsubstanzen werden jeweils 15-20 µg einer TEL-Probe sowie einer aus Glykolipiden und apolaren Lipiden zusammengesetzten Probe (DEAE-Säule) auf unterschiedlichen Bahnen aufgetragen.

Die Platte wird in die Laufmittelkammer eingesetzt, in verschiedenen Laufmitteln entwickelt und mittels methanolischer Schwefelsäure detektiert.

Die eingesetzten Laufmittel sind:

Chloroform/Methanol/Wasser 60:25:4 (v/v/v)
Chloroform/Methanol 9:1 (v/v)
Chloroform/Diethylether 6:1 (v/v)

4.1.2 Hydrolyse mittels 1 M methanolischer Salzsäure (Methanolyse)

TEL-Proben in Chloroform/Methanol 2:1 (v/v) werden in Rundkolben pipettiert, im Rotationsverdampfer evaporiert, im Hochvakuum getrocknet, und das genaue Gewicht der jeweiligen Probe wird ermittelt. Zu den auf diese Weise vorbereiteten Lipidfilmen wird wasserfreies Methanol zugegeben (Lipidkonzentration 0,5 mg/ml), mit 37%-iger methanolischer Salzsäure versetzt (1 M HCl in Methanol) und anschließend alternativ bei folgenden Temperaturen und Zeiten inkubiert:

a) 4°C, 5 Tage
b) RT, 3 Tage
c) unter Rückfluß mit aufgesetztem Rückflußkühler und mit $CaCl_2$-gefülltem Trockenrohr, 18 Stunden.

Der Reaktionsablauf wird durch periodische Probenentnahme mittels DC wie oben beschrieben kontrolliert.

Nach Reaktionsende wird der Reaktionslösung das doppelte Volumen an Chloroform zugegeben, und mit einer 0,5 M $Na_2CO_3$-Lösung (ca. der Hälfte des Volumens der Reaktionslösung) werden in einem Scheidetrichter zwei Phasen gebildet. Es wird gewaschen, die abgetrennte organische Phase wird noch zweimal mit jeweils 1/4 Volumen 0,5 M $Na_2CO_3$-Lösung gewaschen, mit $Na_2SO_4$ getrocknet, im Rotationsverdampfer evaporiert und schließlich das organische Lösungsmittel im Hochvakuum restlos entfernt.

4.1.3 Reinigung der Solvolyse- bzw. Methanolyseprodukte

Die Aufreinigung bzw. Isolierung der Produkte aus der HF-Spaltung sowie der Methanolyse erfolgt mittels MPLC (Büchi-System) auf einer Silica-Säule. Säule: 23 x 2,5 cm, trocken gepackt mit Europrep 60-20 (Eurochrom/Knauer, Berlin) und im Laufmittel Chloroform/Methanol 15:1 (v/v) vorkonditioniert. Fließgeschwindigkeit 3 ml/min; fraktioniert wird in 3 ml Fraktionen. Die Fraktionen werden auf ihren Lipidgehalt sowie Reinheit wie oben beschrieben mittels DC untersucht. Die reinen Fraktionen werden gesammelt, im Rotationsverdampfer evaporiert und anschließend im Hochvakuum getrocknet. Die restlichen Mischfraktionen werden je nach Lipidgehalt entweder gepoolt und erneut fraktioniert oder verworfen.

4.1.4 Analyse der Reaktionsprodukte

Die so erhaltenen Reaktionsprodukte werden wie oben beschrieben mit DC auf ihre Zusammensetzung untersucht. Bei einem typischen Dünnschichtchromatogramm auf einer 5 x 5 cm HPTLC-Platte (Silica-60, 5 µm), die mit Chloroform/Methanol 9:1 (v/v) entwickelt wird, können nach Inkubation in Methanol/Schwefelsäure 9:1 (v/v) der erfindungsgemäßen Tetraetherlipide am Startpunkt beobachtet werden, während Glykolipid einen Rf-Wert von 0,5 und Tetraether ohne polare Kopfgruppen einen Rf-Wert von 0,7 aufweist.

Tabelle 4 gibt einen Überblick über die Aüsbeuten an Glykolipid (GL) und Tetraetherlipid ohne polare Kopfgruppen (TELoK) nach Hydrolyse oder Solvolyse des erfindungsgemäßen Tetraetherlipides aus Thermoplasma acidophilum. Die Verhältnisse zwischen Glykolipid und Tetraetherlipid ohne polare Kopfgruppen sind bei Raumtemperatur zeitabhängig.

Tabelle 4

| Ausbeuten an Glykolipid und Tetraether bei Methanolyse und Solvolyse (die Verhältnisse zwischen Glykolipid und Tetraether sind bei RT zeitabhängig): | | |
|---|---|---|
| METHODE | **Glykolipid** | **Tetraether** |
| **Solvolyse** | | |
| 7 Tage bei 4°C | ca. 70 % | ca. 30 % |
| 7 Tage bei RT | ca. 30 % | ca. 70 % |
| **Methanolyse** | | |
| 5 Tage bei 4°C | ca. 90 % | ca. 10 % |
| 3 Tage bei RT | ca. 50 % | ca. 50 % |
| 18 h unter Rückfluß | - | 100 % |
| RT = Raumtemperatur | | |

Die TEL-Solvolyse bzw. Methanolyse liefern, wie aus Abbildung 3 ersichtlich wird, chromatographisch die

gleichen Produkte, was durch massenspektroskopische und NMR-spektroskopische Aufnahmen bestätigt wird: Eines der Produkte entspricht dem Glykolipid nach der Abspaltung bzw. Hydrolyse des Phosphorylglycerinesters. Im $^1$H-NMR-Spektrum sind die Signale des Phosphorylglycerolesters nicht mehr vorhanden. Dieses Ergebnis wird durch das Massenspektrum bestätigt: die Massendifferenz entspricht dem Phosphorylglycerin-Rest (jeweils im Vergleich zum betreffenden Spektrum des TEL). Die NMR- bzw. Massenspektren sind in den Abb. 4-7 gezeigt.

Die Hydrolyse von TEL bei tieferen Temperaturen bis 4°C liefert als Hauptprodukt (oder sogar ausschließlich) das Glykolipid; daraus läßt sich ein kinetisch kontrollierbarer Angriff an den polaren Kopfgruppen des TEL-Moleküls interpretieren. Dies wird bestätigt durch die Untersuchungen der Produktzusammensetzung bei Reaktionen, die bei höheren Temperaturen durchgeführt wurden. Wird die Reaktion bei Raumtemperatur durchgeführt, so erhält man beide Produkte (Glykolipid und Tetraetherlipid ohne polare Kopfgruppen) in annährend gleich hohen Anteilen. Ist die Reaktionstemperatur wesentlich höher als die Raumtemperatur, so liefert die Reaktion nach einigen Stunden fast ausschließlich ein Tetraetherlipid, bei dem beide polaren Kopfgruppen abgespalten wurden, als Produkt. Somit ist die Reaktionsgleichung für die Hydrolyse von TEL folgendermaßen darzustellen (s. auch Abb. 8):

Reaktionsgleichung

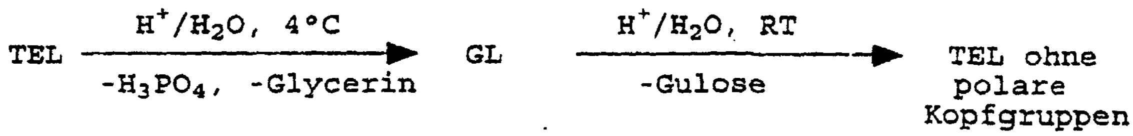


4.2 Acetylierung des Glykolipids (GL)

Zur Identifizierung des Kohlenhydrat-Restes mittels NMR-Spektroskopie wurde das durch Hydrolyse von TEL aus Thermoplasma acidophilum gewonnene Glykolipid (GL) peracetyliert.

Eine 7 mg GL-Probe (5 µmol) wird in ein Röhrchen mit Schraubkappe (Teflon-Dichtung) überführt und das Lösungsmittel im Stickstoffstrom verblasen. Zu der Probe werden jeweils 0,5 ml trockenes Pyridin und Acetanhydrid gegeben. Das Probenröhrchen wird mit der Kappe fest verschlossen, und die Reaktion wird anschließend in einem Thermoblock bei 105°C durchgeführt.

Der Verlauf der Reaktion wird durch Probenentnahme alle 30 min mittels DC kontrolliert. (HPTLC, Si-60, 5 x 5 cm, entwickelt in Chloroform/Methanol 9:1 (v/v), detektiert mit Methanol/Schwefelsäure 9:1 (v/v).) Nach ca. 2 Stunden ist die Reaktion vollständig abgelaufen. Die Reaktionslösung wird durch Verblasen im Stickstoffstrom getrocknet. Essigsäurereste werden nach Zugabe von 1 ml Toluol als Azeotrop durch Verblasen entfernt und das Reaktionsprodukt im Hochvakuum getrocknet. Die Ausbeute beträgt 5 mg an peracetyliertem Glykolipid. Ein $^1$H-NMR-Spektrum des peracetylierten Glykolipids ist in Abb. 1 wiedergegeben.

4.3 Oxidation von TEL mittels Periodsäure

Periodsäure ist ein spezifisches Oxidationsreagenz für vicinale Hydroxylgruppen, denn diese werden nur bis zur Carbonyl-Gruppe oxidiert. In Vorversuchen wurde festgestellt, daß die vicinalen Hydroxylgruppen des Glycerins schneller reagieren als die von Glukose, welches auf einen kinetisch kontrollierbaren Angriff an den Kopfgruppen des TEL-Moleküls hindeutet. Die Vorversuche haben ebenfalls ergeben, daß bei Reaktionstemperaturen um -18°C Glukose kaum oxidiert wird. Führt man also die Oxidation von TEL mittels Periodsäure bei tieferen Temperaturen aus, so sollte es möglich sein, das Produkt mit dem oxidierten freien Glycerin als Hauptprodukt zu erhalten. Diese Tatsache wird hier ausgenutzt, um dem Lipidmolekül eine reaktive funktionelle Gruppe einzufügen, welche wiederum für seitlich orientierte Reaktionen ausgenutzt werden kann.

In einer auf -20°C temperierten Lösung von 10 mg (6 µmol) TEL in 10 ml trockenem Pyridin werden unter Rühren ca. 3 mg (ca. 15 µmol) pulverisierte und bei 105°C getrocknete Periodsäure gegeben. Zur Weiterführung der Reaktion wird diese Lösung im Gefrierschrank bei -20°C temperiert und in periodischen Abständen geschüttelt. Der Verlauf der Reaktion wird mittels DC kontrolliert (HPTLC, Si-60, 5 x 5 cm, entwickelt in Chloroform/Methanol/Wasser 80:25:4 (v/v/v), detektiert mit Methanol/Schwefelsäure 9:1 (v/v) und Dinitrophenylhydrazin-Reagens). Nach 7 Tagen wird die Reaktion durch Zugabe einer Natriumbisulfit-Lösung abgebrochen. Die Produkte werden in einem Scheidetrichter mittels Chloroform/Methanol 3:1 (v/v) extrahiert. Die gesammelten organischen Phasen werden mit einer verdünnten Essigsäure-Lösung neutral gewaschen und über Natriumsulfat getrocknet. Das Lösungsmittel wird in einem

Rotationsverdampfer eingeengt und im Hochvakuum restlos entfernt. Die so gewonnenen Produkte werden unter Stickstoffatmosphäre bei -20°C bis zur weiteren Aufarbeitung bzw. Reaktion gelagert.

Der Reaktionsablauf ist unten schematisch gezeigt.

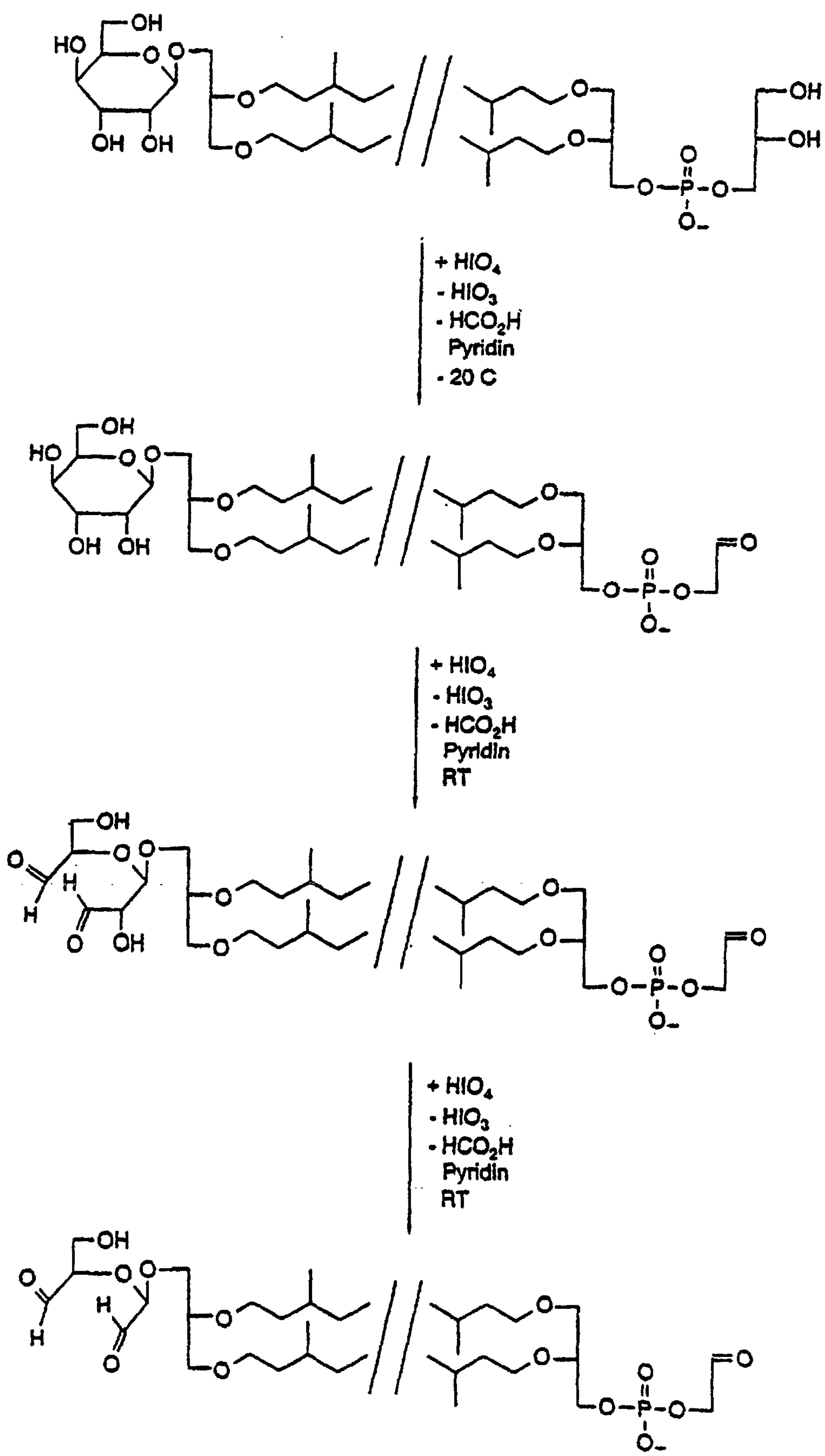
+ HIO4
- HIO3
- HCO2H
Pyridin
- 20 C
+ HIO4
- HIO3
- HCO2H
Pyridin
RT
+ HIO4
- HIO3
- HCO2H
Pyridin
RT

4.4 Beispiele für mögliche Modifikationen des erfindungsgemäßen TEL

Im folgenden werden mehrere mögliche Reaktionen zur Modifizierung des erfindungsgemäßen TEL dargestellt. Die Reaktionsschemata zeigen allgemein Möglichkeiten der Umsetzung des Tetraetherlipides mit anderen Verbindungsklassen, insbesondere mit Aminen und Carboxylgruppen-haltigen Verbindungen. Die Bedeutung von $R-NH_2$ umfaßt ein Amin (z.B. Aminosäuren, biogene Amine, Cholin, Catecholamine und andere Hormone, heterocyclische Amine, aminierte Zucker), ein Polyamin, ein Peptid oder ein Protein. R-COOH kann ebenfalls ein Peptid oder Protein bedeuten. Bei dem Rest R kann es sich um jede beliebige, in Verbindung mit einem Tetraetherlipid nützliche Substanz handeln, bevorzugt um ein Antibiotikum, ein Zytostatikum, einen Antikörper oder ein Antikörperfragment, ein Hormon oder einen anderen gewünschten Wirkstoff, ein an einer Oberfläche fixiertes Polymer oder eine (aminomodifzierte) Oberfläche. Der Rest R umfaßt Wasserstoff, Alkyl, Aryl oder Aralkyl, Halogen, Carboxyl, Alkyloxy, wobei jede dieser Gruppen substituiert oder unsubstituiert sein kann.

4.4.1 Schrittweise Modifikation des TEL aus Thermoplasma acidophilum (DSM 10217)

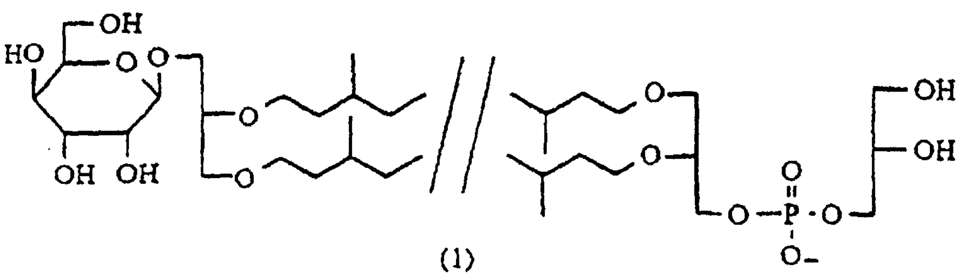

(1)

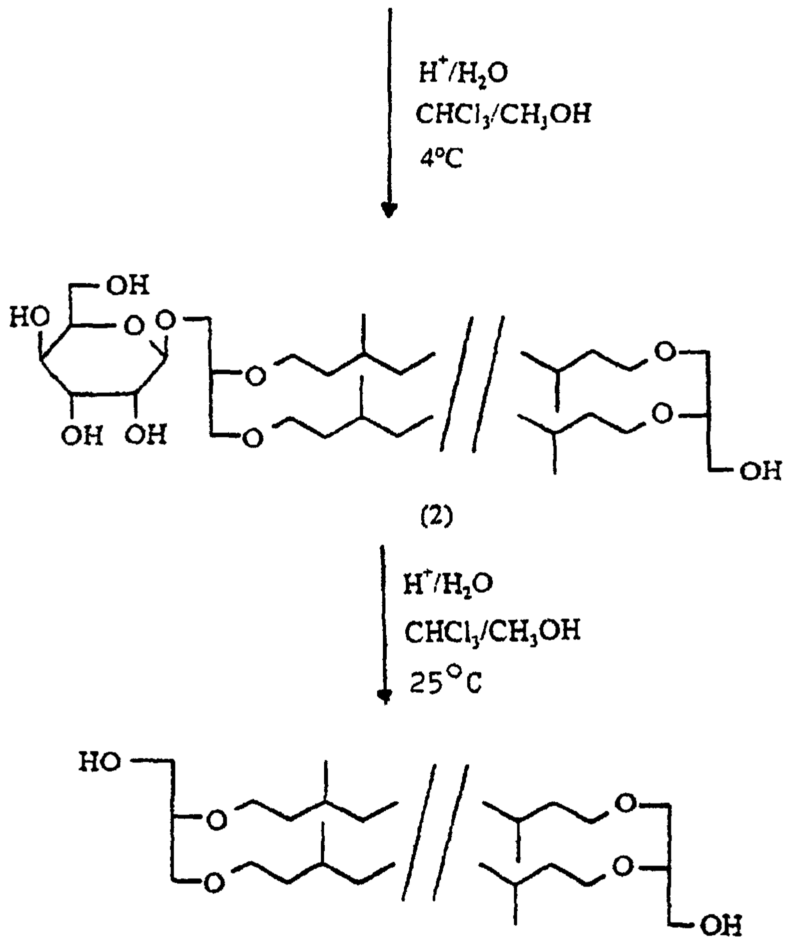


(2)

(3)

Literatur in dieser Umsetzung findet sich z.B. bei Chambers und Clamp [(1971) Biochem. J. 125, 1009-1018], sowie Mort und Lamport [(1977) Anal. Biochem., 82, 289-309].

4.4.2 Umsetzung des Tetraetherlipides aus Thermop1asma acidophilum (DSM 10217) mit einem Amin

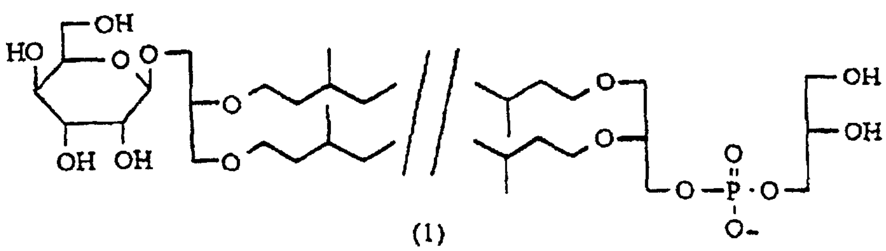

(1)

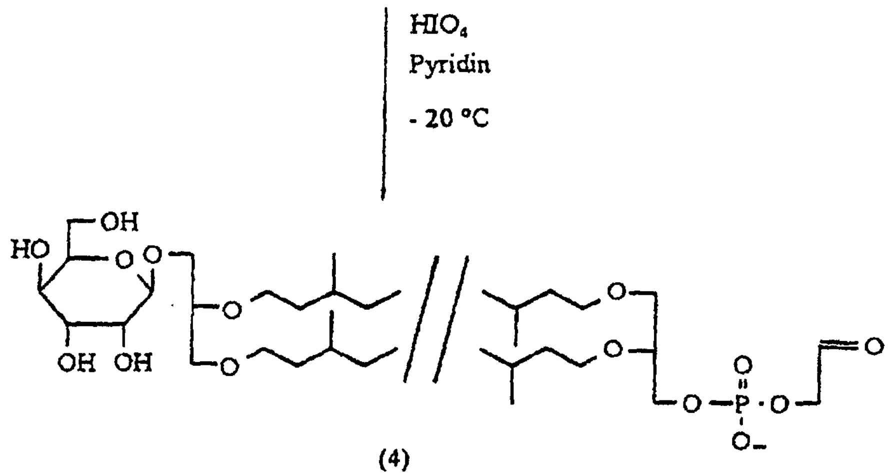


(4)

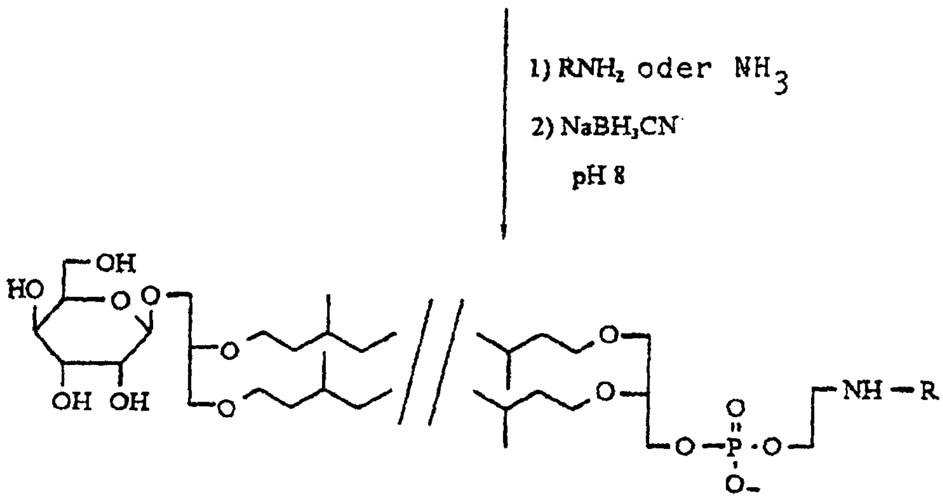


(5)

Die Bedeutung der Gruppe R ist bereits oben erläutert. Literatur zu dieser sowie der folgenden Umsetzung findet sich bei Baumann, Schmid und Mangold [(1961) J. Lipid Res., 10,132-133], Veh, Corfield, Sander und Schauer [(1977) Biochim. Biophys. Acta, 486,145-151], Heath, Macher und Papahadjopoulos [(1981) Biochim. Biophys. Acta, 640,66-81], Bogdanov, Klibanov und Torchilin [(1984) FEBS 175, 178-182], Chai, Stoll, Cashmore und Lawson [(1993) Carbohydr. Res., 239, 107-115], und Antonopoulos, Rothe, Bakowsky und Freisleben [(1993) Biol. Chem. Hoppe-Seyler, 374,143].

4.4.3 Umsetzung von Verbindung (2) mit einem Amin

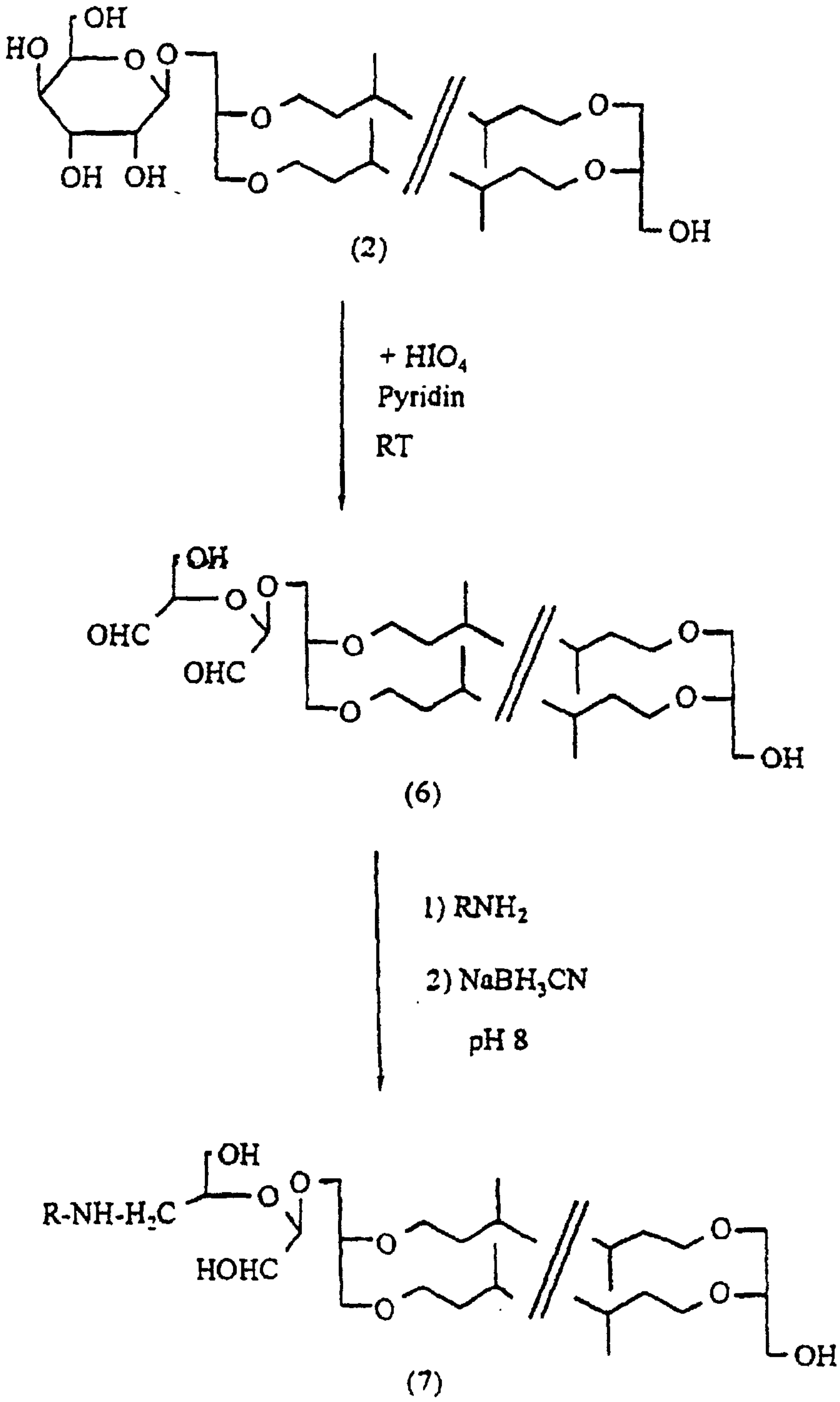


(2)

(6)

(7)

Die Bedeutung für R ist bereits oben angegeben. Literatur zum Thema siehe 4.4.2.

4.4.4 Umsetzung von Verbindung (3) mit Carboxylverbindungen

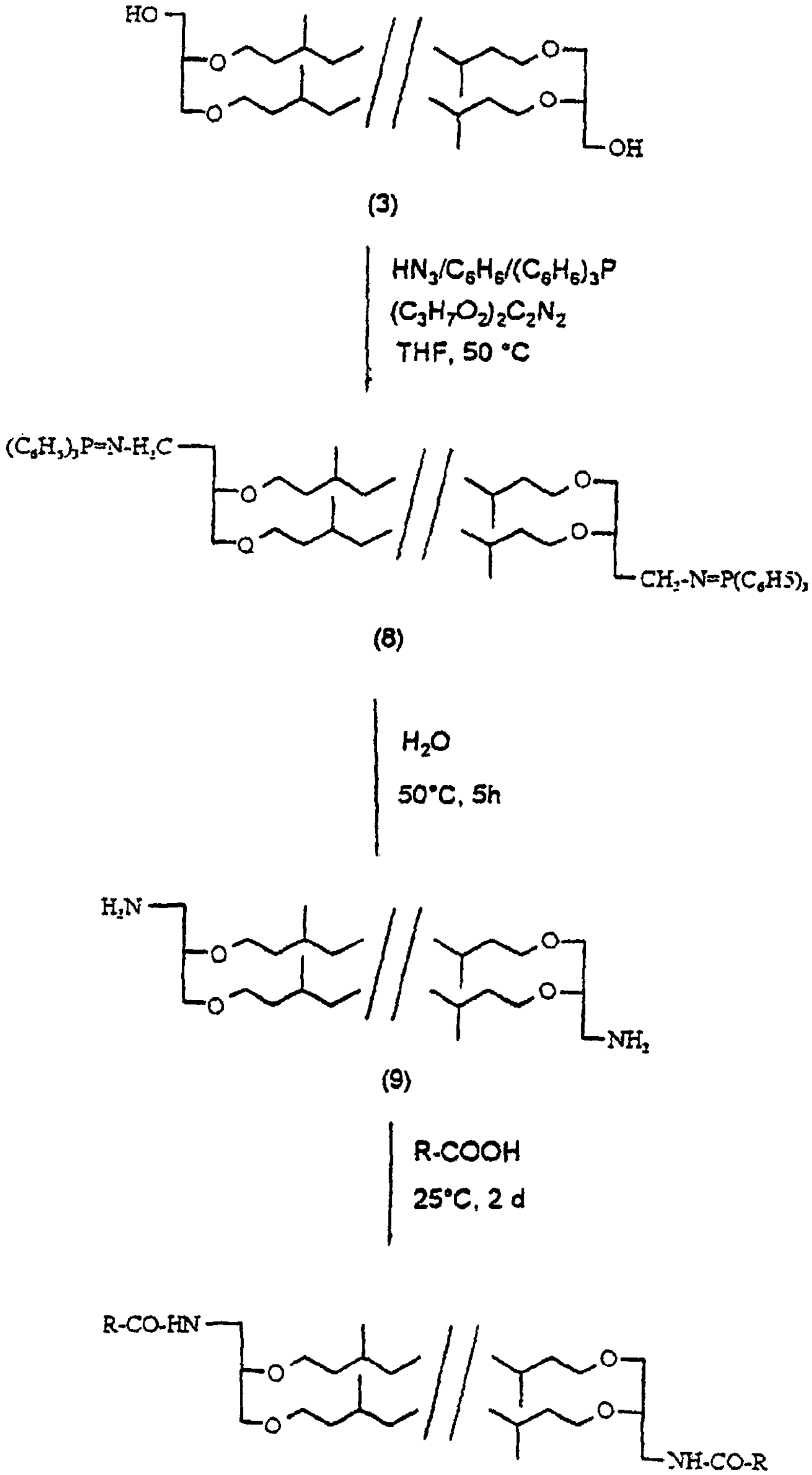


(10)

Die Bedeutung für R ist bereits oben angegeben. Literatur zu dieser Umsetzung findet sich bei Mitsunobu [(1981), Synthesis, 1-3], und Fabiano, Golding und Sadeghi [(1987), Synthesis, 190-192].

4.4.5 Umsetzung von Verbindung (3) mit einem Amin, Aktivierung mit Cyanurchlorid

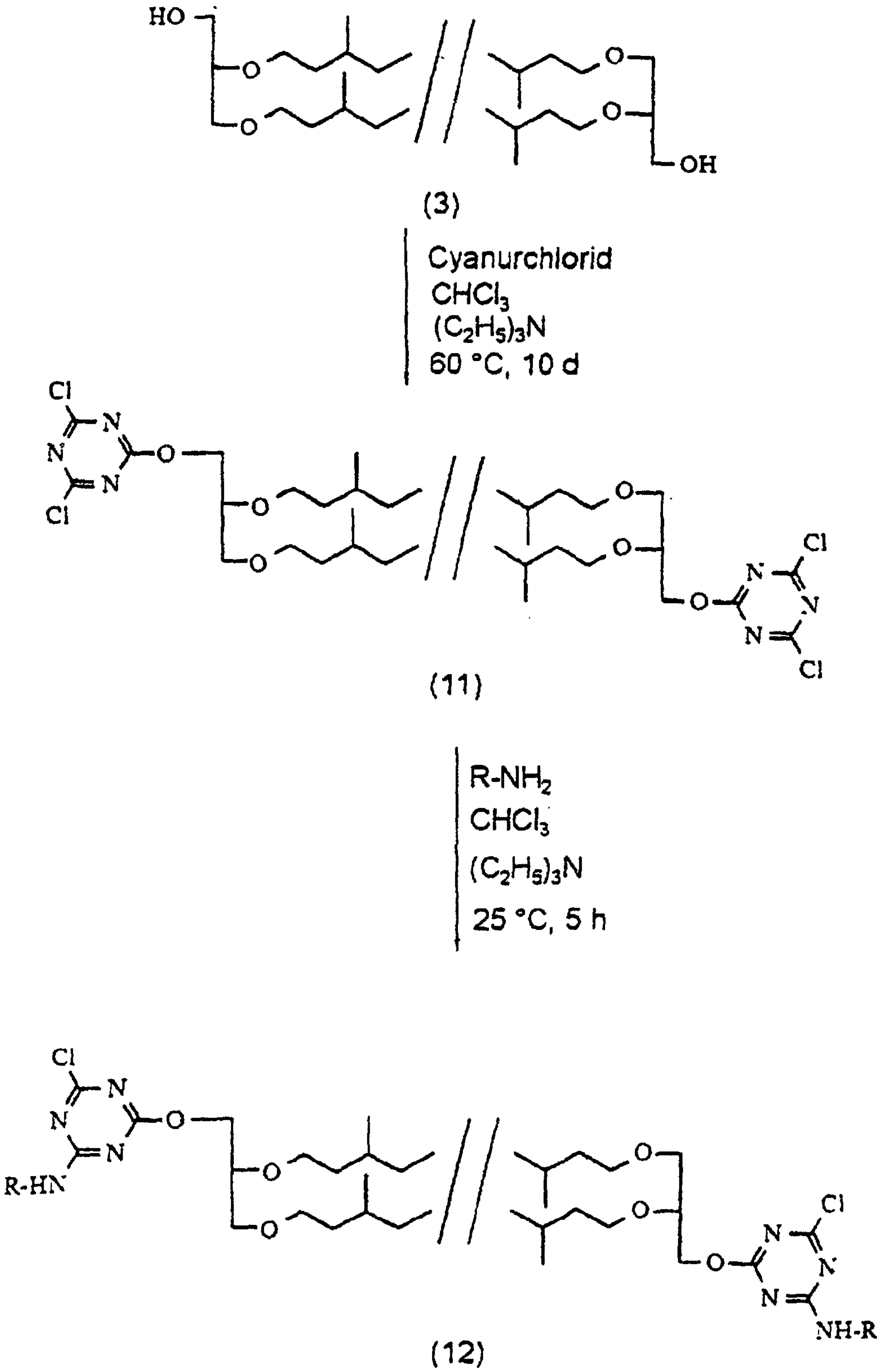


Die Bedeutung für R ist bereits oben angegeben. Literatur zu dieser Umsetzung findet sich bei Sandler [(1970),

J. Org. Chem. 35, 3697-3702], Potnaik [(1994), Biochem. Mol. Biol. Int., 33, 81-89], Ulbrich, Hubert, Dellacherie und Rivat [(1995), J. Mol. Recogn., 8, 11-115], und Li, Li und Lin [(1996), J. Chromatogr., 722, 211-220].

4.4.6 Umsetzung von Verbindung (3) mit einem Amin

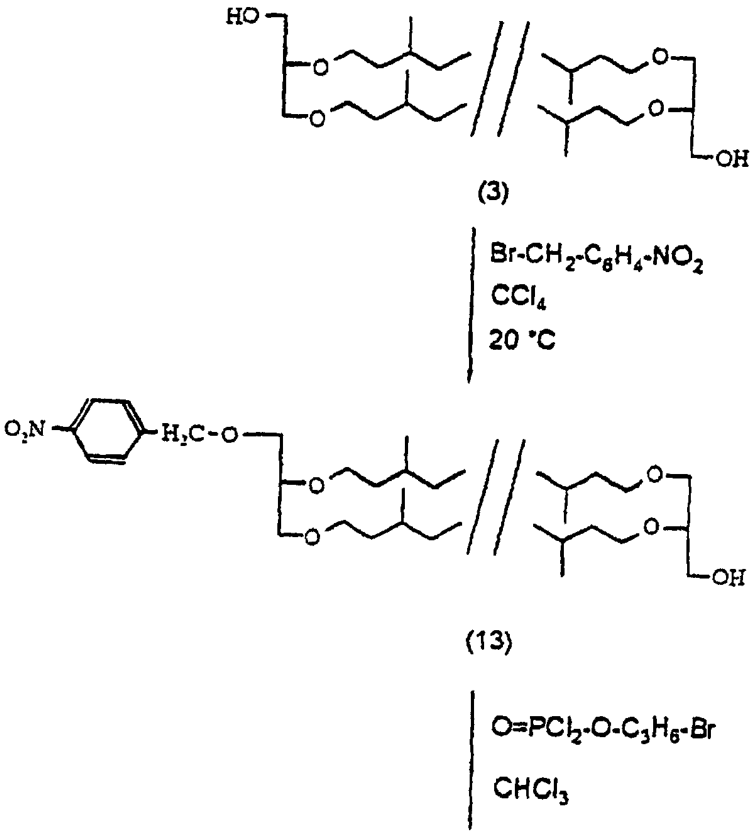


(3)

(13)

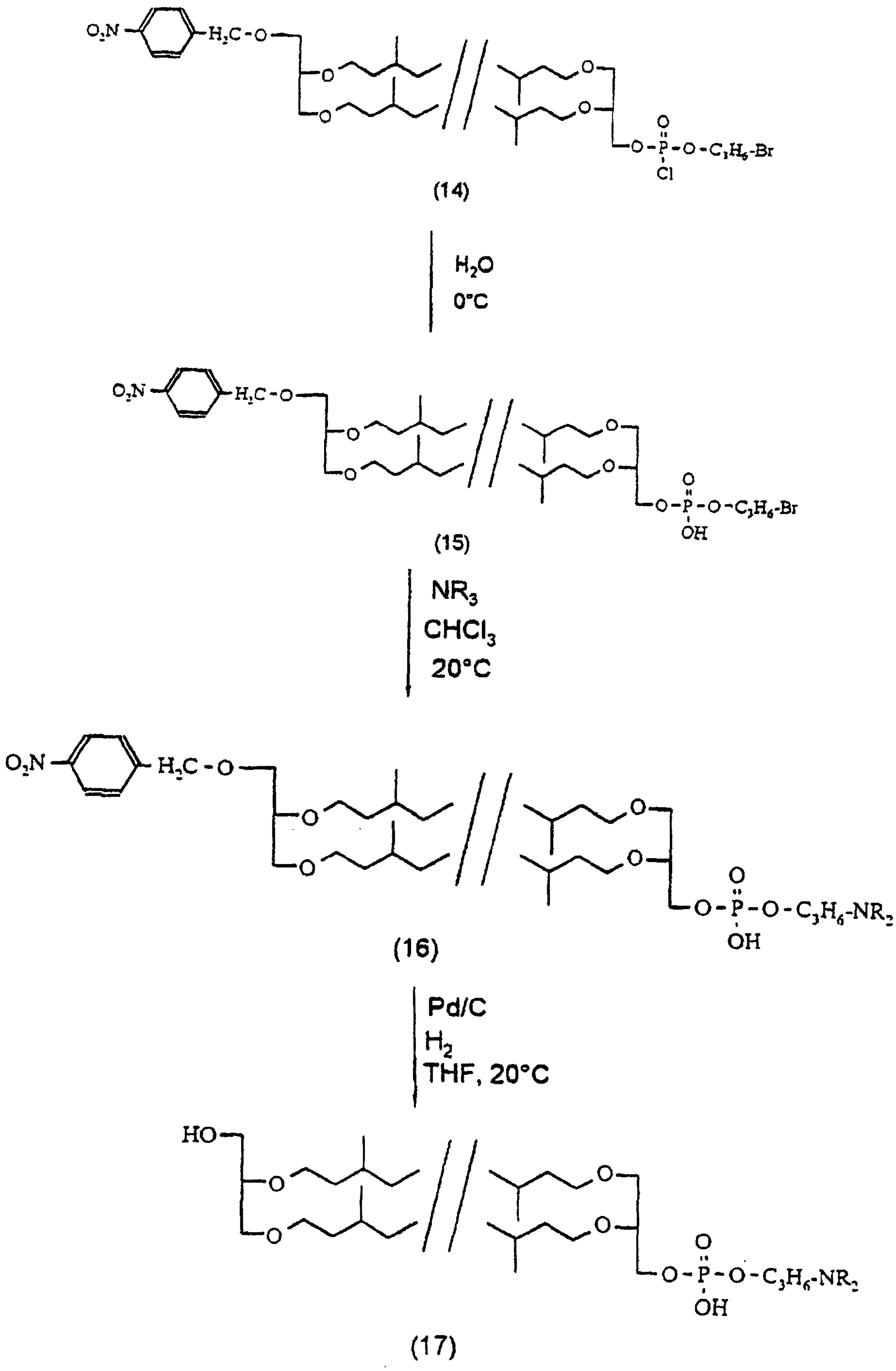


(14)

(15)

(16)

(17)

Die Bedeutung für R ist bereits oben angegeben. Bei $NR_3$ handelt es sich bevorzugt um ein Amin oder Polyamin. R kann gleich oder verschieden sein. Literatur zu diesen Reaktionen findet sich bei Eibl [(1980), Chem. Phys. Lipids, 26, 405-429 und die darin enthaltenen Zitate (131)], und Paltauf und Hermetter [(1994), Progress in Lipid Research, 33, 239-328].

4.4.7 Umsetzung von Verbindung (3) mit einem Amin

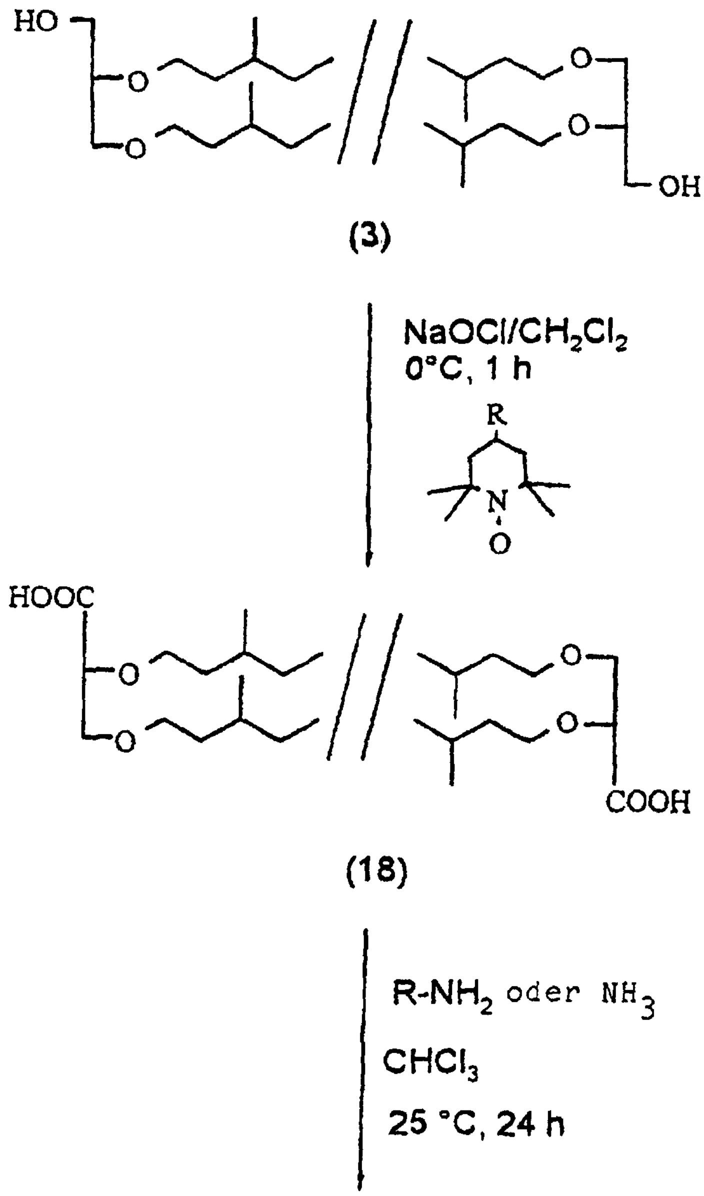


(3)

(18)

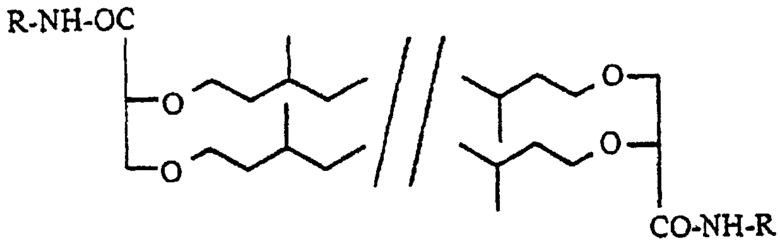

(19)

Die Bedeutung für R ist bereits oben erwähnt. Literatur zu dieser Reaktion findet sich bei
Anelli, Biffi, Montanari und Quici [(1987), J. Org. Chem., 52-2559-2562].

4.5 Oxidation von GL mittels Periodsäure

7 mg GL (5 μmol) werden in 10 ml trockenem Pyridin gelöst. Zu dieser Lösung werden unter Rühren ca. 6 mg (ca. 30 μmol) pulverisierte und bei 105°C getrocknete Periodsäure gegeben. Die Reaktion wird bei RT unter ständigem Rühren durchgeführt. Der Reaktionsverlauf wird mittels DC kontrolliert (HPTLC, Si-60, 5 x 5 cm, entwickelt in Chloroform/Methanol 15:1 (v/v), detektiert mit Methanol/Schwefelsäure 9:1 (v/v) und Dinitrophenylhydrazin-Reagens). Nach einem Tag ist die Reaktion abgelaufen, und die überschüssige Periodsäure wird durch Zugabe von 10 ml einer 30 μM Natriumbisulfid-Lösung reduziert. Das Oxidationsprodukt wird in einem Scheidetrichter zweimal mit jeweils 20 ml Petrolbenzin extrahiert. Die gesammelten organischen Phasen werden mit 10 ml einer stark verdünnten Essigsäurelösung zurückgewaschen, über Natriumsulfat getrocknet, anschließend im Rotationsverdampfer eingeengt, und im Hochvakuum werden die Lösungsmittelreste vollständig entfernt. Die Aufreinigung des oxidierten Produkts erfolgt über eine Kieselgelsäule. Als Laufmittel wird Chloroform/Methanol 25:1 (v/v) eingesetzt. Identifizierung: Dinitrophenylhydrazin-Reaktion auf Carbonylfunktionen (Reio, 1958).

**Beispiel 5**

**Liposomen**

5.1 Herstellung von Liposomen aus reinem TEL aus Thermoplasma acidophilum-DSM 10217

Unabhängig von der Präparationsmethode wird TEL in einem ausreichenden Volumen Chloroform/Methanol (2:1, v/v) gelöst (ca. 20 mg/ml). Der Rundkolben, in dem der Lipidfilm gebildet wird, sollte nur zu einem Drittel seines Volumens gefüllt sein. Das Solvens wird bei 40°C und vermindertem Druck (250 mbar) in einem Rotavapor (Büchi, Flawil, Schweiz) abgezogen. Der trockene Lipidfilm wird 12 Stunden bei Raumtemperatur in einem Hochvakuum gelagert, um sämtliche Lösungsmittelreste zu entfernen.

5.1.1 Präparation von Liposomen ohne Detergenzverwendung

Zur Liposomenpräparation wird TEL in einem gewünschten oder für einen bestimmten Zweck geeigneten Puffersystem suspendiert. Für medizinisch-pharmazeutische Zwecke eignet sich physiologische Kochsalzlösung, pH 7,4, jedoch lassen sich Liposomen aus TEL auch in anderen wäßrigen gepufferten (Beispiel McIlvaine Puffer) oder ungepufferten Lösungen (Beispiel ungepufferte Kalium- oder Natriumchloridlösungen) herstellen, da Unterschiede im pH, an Elektrolyten und in Elektrolytkonzentrationen die Eigenschaften des TEL wenig verändern. Die Puffermenge sollte so berechnet sein, daß sich später eine Liposomendispersion mit maximal 15-20 mg Lipid pro ml Puffer ergibt.
Durch Schütteln mit der Hand werden zunächst große multilamellare Vesikel mit einer Größenverteilung im Mikrometerbereich gebildet. Durch Verwendung von zwei Glaskügelchen und/oder eines Ultraschallbades geringer Schallintensität wird die Suspension des TEL beim Handschütteln erleichtert. Für die eigentliche Präparation von unilamellaren Liposomen definierter Größe wurden folgende Methoden getestet und als für die Herstellung von Liposomen aus TEL geeignet befunden:

(a) Ultraschallbehandlung: Die Suspension multilamellarer Liposomen wird bei 20 kHz 5 Minuten beschallt (Branson Sonifer B 15). Ein Eisbad und Inertbegasung sind beim Arbeiten mit reinem TEL nicht erforderlich, da keine

Oxidationsreaktionen auftreten können, Beschallung im Eisbad ist bei TEL jedoch ebenfalls erfolgreich. Es bilden sich Liposomen mit einem Durchmesser um 500 nm.

(b) Extrusion durch eine French Druckzelle: Die Suspension multilamellarer Liposomen wird bei 16000 p.s.i. viermal in der French Druckzelle extrudiert (SLM-Aminco Inc., Urbana IL, USA). Es bilden sich Liposomen mit einem Durchmesser um 120 nm.

(c) Extrusion durch Polycarbonatfilter: Die Suspension multilamellarer Liposomen wird durch Polycarbonatfilter definierter Porengröße extrudiert (LiposoFast$^{m}$, Avestin, Ottawa, Kanada). Bei Verwendung von Filtern mit einer Porengröße von 100 oder 200 nm entstehen Liposomen, die in der Größenverteilung geringfügig oberhalb der Porengröße liegen. Die Extrusion durch Filter dieser geringen Porengröße kann gleichzeitig als Sterilfiltration dienen, sofern alle sonstigen Voraussetzungen für eine Sterilfiltration (z.B. exakte Trennung der unsterilen und sterilen Seiten der Filtrationsapparatur) eingehalten werden. Um die eigentliche Filtration zu erleichtern, können einige Vorbereitungen getroffen werden:

(ca) Die Suspension multilamellarer Liposomen wird bei 20 kHz zwischen 1 und 5 Minuten beschallt (s. (a))
(cb) Die Suspension multilamellarer Liposomen wird 1-3 mal gefroren und wieder aufgetaut.
(cc) Die Suspension multilamellarer Liposomen nach (ca) oder (cb) wird durch einen Polycarbonatfilter von 600 bis 800 nm Porengröße vorfiltriert.

Im Anschluß an die Ultraschallbehandlung bzw. Extrusion werden die Liposomen in einer Eppendorfzentrifuge 3200 10 Minuten zentrifugiert. um nichtliposomales Material zu entfernen. Intakte, geschlossene Vesikel bleiben im Überstand.

5.1.2. Präparation von Liposomen durch Detergenzsolubilisierung mit anschließender Detergenzdialyse

Wie oben beschrieben wird zunächst ein Lipidfilm gebildet. Dieser wird in einem detergenzhaltigen Puffersystem suspendiert (Beispiele dialysierbarer Detergenzien: Octyl-β-D-Glucopyranosid oder Octyl-β-D-Thioglucopyranosid). Das molare Verhältnis (TEL: Detergenz) sollte bei den genannten Detergenzien zwischen 0,05 und 0,3 liegen und die Puffermenge so berechnet sein, daß sich später eine Liposomendispersion mit maximal 15-20 mg Lipid pro ml Puffer ergbibt. Durch Schütteln mit der Hand werden Mischmizellen aus Detergenz und TEL gebildet.

Die Suspension der Mischmizellen wird nun in Dialyseschläuche, in eine Lipoprep®-Dialysezelle oder in eine Mini-Lipoprep® Dialysezelle (Diachema AG, Langnau, Schweiz) übertragen und bei RT 24 Stunden dialysiert. Bei Entzug des Detergenz durch die Dialyse bilden sich aus den Mischmizellen Liposomen (Weder & Zumbühl, 1984) von etwa 400 nm Durchmesser.

Die Liposomen-Präparation wird in einer Eppendorfzentrifuge 3200 10 Minuten zentrifugiert, um nichtliposomales Material zu entfernen. Intakte, geschlossene Vesikel bleiben im Überstand.

5.2 Charakterisierung der TEL-Liposomen

5.2.1 Größenverteilung und Ladung der Liposomen:

Die Größenbestimmung bzw. die Bestimmung der Größenverteilung wird elektronenmikroskopisch und im Laser Particle Sizer (Malvern Autosizer II), die zeta-Potentialbestimmungen im Zeta Sizer PCS 1.1 (Malvern Instr., Malvern, UK) durchgeführt.

Tabelle 5

| Mittlere Größe reiner TEL-Liposomen | | |
|---|---|---|
| Präparationsmethode | mittlere Größe | (SD) |
| Handschütteln | 7500 nm | (2500 > 20000) |
| Ultrabeschallung | 500 nm | (± 100) |
| French Druckzelle | 120 nm | (± 40) |
| Polycarbonatfilter (Porengröße 200 nm) | 220 nm | (± 60) |
| Detergenzdialyse (Lipoprep) | 370 nm | (± 40) |

Die kleinsten Liposomen aus TEL, die mit der French-Druckzelle und Polycarbonatfiltern mit 100 nm Porengröße hergestellt werden können, haben einen Durchmesser von etwa 80 nm. Dies scheint die untere Größenlimitierung für TEL-Liposomen aufgrund des Krümmungsradius zu sein.

5.2.2 Bestimmung des zeta-Potentials:

Durch die negative Ladung des Phosphatrestes sind Liposomen aus reinem TEL ebenfalls negativ geladen. Die Bestimmung des zeta-Potentials wurde mit Liposomen mit 80 nm und 150 nm Durchmesser durchgeführt. Zeta-Potentialbestimmungen bei 137,4 Volt in 75 mM NaCl, 5mM Natriumphosphat, pH 7,4 (entsprechend einer zur Messung notwendigen Verdünnung der Liposomensuspension in physiologischer Kochsalzlösung mit destilliertem Wasser von 1:1) ergaben -50 mV. Bei den gegebenen Verhältnissen, die das zeta-Potential entscheidend beeinflussen, folgt daraus eine Elementarladung pro 14 bis 15 $nm^2$ auf der Liposomenoberfläche. Dies bedeutet, daß die Mehrzahl der TEL-Moleküle in der Liposomenmembran mit den Zuckerresten nach außen orientiert ist, etwa in einem Verhältnis von einer negativen Ladung des Phosphorsäurerestes pro 15-20 Lipidmolekülen mit Zuckerresten an der Membranoberfläche.

5.2.3 Gefrierbruchverhalten bei der Elektronenmikroskopie:

Die Zellmembran von Thermoplasma acidophilum und die Membran der TEL-Liposomen brechen nicht in der Membranebene (d.h. tangential wie alle Doppelschicht-Membranen), sondern senkrecht zur Membranebene, d.h. entlang der Achsen der membrandurchspannenden TEL-Moleküle.

Aus diesem Verhalten läßt sich eine mechanische oder physikalische Stabilität ableiten, die Doppelschichten nicht gegeben ist und bei technischem Einsatz von TEL zum Tragen kommt. Bei kovalenter Bindung an Matrixoberflächen ("Oberflächenbeschichtung", "Lipid-coating". s.u.) ist bei Doppelschichten üblicher Lipidfilme nur eine Schicht fixiert. Die äußere, nur über hydrophobe Wechselwirkungen zwischen den Kohlenwasserstoffketten assoziierte Schicht spreitet beim Transfer in andere Medien ab, d.h. solche Membranen sind nur sehr bedingt transferabel. Dieses Problem existiert nicht bei der Beschichtung mit bipolaren Tetraetherlipiden.

5.2.4 Ordnungsparameter der liposomalen Membran:

Die Ordnungsparameter wurden mit ESR Spin labels in einem Bruker B-R 70 Digital ESR-Spectrometer gemessen.

Tabelle 6

| Spin label | Ordnungsparameter (TEL) | Vergleich (Eilecithin) |
|---|---|---|
| 3-Doxylcholestan | 0,9 | 0,655 |
| 5-Doxylstearat | 0,781 | 0,644 |
| 16-Doxylstearat | 0,258 | 0,201 |
| 5-Doxyldekan | 0,187 | 0,130 |
| di-tert.-Butylnitroxid | 0,1 | 0,1 |

5.2.5 Lagerstabilität:

TEL-Liposomen (100% TEL) wurden in PBS (10 mg TEL/ml) bei 37°C, RT und im Kühlschrank (4°C) gelagert. Außerdem wurde die Stabilität der Liposomen bei höheren Lagerungstemperaturen untersucht. Die Größenverteilung wurde im Laser Particle Sizer gemessen (Ausgangswert: mittlerer Durchmesser der Liposomen 150 nm).

Tabelle 7

| Lagerungstemperatur (°C) | mittlerer Durchmesser (nm) | Lagerungsdauer (Wochen) |
|---|---|---|
| 4-8 | 155 | 109 |
| RT | 160 | 109 |
| 37 | 165 | 109 |
| 60 & 70 | 150 | 10 |
| 80 & 90 | 155 | 10 |
| 100 | 177 | 10 |
| 120 | nicht meßbar | 10 |

5.2.6 Stabilität der Liposomen und Freisetzung eingeschlossener Substanzen

Die Freisetzung von 6-Carboxyfluorescein (CF) wurde aus Liposomen gemessen, die durch Ultrabeschallung und/oder Extrusion durch Polycarbonatfilter hergestellt wurden. CF wurde 100 mM dem Puffer (PBS) bei der Liposomenbildung zugesetzt, nicht eingeschlossenes CF wurde durch mehrmalige Säulenchromatographie (Sephadex G 25) und Zentrifugation (2 Minuten bei 10000 UpM) abgetrennt (New, 1990).

**Tabelle 8**

CF-Freisetzung über 24 Stunden bei pH 7,4

| Temperatur | TEL (100%) | Vgl. Eilecithin |
|---|---|---|
| 4°C | 2,5 % | 9 % |
| 30°C | 3,0 % | 41 % |
| 50°C | 8,0 % | 100 % |

CF-Freisetzung bei verschiedenen pH-Werten innerhalb von 14 min

| pH | TEL | Vgl. Eilecithin |
|---|---|---|
| 6,1 | 0 % | 31,4 % |
| 6,6 | 2,1 % | 23,3 % |
| 7,0 | 4,2 % | 17,5 % |
| 7,4 | 5,8 % | 19,4 % |

(Methode nach Weinstein et al., 1984, Temperatur = RT, nach 15 Minuten wurden 2 % Triton X-100 zugegeben = 100 % Freisetzung)

Freisetzungsstudien wurden auch mit $^{125}I$-Insulin durchgeführt.

Die Freisetzung des Insulins aus TEL-Liposomen wurde über 7 Tage unter Variation folgender Parameter verfolgt.

1 - Temperatur: 8°C, RT, 37 und 47°C;
2 - pH: 3,3; 5,0; 7,4; 8,0; 8,5;
3 - Elektrolyte: NaCl, $MgCl_2$, $CaCl_2$;
4 - Osmolarität: PBS/NaPhosphat, McIlvaine, 150-350 mosmol.

Während herkömmliche Eilecithin-Liposomen insbesondere bei saurem pH innerhalb eines Tages eingeschlossenes Insulin praktisch vollständig freisetzen, beträgt die freigesetzte Insulinmenge bei TEL-Liposomen weniger als 50 % (unabhängig von den äußeren Bedingungen, abgesehen vom pH). Bei alkalischem pH von 8-8,5 steigt die Freisetzung aus TEL-Liposomen an und entspricht in etwa der herkömmlicher Liposomen. Bei einem pH von 5 sind nach 7 Tagen aus TEL-Liposomen nur 54 % Insulin freigesetzt, bei einem pH von 3,3 sind es 65 %.

Die Stabilität der Liposomen gegenüber mehreren Detergenzien und Alkoholen wurde ebenfalls untersucht. Die Ergebnisse sind in der folgenden Tabelle 9 wiedergegeben:

Tabelle 9

| Konzentrationen an Alkoholen (%) und an Detergenzien (mM), die 50 % CF freisetzen. | | |
|---|---|---|
| Alkohol/Detergenz | TEL-Liposomen | Eilecithin-Liposomen |
| Methanol | 35 % | 25 % |
| Ethanol | 30 % | 15 % |
| n-Propanol | 13 % | 7 % |
| Triton X-100 | 0,01 % | 0,002 % |
| n-Octylglucosid | 12 mM | 8 mM |
| Desoxycholat | 0,7 mM | 0,2 mM |
| SDS | nicht bestimmbar | 0,5 mM |

Unter den Versuchsbedingungen setzte Natriumdodecylsulfat (SDS) nicht mehr als 10 % CF aus TEL-Liposomen frei, es ließ sich also keine halbmaximale Freisetzung messen.

Der Einfluß der Gallensäurekonzentration auf die Liposomengröße ist aus der folgenden Tabelle ersichtlich:

Tabelle 10

| Liposomen aus | Gallensäurekonzentration | | |
|---|---|---|---|
| | 0 | 10 mM | 30 mM |
| TEL | 133,1 | 109,3 | 118,3 |
| Eilecithin | 140,6 | 38,4 | 1302,6 |

Die hauptsächlichen physiologischen Gallensäuren sind Chenodesoxycholsäure/Cholsäure/Desoxycholsäure, die im Verhältnis 2:2:1 und einer Gesamtkonzentration von etwa 10 mM im Duodenum vorliegen. Die experimentellen Bedingungen sollten diese Verhältnisse simulieren. Obwohl hier der Eindruck entsteht, Gallensäuren hätten auch in sehr hohen, unphysiologischen Konzentrationen keine Wirkung auf TEL-Liposomen, deuten die parallel gemessenen und mit den Gallensäurekonzentrationen variierenden Ordnungsparameter an, daß durchaus auch die TEL-Membran von Gallensäuren beeinflußt wird. Weiterhin zeigt das Freisetzungsexperiment für Desoxycholsäure, daß 0,7 mM ausreichen, 50 % CF freizusetzen.

5.2.7 Protonenpermeabilität

Die Membran der TEL-Liposomen ist um 2 Zehnerpotenzen weniger protonendurchlässig als Eilecithinmembranen. Dies bezieht sich auf RT, die Differenz nimmt bis 74°C (so weit wurde gemessen) zu, da sich die Permeabilität bei TEL-Liposomen kaum erhöht, bei Vergleichsliposomen jedoch um ein Vielfaches zunimmt. Somit bilden die Liposomenmembranen aus TEL die protonendichtesten bekannten Membranen aus einem natürlichen Konstituenten.

5.3 Vergleich der Affinität von TEL-Liposomen und Phosphatidylcholin(PC}-Liposomen zu verschiedenen Zellinien

Die Affinität von TEL-Liposomen zu verschiedenen Zellinien wurde mit der von herkömmlichen Phosphatidylcholin(PC)-Liposomen verglichen. Die relative Affinität wurde ermittelt durch den Vergleich der Menge des von den Zellen aufgenommenen Fluoreszenzfarbstoffes Carboxyfluorescein, der eingekapselt in TEL- bzw. PC-Liposomen angeboten wurde. Die TEL-Liposomen wurden gemäß Beispiel 5.1-I.c bzw. 5.2.6 hergestellt, jedoch wurde anstatt einer 100 mM Carboxyfluoresceinlösung eine 50 mM Lösung verwendet. Für die Herstellung der PC-Liposomen wurden die gleichen Vorschriften unter Verwendung von Phosphatidylcholin (Sigma, Deisenhofen; 99 % rein) anstelle von Tetraetherlipiden angewendet.

Die Untersuchung wurde an Hep-GII-Zellen (Hepatokarzinom), CHO-Zellen (Chinese Hamster Ovary), CF-Pac-Zellen (Pankreaskarzinom) und HT29-Zellen (Colonkarzinom) durchgeführt. Die Zellen wurden in einem FCS-freien Medium bis zu einer Dichte von ca. $10^5$ Zellen pro ml Medium kultiviert. Anschließend wurden die Carboxyfluorescein-haltigen Liposomen zugefügt, wobei folgende Lipidkonzentrationen eingesetzt wurden: 0,5 mg/ml, 1 mg/ml, 2 mg/ml und 4 mg/ml. Die Zellen wurden anschließend weitere 4,5 Stunden inkubiert.

Nach Ablauf der 4,5 stündigen Inkubationsperiode wurden die Zellen dreifach mit je 1 ml PBS-Puffer, pH 7,4, gewaschen, in 1 ml Puffer sehr vorsichtig aufgenommen und in eine Küvette überführt. Die Menge des von den Zellen aufgenommenen Carboxyfluoresceins wurde mittels Fluoreszenzspektroskopie quantitativ ermittelt (Shimadzu RF

5000; Anregung: 490 mm, Emission: 530 nm).

Die Ergebnisse der Versuche sind in den Abbildungen 9a-d gezeigt. Die Abbildungen zeigen, daß aus den TEL-Liposomen deutlich mehr Carboxyfluorescein in die Zellen aufgenommen wurden, als aus den PC-Liposomen.

5.4 Herstellung von TEL enthaltenden Mischliposomen

TEL läßt sich mit anderen Etherlipiden und mit Esterlipiden (Eilecithin, DPPC) und auch Cholesterin mischen. Abhängig vom Mischungsverhältnis bilden sich stabile Mischliposomen. Die Stabilität nimmt generell bei geringen TEL-Anteilen bis 25 % des Gesamtlipides im Vergleich zu reinen Lecithinliposomen zu, mit höheren Anteilen an TEL jedoch wieder ab. Bei TEL-Anteilen von über 50 % kann es zur Trennung in verschiedene Populationen kommen. Zur Herstellung von Mischliposomen werden die Lipide (z.B. Eilecithin und TEL im gewünschten Verhältnis) im organischen Lösungsmittel wie bei der Herstellung reiner TEL-Liposomen gelöst und das Lösungsmittel abgezogen. Der Film aus den gemischten Lipiden wird wie bei der Herstellung von TEL-Liposomen weiter behandelt. Bei der Detergenzmethode unter Verwendung von n-Octylglucosid sollten bei TEL-Anteilen bis 25 % TEL Lipid: Detergenz-Verhältnisse zwischen 0,1 und 0,2 eingehalten werden. Bereits bei geringen TEL-Anteilen können die Mischliposomen beträchtlich größer werden als reine Lecithinliposomen unter vergleichbaren Bedingungen (vor allem der Herstellungsmethode). Teilweise werden Mischliposomen auch größer als reine TEL-Liposomen unter vergleichbaren Bedingungen.

Der Zusatz von TEL erhöht die Stabilität von Eilecithinliposomen. Die Stabilität wurde bis zu 88 Wochen untersucht. Über diesen Zeitraum veränderten Mischliposomen mit bis zu 25 % TEL ihre Größe um maximal 25 %. Reine Eilecithinliposomen zeigen innerhalb von 2-5 Wochen stärkere Größenzunahme.

**Beispiel 6**

**Pharmazeutische Anwendung der Liposomen**

Reine MPL-Liposomen können als Transportmittel durch den Magen-Darm-Trakt und in der Blase eingesetzt werden, z.B. zur Behandlung dort lokalisierter Tumoren von der Lumenseite. In den sauren Kompartimenten Magen und Blase schützen die säurestabilen TEL-Liposomen die Wirkstoffe, mit denen sie beladen sind. Die Freisetzung des jeweiligen Wirkstoffs erfolgt entweder durch Aufnahme der gesamten Vesikel in die Tumorzelle oder durch Intermembranaustausch, d.h. lipophile Wirkstoffe werden direkt von der liposomalen auf die Tumorzellmembran übertragen. Eine hohe Affinität von TEL-Liposomen zu Colon- und Blasenkarzinom-Zellinien konnte bereits nachgewiesen werden. Somit ergibt sich die Möglichkeit, liposomal eingeschlossene Chemotherapeutika oder Inhibitoren von Wachstumsfaktoren zur lokalen Tumortherapie einzusetzen mit dem Ziel, nur Tumorzellen zu behandeln, gesunde Zellen zu schonen und insbesondere systemische Nebenwirkungen zu vermeiden. Die hohe Affinität von TEL-Liposomen zu Tumorzellen kann durch Konjugation der Liposomen mit tumorspezifischen Antikörperfragmenten (Fab-Fragmenten) oder tumorspezifischen Liganden (z.B. Wachstumsfaktoren, IGF-I) über deren spezifische Rezeptorbindung (z.B. IGF-I-Rezeptor) verstärkt werden.

Die Aufnahme Cytostatika-haltiger TEL-Liposomen in Tumorzellen wird durch Oberflächenmodifikation mit Apoproteinanalogen Fragmenten, z.B. ApoE, β-Amyloid, optimiert.

Es empfiehlt sich, TEL-Liposomen u. U. durch Zusatz anderer Komponenten, z.B. Lecithin, etwas zu destabilisieren, um hydrophile Wirkstoffe leichter freizusetzen. Die erfindungsgemäßen Mischliposomen bedürfen dieser Destabilisierung im Einklang mit bisherigen Ergebnissen nicht, da sich je nach dem medizinisch-pharmazeutischen Verwendungszweck bzw. je nach Zusammensetzung (Mischungsverhältnis) beliebige Übergänge von instabilen Lecithin-Liposomen zu stabilen TEL-Liposomen erreichen lassen.

Im Darm werden TEL-Liposomen durch Gallensäuren und durch das alkalische Milieu destabilisiert. Die Zerstörung der Liposomen ist auch hier nicht so massiv wie bei Lecithin, der Stabilitätsvorteil schrumpft jedoch erheblich. Dies kann genutzt werden, um Wirkstoffe im Darm freizusetzen.

Bezogen auf das Intestinum werden zwei Ziele verfolgt:

(a) Lokale Behandlung von der Lumenseite (vergleichbar Magen und Blase), z.B. bei Coloncarcinom (hohe Affinität von TEL-Liposomen zu CoCa-Zellinien)

(b) Resorption von TEL-Liposomen aus dem Darmlumen (Liposomen unter oder um 100 nm sollen das Darmepithel permeieren, größere gehen wahrscheinlich durch die sog. "Payers patches" (gap junctions).

Die erfindungsgemäßen Liposomen können z.B. mit Insulin, Wachstumshormonen, Interleukinen, säurelabilen Gerinnungsfaktoren, Immunglobulinen, Impfstoffen etc. beladen werden.

Wegen der hohen Anreicherung in der Leber und wegen hoher Affinität zu HepG2-Zellen ist die Anwendung

reiner TEL-Liposomen zur Behandlung des hepatocytären Leberzellkarzinoms vorgesehen. Bei ausreichender Resorption aus dem Intestinum kann diese Behandlung oral durchgeführt werden, ansonsten intravenös (i.v.).

Die rasche Anreicherung der TEL-Liposomen in Leber und Milz nach intravenöser Verabreichung an Mäuse belegt eine hohe Affinität zum retikuloendothelialen System und ermöglicht, diese Liposomen zur Modulation des Immunsystems einzusetzen. Hierbei können die Liposomen Träger für Arzneistoffe zur Behandlung dieses Systems ein, für Immunstimulantien oder Impfstoffe oder auch für Immunsuppressiva, z.B. zur Verhinderung von Abstoßungsreaktionen nach Organtransplantation. In letzterem Fall können sowohl lipophile wie hydrophile Formen von Immunsuppressiva erfolgreich eingesetzt werden, z.B. Methylprednisolon oder dessen wasserlösliches Natrium-Hydrogensuccinat-Derivat. Das lipophile Methylprednisolon hat gegenüber dem wasserlöslichen Derivat die Vorteile der einfacheren liposomalen Verarbeitung und der erleichterten Freisetzung durch Intermembranaustausch auf die Membranen immunkompetenter Zellen, ohne daß die gesamten Vesikel in das Zellinnere aufgenommen werden müssen. Liposomales Methylprednisolon wurde bereits bei tierexperimentellen Herztransplantationen eingesetzt. Derzeit werden verschiedene Formen Methylprednisolon- und TEL-haltiger Liposomen und verschiedene Applikationswege (oral, intravenös) für diese Liposomen zur immunsuppressiven Therapie bei tierexperimentellen Nierentransplantationen getestet.

Eine weitere Möglichkeit zur Verwendung der erfindungsgemäßen Liposomen liegt in ihrem Einsatz zur Gentherapie. Grundsätzlich ist neben viraler Vektortransfektion auch liposomale DNA- und RNA-Transfektion gelungen. Selektiv können einzelne humane oder tierische Gene mittels DNA-Vektoren in TEL-haltige Liposomen verpackt werden und somit unspezifisch oder durch Konjugation der Liposomen (s.o.) zielgerichtet in Targetzellen transfiziert werden. Andererseits dient TEL zur Stabilisierung instabiler DNA-Transfektionskomplexe mit stark positiv geladenen lipidähnlichen Molekülen (z.B. Polyaminen). Eine weitere Möglichkeit besteht im Transfer von antisense c-DNA oder RNA-Fragmenten zur Abschaltung einzelner zellspezifischer Gene.

Virale DNA-Vektoren für β-Galaktosidase und Urokinase wurden in TEL-Liposomen verpackt und mit Cos 7-Zellen in Gegenwart von 3 % Lipofektin inkubiert. Dabei zeigte sich eine erfolgreiche Transfektion sowohl bei reinen TEL-Liposomen als auch bei Mischliposomen aus Eilecithin/TEL mit 1 %, 5 % und 10 % Gewichtsanteilen von TEL. Gemessen wurde die Urokinase-Aktivität colorimetrisch über die Blaufärbung der Zellen und die β-Galaktosidaseaktivität mittels 5-Brom-4-chlor-3-indolyl-β-D-galaktosidase. Auch bei dieser Reaktion zeigt sich eine erfolgreiche Transfektion durch Blaufärbung der Zellen.

**Beispiel 7**

Bildung planarer Membranen

Die Bildung planarer Membranen ist grundsätzlich mit TEL möglich als sog. Black Lipid-Membran oder an der Grenzfläche zwischen Wasser bzw. Puffer und Luft z.B. auf dem Langmuir-Trog. Die Übertragung vom Langmuir-Trog auf feste Matrix-Unterlagen (analog Langmuir-Blodgett-Verfahren) oder direkt nach dem sog. Self-Assembly-Verfahren ist ebenfalls gelungen. Derartige Membranen stellen für sich oder auf festen Unterlagen Modelle für biologische Separations- und Permeations-Systeme dar. Wie herkömmliche Phospho- oder sonstige Lipide bilden TEL auf nichtporösen, glatten Oberflächen defektfreie, ultradünne Schichten aus. Mit herkömmlichen Lipiden ergibt sich normalerweise das Problem einer defektfreien Beschichtung, wenn die Matrixoberfläche rauh oder porös ist. Porenbereiche werden nicht bzw. nicht defektfrei überspannt. Das erfindungsgemäße TEL und ähnlich strukturierte Tetraetherlipide bieten hier wesentliche Vorteile, da sie eine bipolare Monoschicht ausbilden, die außerdem inert, ausreichend fluid und oxidations-, thermo-, säure-, und hydrolysebeständig sind. Die Kombination dieser chemisch ungewöhnlich inerten Lipide mit hoher Tendenz zur Selbstorganisation und neuartiger Konzepte zur Erzeugung von supramolekularen Systemen mit gezielter Mikrostrukturierung stellt einen neuen Ansatz zur Beschichtung von mikroporösen Substraten dar und bietet Möglichkeiten zur Lösung folgender bekannter, aber bisher nicht befriedigend gelöster Probleme: Stabilität, Homogenität, Steuerbarkeit der lateralen Organisation, selektive und gerichtete Inkorporation von Funktionsmolekülen in die Matrix. Damit können im Grenzbereich zwischen anorganischen und organischen Strukturen folgende Prinzipien verbunden werden:

| Stützmatrix | Lipidschicht |
|---|---|
| anorganisch | organisch |
| starr | selbstassoziierend |
| strukturierbar | supramolekular |
| im | trigger- und funktionalisierbar |
| Mikrometerbereich mit ko- | im Nanometerbereich mit |
| valenter Strukturfixierung | nichtkovalenter Strukturierung |

Neben der konventionellen Lipidbeschichtungstechnik können auch neuere Konzepte wie "Tethered supported lipid" und "Chelatorlipid" mit Tetraetherlipiden ausgeführt werden, auf die hier im einzelnen nicht eingegangen werden soll, da sie keine für Tetraetherlipide spezifischen Techniken darstellen. Das Ergebnis einer bipolaren Lipid-Monobeschichtung ist neuartig und stellt für sich eine Oberflächenmodifikation dar.

Darüber hinaus können in diese Membranschicht natürliche oder synthetische Transport-, Kanal-, Signaltransduktions- oder sonstige funktionelle Moleküle (z.B. auch integrale oder assoziierte Proteine, Enzyme) inkorporiert werden. Dabei ist an lichtgetriebene Ionenpumpen wie Halorhodopsin, Protonenpumpen wie Bakteriorhodopsin oder natürliche und (semi)-synthetische Kanalmoleküle, Iono- und Protonophoren wie Valinomycin, Alamethicin, Nonactin, Mellitin, Gramicidin oder spezifisch modifizierte Cyclodextrine, Kronenether und Calixarene gedacht.

Der Arbeitsablauf gliedert sich wie folgt:

a) Isolierung, Reinigung und Funktionalisierung des bipolaren Tetraetherlipids
b) Transfer des bipolaren Lipids auf die Trägerunterlage
c) Mikrostrukturierung der lipidbeschichteten Matrix
d) Einbau von Proteinen oder Kanälen etc.
e) Charakterisierung der Separations- und Permeationseigenschaften
f) und Verwendung des asymmetrischen Membransystems.

a) Isolierung, Reinigung und Funktionalisierung des Lipids sind in den Beispielen 2 und 4 beschrieben. Die Funktionalisierung erfolgt gemäß der Oberfläche der anorganischen Matrixunterlage und der Beschichtungstechnik; für das Chelatorkonzept muß eine SH-Gruppe in eine Kopfgruppe des Lipids eingeführt werden, für das "tethered supported monolayer"-Konzept muß ein flexibles, hydrophiles Polymer angebunden werden.

b) Der Transfer des bipolaren Lipids auf die Trägerunterlage kann durch Langmuir-Blodgett-Technik erfolgen oder alternativ über einen Self-Assembly-Prozeß. Die Adsorption des Lipids an die Trägeroberfläche soll durch kovalente Kopplung unterstützt und dauerhaft stabilisiert werden. Für die kovalente Kopplung ist eine Funktionalisierung sowohl der Oberfläche der Matrix als auch des Lipids (s. oben) notwendig. Diese Funktionalisierung (Aktivierung) ist für beide Reaktanden vorher, während des Transferprozesses oder nachträglich möglich.

c) Mikrostrukturierung der lipidbescbichteten Matrix wird durch klassische mikrotechnische Verfahren (z.B. Herausätzen von Poren gewünschter Größe etc.) erfolgen und muß den speziellen Bedürfnissen der lipidbeschichteten Trägermaterialien angepaßt sein, d.h. die Lipidbeschichtung darf nicht zerstört werden. Auch hier bieten TEL und seine Derivate durch ihre Stabilität Vorteile gegenüber bislang eingesetzten Lipiden.

d) Der Einbau von Proteinen und Ionenkanälen kann grundsätzlich während des Prozesses der Lipidbeschichtung oder nachträglich erfolgen. Als Techniken stehen die direkte Insertion der detergenzsolubilisierten Membranproteine oder Ionophoren etc. oder der primäre Einbau derselben in Liposomen und Übertragung daraus auf die planare Membran zur Verfügung. Die Liposomentechnik erlaubt wiederum zwei Wege, erstens die Fusionstechnik und zweitens bei nachträglichem Einbau in die Träger-fixierte Lipidschicht, die Liposomenabrolltechnik. Bacteriorhodopsin und Halorhodopsin werden möglichst gerichtet in die Membran eingebaut.

e) Charakterisierung der Separationseigenschaften bezüglich einer praktischen Nutzung der erhaltenen Systeme: Die Funktionsprüfung soll im Hinblick auf lichtgetriebene Wasserentsalzung mit getriggertem Chloridtransport gegen einen Konzentrationsgradienten überprüft werden und bezüglich eines für die Gewinnung von chemischer

Energie ausnutzbaren Protonengradienten und selektive Ionenpermeabilität durch Einbau von Ionophoren, z.B. Kronenethern, erreicht und überprüft werden.

Matrix-Substrate (Trägerfolien) können z.B. Unterlagen aus photostrukturierbarem Glaskohlenstoff (Foturan), Silizium, Zellulose oder Keramikmaterial sein, die die zur Funktionalisierung notwendigen OH-Gruppen an der Oberfläche tragen. Die Mikrostrukturierung der Unterlagen erfolgt durch Photolithographie (Glas) oder anisotropes Ätzen. Die Verwendung der stabilen Tetraetherlipide ermöglicht auch die Umkehr der Reihenfolge der Lipidbeschichtung und Mikrostrukturierung:

Der konventionelle Weg besteht darin, daß ein bereits mikrostrukturiertes, poröses Substrat lipidbeschichtet wird. Falls die Poren nicht überspannt werden sollen, kann nachträglich Lipid eingefüllt werden, z.B. durch "Abrollen" von Liposomen. Dies kann mit dem ursprünglich zur Beschichtung verwandten TEL erfolgen oder mit einem anderen, mit TEL mischbaren herkömmlichen Esterlipid, mit einem Dietherlipid oder mit einem weiteren Tetraetherlipid. Die kovalente Bindung der Lipide an die Trägerunterlage kann direkt erfolgen oder über flexible, polymere Ethylenoxid-Spacer, wodurch eine gewisse räumliche Entkopplung der Membranschicht vom Substrat erreicht wird. Dies erleichtert sowohl den Selbstorganisationsprozeß der Membranschicht als auch die spätere Insertion integraler Membranproteine.

Der alternative Prozeßablauf in der umgekehrten Reihenfolge umfaßt zunächst die Beschichtung nichtporösen Substrats, die defektfrei erfolgt, da keine Poren überspannt werden müssen. Voraussetzung ist lediglich, daß die Unebenheiten der Oberfläche nicht so gravierend sind, daß sich kein einheitlicher Beschichtungsfilm bilden kann. Anschließend erfolgt die Mikrostrukturierung, d.h. es werden nur die definierten Poren in.das Substrat geätzt. Dieser Prozeß kann weiter modifiziert werden, indem die Trägerunterlage anisotrop von einer Seite angeätzt und von der anderen, noch intakten Seite beschichtet wird. Nach der Beschichtung werden die Poren "durchgeätzt". Die beim nachträglichen Ätzen auch der Membran zugefügten Schäden werden ebenfalls durch "Abrollen" von Liposomen ausgebessert (s.o.). Der Vorteil des Vorätzens ist die kürzere Behandlungsdauer des bereits beschichteten Systems mit den aggressiven Ätzmitteln (z.B. HF, KOH). Dadurch können die Schäden an den Lipidmolekülen und an der Lipidschicht minimiert werden.

Ein weiterer Prozeß beinhaltet die Einführung einer SH-Gruppe in eine polare Kopfgruppe des TEL, und chemische Kopplung der Lipide an ein goldbeschichtetes Substrat, das bereits porös ist, dessen Poren aber mit der dünnen Goldschicht überspannt sind. Zur Filmbildung auf der Langmuir Filmwaage werden dem TEL kristallisierbare Chelatorlipide beigemischt und durch Erhöhung der Kompression des Films Domänen erzeugt, deren Größe und Gestalt von Temperatur, Filmdruck und Kompressionsgeschwindigkeit abhängen. In die Subphase wird ein bifunktionelles wasserlösliches Molekül eingebracht, das durch Nickelionen aus der Subphase über Chelatbrücken an die Chelator-Domänen fixiert wird. Nach Transfer auf die Goldoberfläche werden die Chelatorlipid-Domänen gezielt durch Zugabe eines stärkeren Komplexbildners aus dem Membranverband herausgelöst. Nun wird in den entstandenen Membranlöchern die dünne Goldschicht durch Komplexierung aufgelöst, wodurch im Bereich der Mikroporen des Substrats Nanoporen in der Goldschicht entstehen, die mit Funktionsmolekülen gefüllt werden können.

Das Überspannen von Poren mit einer Lipidschicht ermöglicht nicht nur Einsatz solcher Systeme bei Separations- und Transportvorgängen (Dialyse, Umkehrosmose etc.), sondern auch im Bereich von Piezo- und Pyroelektrik, Mikrokondensatortechnik, nicht-linearer Optik und Biosensorik. Die Kapazität einer stabilen Black-Lipid-Membran aus TEL ist bei einer Porengröße von 0,8 mm 0,744 $\mu F/cm^2$.

Nachteile aller bisher verwendeten Systeme ist ihre geringe mechanische, thermische und chemische Stabilität. Bindet man dagegen Tetraetherlipidmembranen kovalent an einer Trägeroberfläche, so ist die gesamte Membran fixiert, ohne ihre wesentlichen membranspezifischen Eigenschaften zu verlieren. Derartig stabile Systeme sind bisher nur mit nicht-natürlichen oder anorganischen Substanzen gelungen (z.B. gamma-$Al_2O_3$-Membranen oder Silikagele). Genereller Wunsch der Technik ist jedoch, ein "molecular sieving" durch stabile Polymermembranen zu erreichen.

Zur kovalenten Kopplung der Lipide sind funktionelle Gruppen an der Matrixoberfläche nötig, die durch Auswahl des Substrats gewährleistet wird: Hydroxylfunktionen sind bei Glas und Silicium gegeben, Aminofunktionen sind bei aminomodifizertem Silikagel vorhanden. Zur Anbindung über Thiolgruppen wird eine dünne Goldschicht durch Aufdampfen oder "Sputtern" auf die Matrixoberfläche aufgebracht.

In das Lipidmolekül muß ebenfalls eine funktionelle Gruppe eingeführt werden. Die gebräuchlichste Methode ist die in Beispiel 4.3 beschriebene Oxidation mit Periodsäure zur Erzeugung von Carbonylfunktionen.

Die Kopplungsreaktion einer Lipid-Carbonylfunktion mit einer aminomodifizierten Siliciumoberfläche zeigt nachfolgendes Schema:

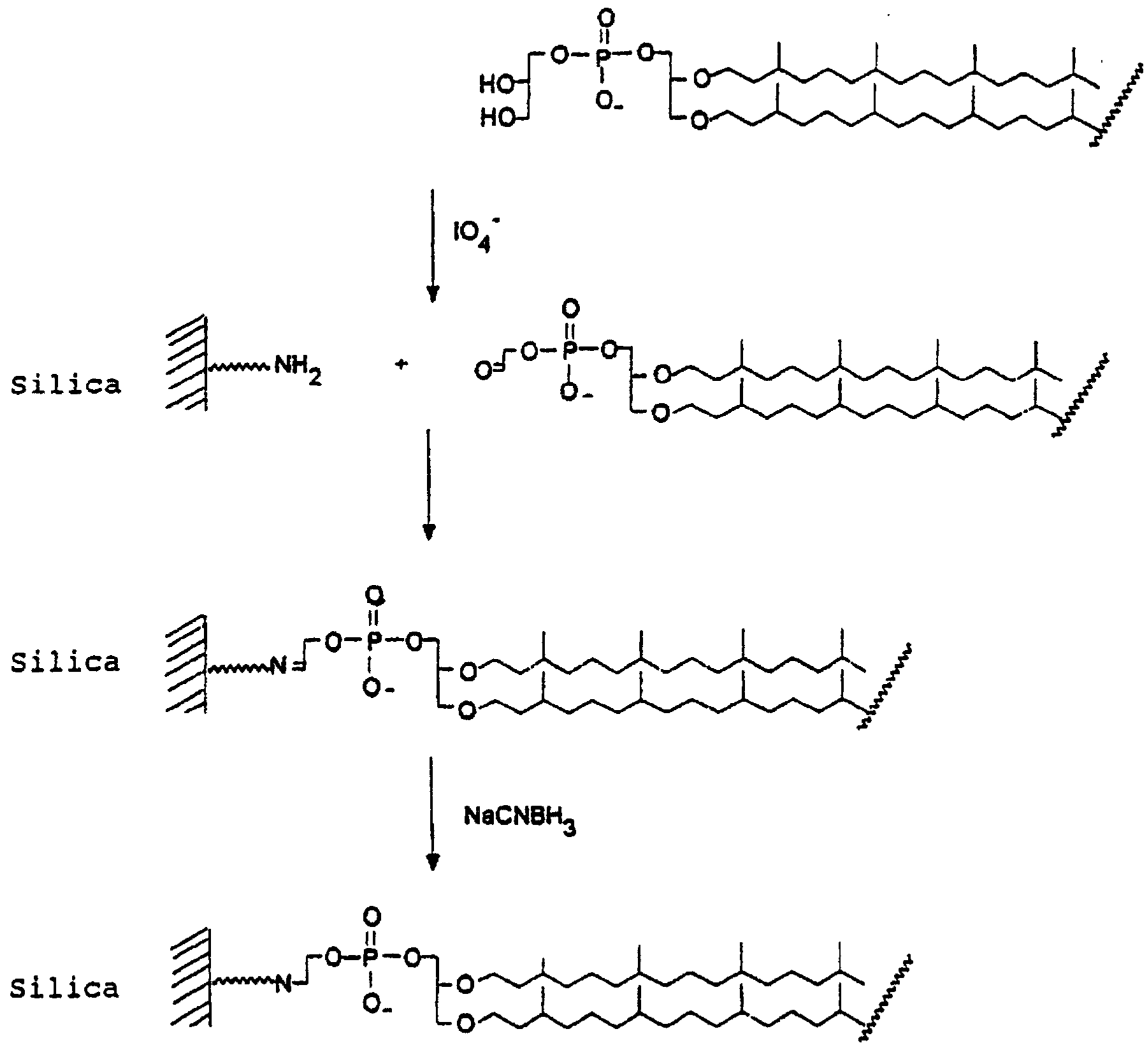


**Zitierte Literatur:**

Christiansen C., Freundt E.A. and Black F.T. (1975) Genome size and DNA base composition of Thermoplasma acidophilum. Int. Syst. Bacteriol. 25(2), pp. 99-101.

Dittmer J.C. and Lester R.L. (1964) A simple specific spray for the detection of phospholipids on thin-layer chromatograms. J. Lipid Res. 5, 126-127.

Karlsson K.A. and Martensson E. (1986) Studies on sphingosines. XIV. On the phytosphingosine content of the major human kidney glycolipids.
Biochim. Biophys. Acta 152, 230-231.

Langworthy T.A. and Pond J.L. (1986) Archaebacterial ether lipids and chemotaxonomy.
System. Appl. Microbiol. 7, 253-257.

New R.R.C. (1990) Characterization of liposomes. In: Liposomes a practical approach (New R.R.C., ed.), IRL Press/Oxford University Press, Oxford, New York, Tokyo, pp. 105-161.

Reio L. (1958) A method for the paper-chromatographic separation and identification of phenol derivatives, mould metablites and related compounds of biochemical interest, using a "reference system". J. Chromatography 1,

338-373.

Rouser G., Kritcherevsky G. and Yamamoto A. (1986) Column chromatographic associated procedures for Separation and determination of phosphatides and glycolipids. In: Marinetti G.V. (ed.) Lipid chromatographic analysis Vol. 3, pp. 374-380.

Weder H.-G. and Zumbühl O. (1984) The preparation of varably sized homogeneous liposomes for laboratory, clinical, and industrial use by controlled detergent dialysis. In: Liposome Technology (Gregoriadis G., ed.) Vol. 1, CRC Press, Boca Raton; pp. 79-107.

Weinstein J.N., Ralston E., Leserman L.D., Klausner R.D., Dragsten P.D., Kenkart P. and Blumenthal R. (1984) Selfquenching of carboxyfluorescein fluorescence: uses in studying lioposome stability and liposome-cell interaction. In: Liposome Technology (Gregoriadis G., ed.) Vol. 3, CRC Press, Boca Raton, pp. 183-204.

**Patentansprüche**

**1.** Tetraetherlipid (TEL) mit der allgemeinen Formel

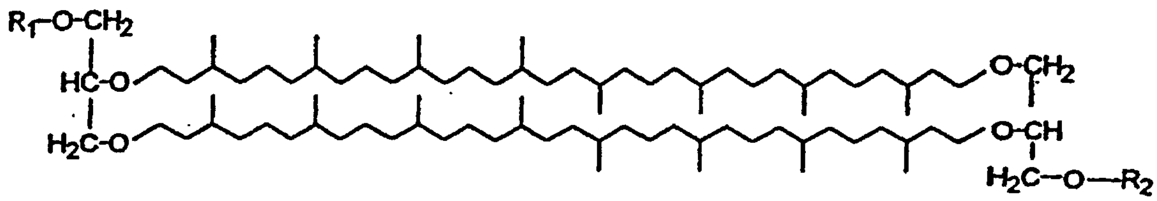

**dadurch gekennzeichnet, daß** $R_1$ aus der die Reste $R_1A$-$R_1J$ umfassenden Gruppe ausgewählt ist, in der

$R_1A$:

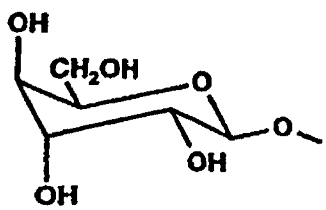

$R_1B$:

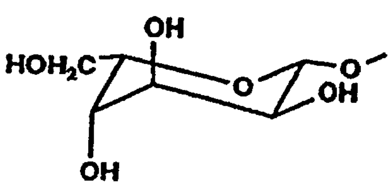

$R_1C$:

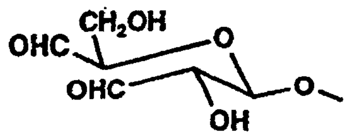

$R_1D$:

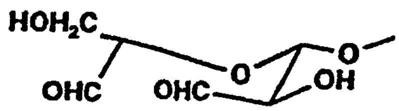

$R_1E$:

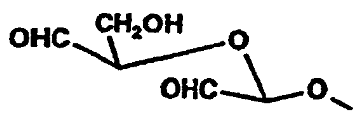

$R_1F$:

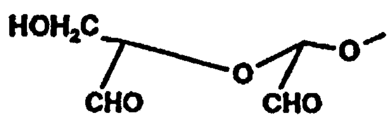

$R_1G$:

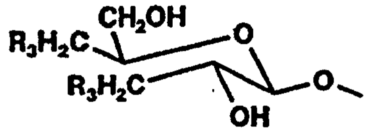

$R_1H$:

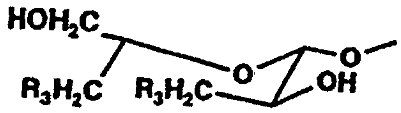

$R_1I$:

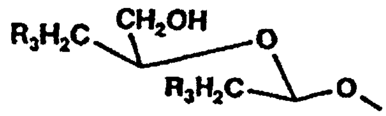

$R_1J$:

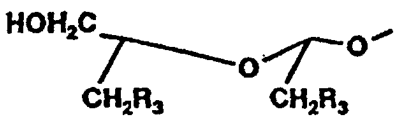

bedeutet und wobei $R_2$ aus der die Reste $R_2K$, $R_2L$, $R_2M$, und Wasserstoff umfassenden Gruppe ausgewählt ist, worin

$R_2K$:

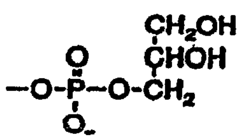

$R_2L$:

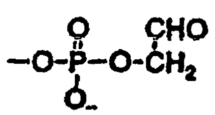

$R_2M$:

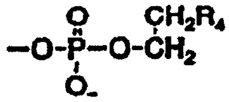

bedeutet, wobei $R_3$ und $R_4$ gleich oder voneinander verschieden sind und aus der Wasserstoff, Peptide, Proteine, Polyamine, Cholin, Polysaccharide, Polyethylenglykole und Thiole umfassenden Gruppe ausgewählt sind.

2. TEL nach Anspruch 1, **dadurch gekennzeichnet, daß** $R_3$ und/oder $R_4$ aus der LDL-Rezeptorenliganden und Antikörper oder Teile davon umfassenden Gruppe ausgewählt sind.

3. Verfahren zum Herstellen eines TEL nach Anspruch 1, in dem $R_1$ Gulose und $R_2$ ein Phosphoglycerinrest ist, umfassend die folgenden Schritte:

   (a) Kultivieren von Thermoplasma acidophilum (DM 10217),

   (b) Ernten der angezüchteten Mikroorganismen und Trocknen derselben,

   (c) Extraktion des getrockneten Zellmaterials mit organischen Lösungsmitteln,

   (d) Trennen der organischen Phase von festen oder wäßrigen Bestandteilen,

   (e) Verdampfen des Lösungsmittels,

   (f) Lösen des getrockneten Rückstands in organischem Lösungsmittel,

   (g) Chromatographie auf DEAE-Zelluloseacetat, Sammeln der TEL-haltigen Fraktionen,

   (h) Chromatographie auf Kieselgel

   (i) Verdampfen der organischen Phase.

4. Verfahren nach Anspruch 3, **dadurch gekennzeichnet, daß** der Verfahrensschritt (c) der Extraktion des getrockneten Zellmaterials mit organischen Lösungsmitteln mit einer rekurrierenden Soxhlet-Apparatur und einem Petrolether/Isopropanol-Gemisch durchgeführt wird.

5. Verwendung eines TEL nach einem der Ansprüche 1 oder 2 als Beschichtung von Substratoberflächen in Form einer planaren Membra

6. Verwendung des TEL nach einem der Ansprüche 1 oder 2 zum Herstellen von Trennmaterialien.

7. Verwendung des TEL nach einem der Ansprüche 1 oder 2 als Modellmembran.

8. Verwenden des TEL nach einem der Ansprüche 1 oder 2 zum Herstellen von reinen oder gemischten Liposomen.

9. Liposom, enthaltend 0,1 bis 100 Gew-% TEL nach einem der Ansprüche 1 oder 2, bezogen auf das Gesamtlipid.

10. Liposom nach Anspruch 9, enthaltend übliche Doppelschicht-bildende Phospholipide und 0,1 bis 20 Gew.-% TEL nach einem der Ansprüche 1 oder 2, bezogen auf das Gesamtlipid.

11. Liposom nach Anspruch 9, umfassend 1 bis 15 Gew.-% TEL, bevorzugt 5 bis 10 Gew.-% TEL, bezogen auf das Gesamtlipid.

**12.** Verwendung von Liposomen nach einem der Ansprüche 9 bis 11 als Wirkstoffträger zur Applikation kosmetischer und/oder pharmazeutischer Wirkstoffe.

**13.** Verwendung nach Anspruch 12, wobei der Wirkstoff im wässrigen Lumen des Liposoms enthalten ist.

**14.** Verwendung nach Anspruch 12, wobei der Wirkstoff in die Liposomenmembran integriert ist.

**15.** Verwendung nach Anspruch 12, wobei der Wirkstoff kovalent an das im Liposom enthaltene TEL gebunden ist.

**16.** Verwendung nach einem der Ansprüche 13 oder 14, wobei der Wirkstoff ein Cytostatikum ist.

**17.** Verwendung nach Anspruch 16, **dadurch gekennzeichnet, daß** das Cytostatikum Doxorubicin oder Daunorubicin ist.

**18.** Verwendung nach einem der Ansprüche 13 bis 15, wobei der Wirkstoff ein Immunsuppressivum ist.

**19.** Verwendung nach Anspruch 18, wobei das Immunsuppressivum Methytprednisolon oder eines seiner Derivate ist.

**20.** Verwendung nach einem der Ansprüche 13 bis 15, wobei der Wirkstoff ein Immunstimulanz oder Impfstoff ist.

**21.** Verwendung nach Anspruch 20, wobei der Wirkstoff ein Antikörper, ein Antikörperfragment oder ein Lectin ist.

**22.** Verwendung nach einem der Ansprüche 13 bis 15, wobei der Wirkstoff ein Hormon ist.

**23.** Verwendung nach Anspruch 22, wobei das Hormon Insulin oder ein Wachstumshormon ist.

**24.** Verwendung von Liposomen nach einem der Ansprüche 9 bis 11 als Transportvehikel für Nukleinsäuren in der Gentherapie.

**25.** Verwendung eines TEL nach Anspruch 1 oder 2 oder von Liposomen nach einem der Ansprüche 9 bis 11 zur Formulierung einer Hautcreme.

**26.** Verwendung von TEL nach einem der Ansprüche 1 bis 3 oder von Liposomen nach einem der Ansprüche 9 bis 11 zum Beschichten von Mikrotiterplatten.

## Claims

**1.** Tetraether lipid (TEL) having the general formula

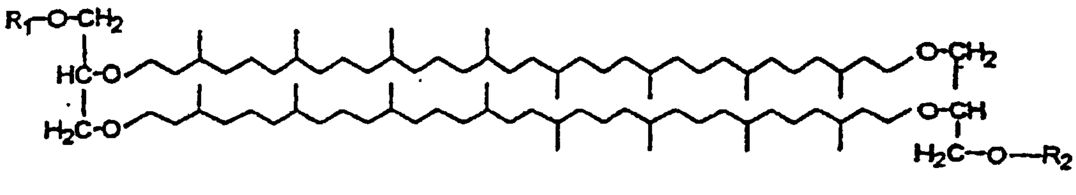

**characterized in that** $R_1$ is selected from the group comprising radicals $R_1A$-$R_1J$ in which

$R_1A$ denotes:

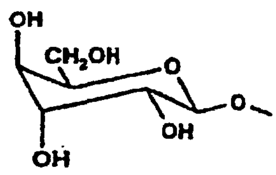

$R_1B$ denotes:

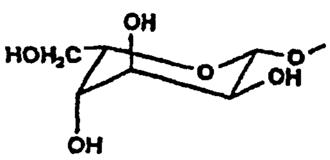

$R_1C$ denotes:

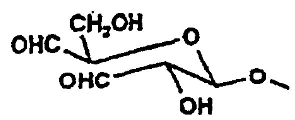

$R_1D$ denotes:

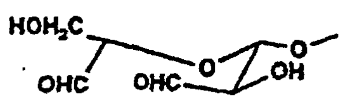

$R_1E$ denotes:

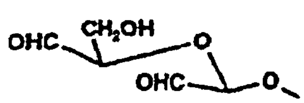

$R_1F$ denotes:

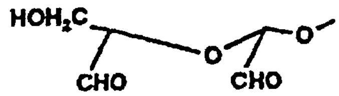

$R_1G$ denotes:

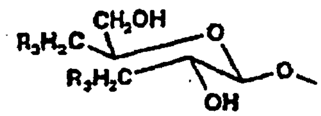

$R_1H$ denotes:

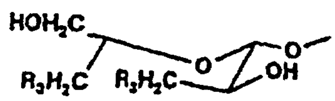

$R_1I$ denotes:

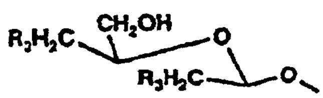

$R_1J$ denotes:

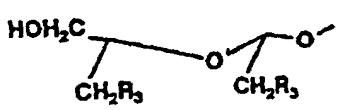

and where $R_2$ is selected from the group comprising the radicals $R_2K$, $R_2L$, $R_2M$ and hydrogen, in which

$R_2K$ denotes:

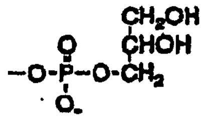

$R_2L$ denotes:

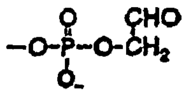

$R_2M$ denotes:

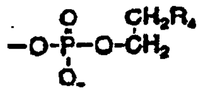

where $R_3$ and $R_4$ are identical or different from each other and are selected from the group comprising hydrogen, peptides, proteins, polyamines, choline, polysaccharide, polyethylene glycols and thiols.

2. TEL according to Claim 1, **characterized in that** $R_3$ and/or $R_4$ is/are selected from the group comprising LDL receptor ligands and antibodies or parts thereof.

3. Process for preparing a TEL according to Claim 1, in which $R_1$ is gulose and $R_2$ is a phosphoglycerol radical, which process comprises the following steps:

(a) culturing Thermoplasma acidophilum (DM 10217),
(b) harvesting the propagated microorganisms and drying them,
(c) extracting the dried cell material with organic solvents,
(d) separating the organic phase from solid or aqueous components,
(e) evaporating the solvent,
(f) dissolving the dried residue in the organic solvent,
(g) chromatography on DEAE-cellulose acetate, collecting the TEL-containing fractions,
(h) chromatography on silica gel
(i) evaporating the organic phase.

4. Process according to Claim 3, **characterized in that** the process step (c), involving the extraction of the dried cell material with organic solvents, is carried out using a continuous soxhlet apparatus and a petroleum ether/isopropanol mixture.

5. Use of a TEL according to Claim 1 or 2 as a coating of substrate surfaces in the form of a planar membrane.

6. Use of the TEL according to Claim 1 or 2 for preparing separating materials.

7. Use of the TEL according to Claim 1 or 2 as a model membrane.

8. Use of the TEL according to Claim 1 or 2 for preparing pure or mixed liposomes.

9. Liposome, containing from 0.1 to 100% by weight of TEL according to Claim 1 or 2, based on the total lipid.

**10.** Liposome according to Claim 9, containing customary double layer-forming phospholipids and from 0.1 to 20% by weight of TEL according to Claim 1 or 2, based on the total lipid.

**11.** Liposome according to Claim 9, comprising from 1 to 15% by weight of TEL, preferably from 5 to 10% by weight of TEL, based on the total lipid.

**12.** Use of liposomes according to one of Claims 9 to 11 as an active compound excipient for administering cosmetic and/or pharmaceutical active compounds.

**13.** Use according to Claim 12, where the active compound is contained in the aqueous lumen of the liposome.

**14.** Use according to Claim 12, where the active compound is integrated into the liposome membrane.

**15.** Use according to Claim 12, where the active compound is covalently bonded to the TEL which is contained in the liposome.

**16.** Use according to Claim 13 or 14, where the active compound is a cytostatic agent.

**17.** Use according to Claim 16, **characterized in that** the cytostatic agent is doxorubicin or daunorubicin.

**18.** Use according to one of Claims 13 to 15, where the active compound is an immunosuppressant.

**19.** Use according to Claim 18, where the immunosuppressant is methylprednisolone or one of its derivatives.

**20.** Use according to one of Claims 13 to 15, where the active compound is an immunostimulant or vaccine.

**21.** Use according to Claim 20, where the active compound is an antibody, an antibody fragment or a lectin.

**22.** Use according to one of Claims 13 to 15, where the active compound is a hormone.

**23.** Use according to Claim 22, where the hormone is insulin or a growth hormone.

**24.** Use of liposomes according to one of Claims 9 to 11 as transporter vehicles for nucleic acids in gene therapy.

**25.** Use of a TEL according to Claim 1 or 2, or of liposomes according to one of Claims 9 to 11, for formulating a skin cream.

**26.** Use of TEL according to one of Claims 1 to 3, or of liposomes according to one of Claims 9 to 11, for coating microtiter plates.

## Revendications

**1.** Tétraétherlipide (TEL) de formule générale

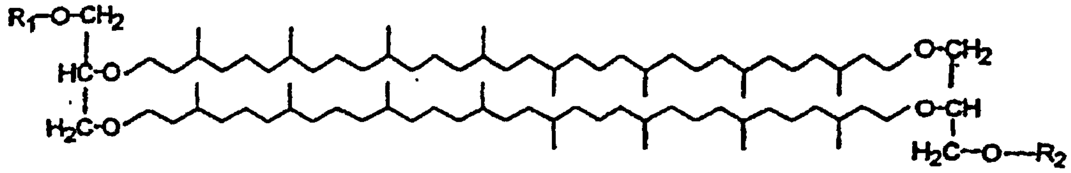

**caractérisé en ce que** $R_1$ est choisi dans le groupe comprenant les radicaux $R_1A$-$R_1J$, dans lequel

$R_1A$ représente :

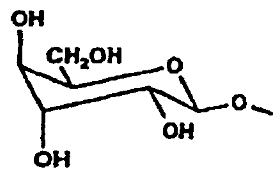

$R_1B$ représente :

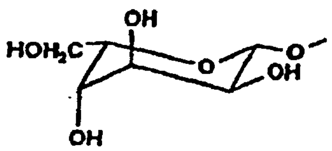

$R_1C$ représente :

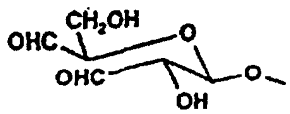

$R_1D$ représente :

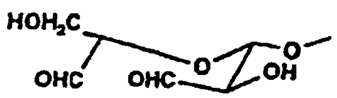

$R_1E$ représente :

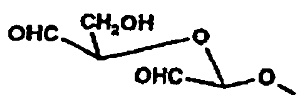

$R_1F$ représente :

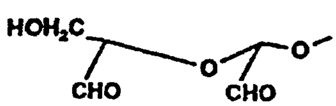

$R_1G$ représente :

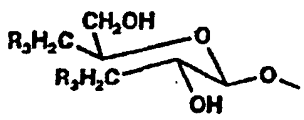

$R_1H$ représente :

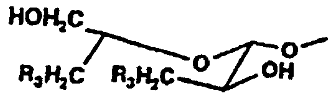

$R_1I$ représente :

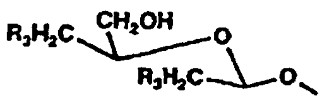

$R_1J$ représente :

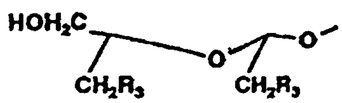

et $R_2$ est choisi dans le groupe comprenant les radicaux $R_2K$, $R_2L$, $R_2M$ et l'atome d'hydrogène, où $R_2K$ représente :

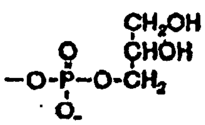

$R_2L$ représente :

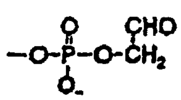

$R_2M$ représente :

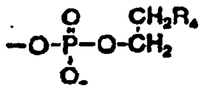

$R_3$ et $R_4$ étant identiques ou différents l'un de l'autre et étant choisis dans le groupe comprenant l'atome d'hydrogène, des peptides, des protéines, des polyamines, la choline, des polysaccharides, des polyéthylèneglycols et

des thiols.

**2.** TEL selon la revendication 1, **caractérisé en ce que** $R_3$ et/ou $R_4$ sont choisis dans le groupe comprenant des ligands de récepteurs de LDL (lipides basse densité) et des anticorps ou des parties de ceux-ci.

**3.** Procédé pour la préparation d'un TEL selon la revendication 1, dans lequel $R_1$ est le gulose et $R_2$ est un reste de phosphoglycérol, comprenant les étapes suivantes :

(a) culture de *Thermoplasma acidophilum* (DM 10217),
(b) récolte des micro-organismes cultivés et séchage de ceux-ci,
(c) extraction par des solvants organiques du matériel cellulaire séché,
(d) séparation de la phase organique d'avec les composants solides ou aqueux,
(e) évaporation du solvant,
(f) dissolution du résidu séché dans un solvant organique,
(g) chromatographie sur DEAE-acétate de cellulose, collecte des fractions contenant le TEL,
(h) chromatographie sur gel de silice
(i) évaporation de la phase organique.

**4.** Procédé selon la revendication 3, **caractérisé en ce que** l'étape (c) du procédé, de l'extraction par des solvants organiques du matériel cellulaire séché, est effectuée à l'aide d'un appareil de Soxhlet de récurrence et d'un mélange d'éther de pétrole et d'isopropanol.

**5.** Utilisation d'un TEL selon la revendication 1 ou 2, en tant que revêtement de surfaces de support sous forme d'une membrane plane.

**6.** Utilisation du TEL selon la revendication 1 ou 2, pour la préparation de matériaux de séparation.

**7.** Utilisation du TEL selon la revendication 1 ou 2, en tant que membrane modèle.

**8.** Utilisation du TEL selon la revendication 1 ou 2, pour la préparation de liposomes purs ou mélangés.

**9.** Liposome contenant de 0,1 à 100 % en poids de TEL selon la revendication 1 ou 2, par rapport au lipide total.

**10.** Liposome selon la revendication 9, contenant des phospholipides usuels constituant une double couche et de 0,1 à 20 % en poids de TEL selon la revendication 1 ou 2, par rapport au lipide total.

**11.** Liposome selon la revendication 9, comprenant de 1 à 15 % en poids de TEL, de préférence, de 5 à 10 % en poids de TEL, par rapport au lipide total.

**12.** Utilisation de liposomes selon l'une quelconque des revendications 9 à 11, en tant que vecteur de substance active pour l'administration de substances actives cosmétiques et/ou pharmaceutiques.

**13.** Utilisation selon la revendication 12, dans laquelle la substance active est contenue dans la lumière aqueuse du liposome.

**14.** Utilisation selon la revendication 12, dans laquelle la substance active est intégrée dans la membrane du liposome.

**15.** Utilisation selon la revendication 12, dans laquelle la substance active est liée par covalence au TEL contenu dans le liposome.

**16.** Utilisation selon la revendication 13 ou 14, dans laquelle la substance active est un agent cytostatique.

**17.** Utilisation selon la revendication 16, **caractérisée en ce que** l'agent cytostatique est la doxorubicine ou la daunorubicine.

**18.** Utilisation selon l'une quelconque des revendications 13 à 15, dans laquelle la substance active est un immunosuppresseur.

**19.** Utilisation selon la revendication 18, dans laquelle 1'immunosuppresseur est la méthylprednisolone ou un de ses dérivés.

**20.** Utilisation selon l'une quelconque des revendications 13 à 15, dans laquelle la substance active est un immunostimulant ou un vaccin.

**21.** Utilisation selon la revendication 20, dans laquelle la substance active est un anticorps, un fragment d'anticorps ou une lectine.

**22.** Utilisation selon l'une quelconque des revendications 13 à 15, dans laquelle la substance active est une hormone.

**23.** Utilisation selon la revendication 22, dans laquelle l'hormone est l'insuline ou une hormone de croissance.

**24.** Utilisation de liposomes selon l'une quelconque des revendications 9 à 11, en tant que véhicule de transport d'acides nucléiques dans la thérapie génique.

**25.** Utilisation d'un TEL selon la revendication 1 ou 2, ou de liposomes selon l'une quelconque des revendications 9 à 11, pour la formulation d'une crème pour la peau.

**26.** Utilisation d'un TEL selon l'une quelconque des revendications 1 à 3, ou de liposomes selon l'une quelconque des revendications 9 à 11, pour le tapissage de plaques de microtitrage.

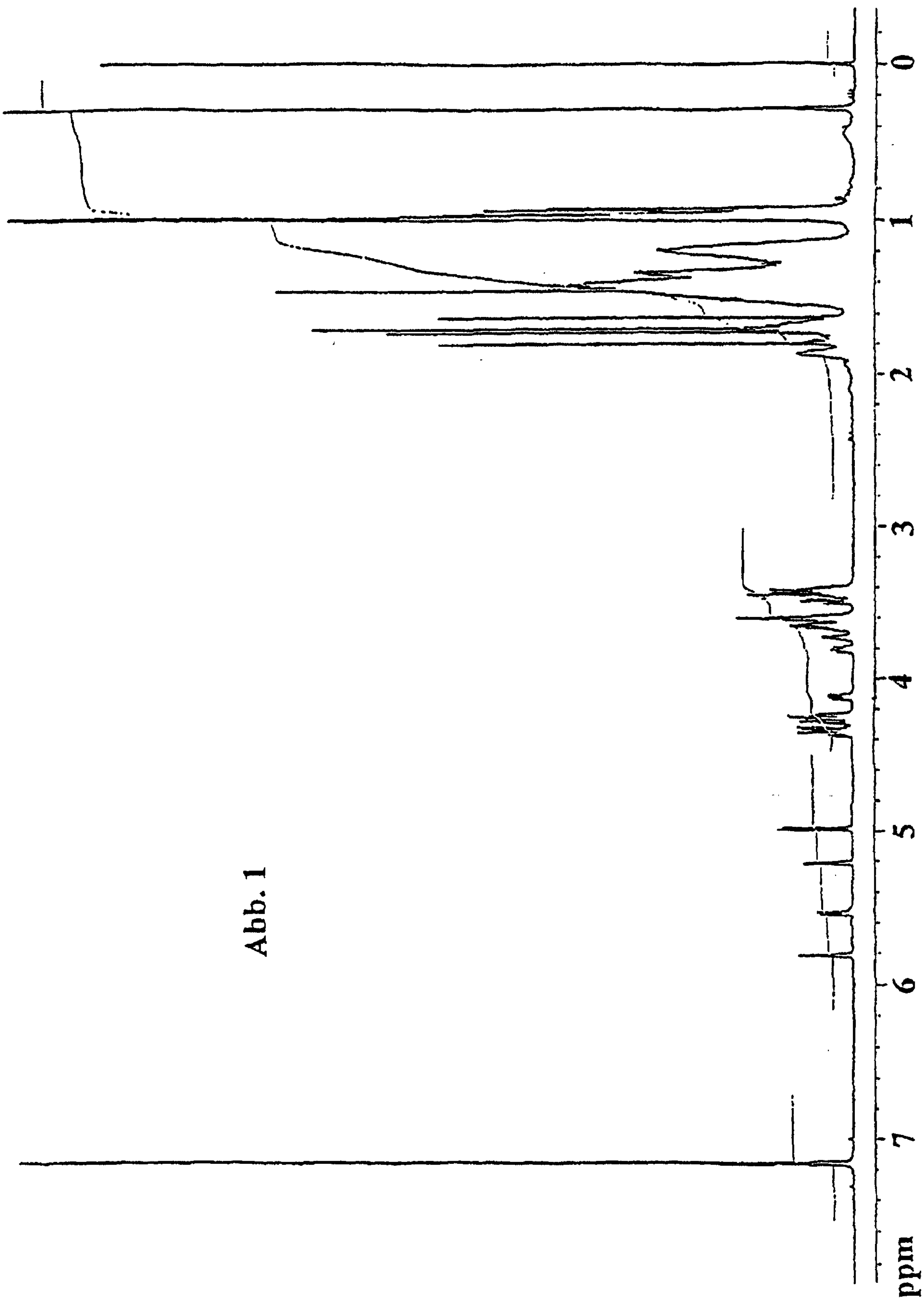


Abb. 1

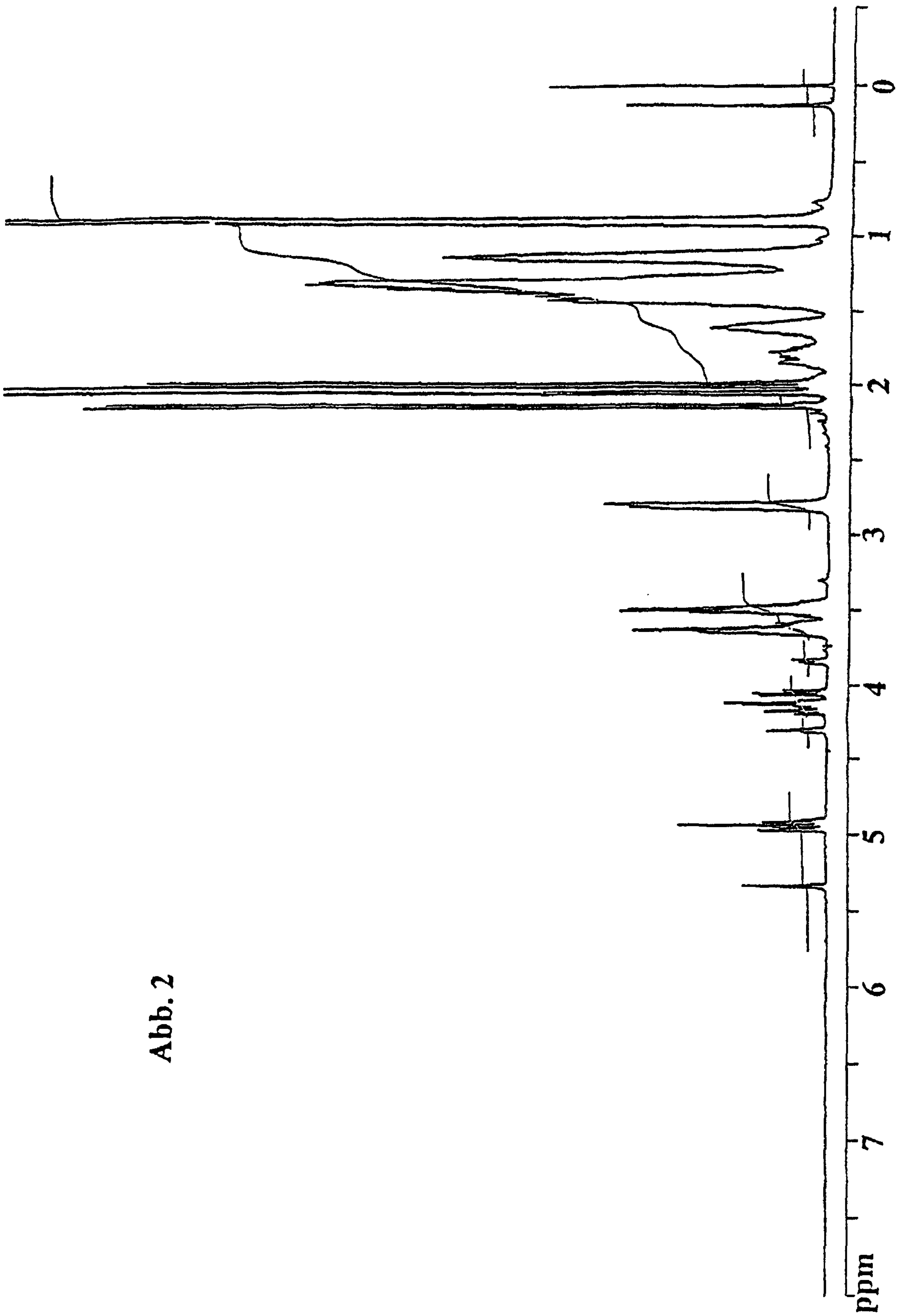


Abb. 2

Abb. 3a

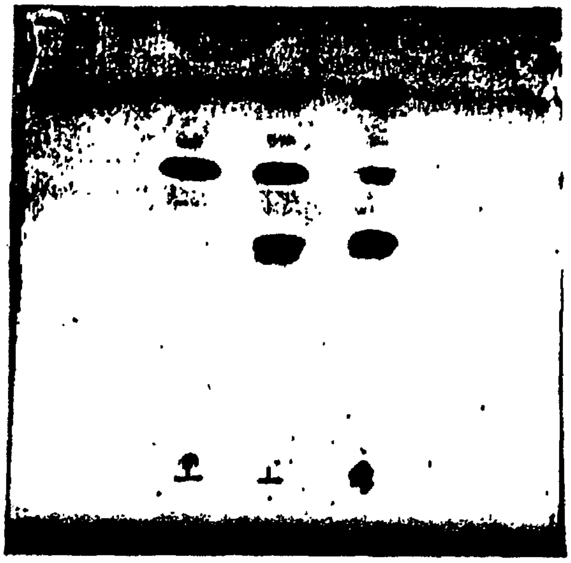

Abb. 3b

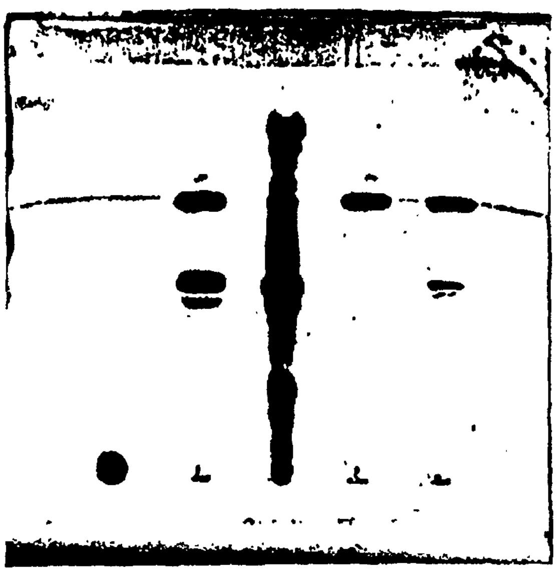

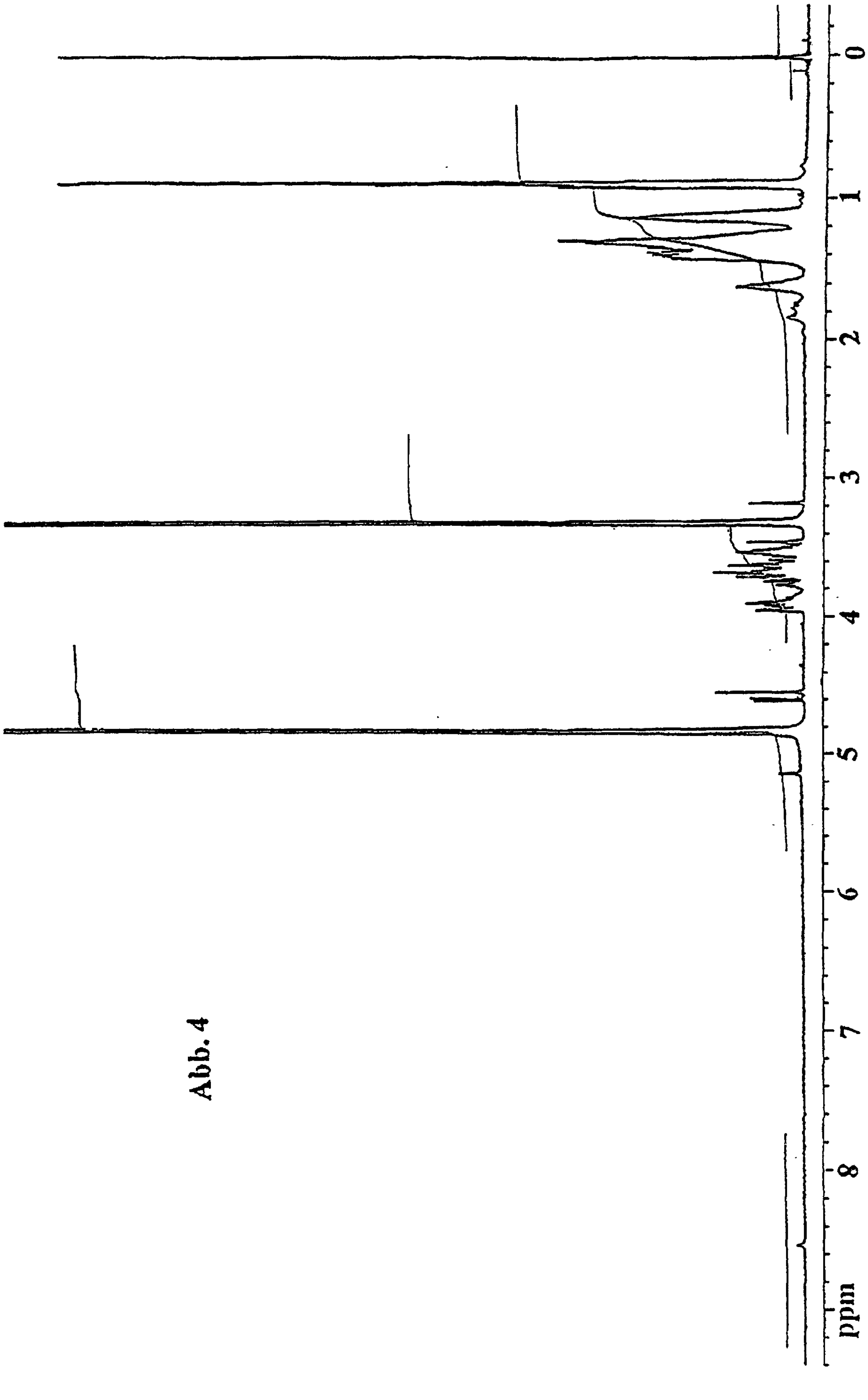


Abb. 4

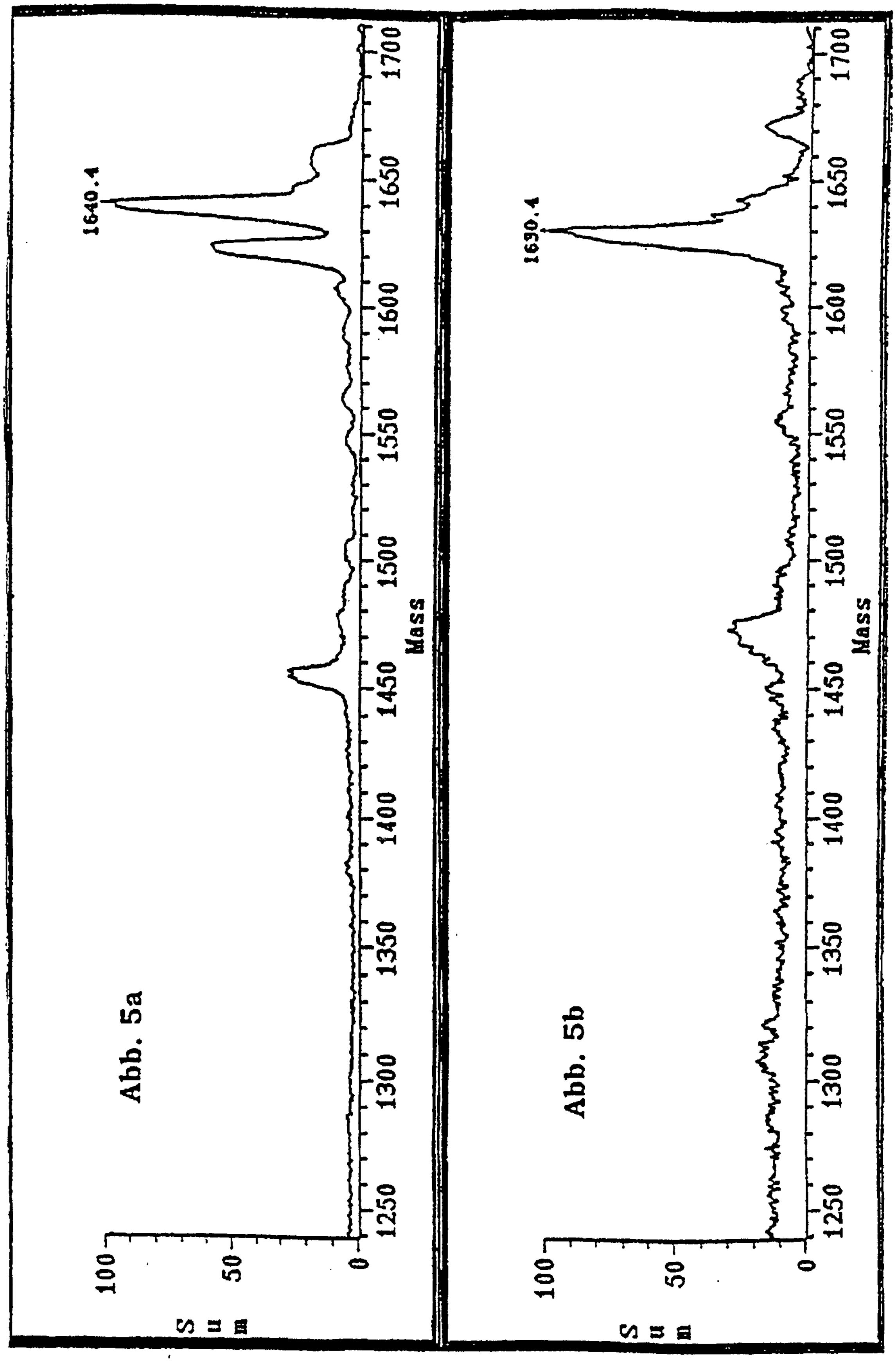
Abb. 5a
1640.4
Abb. 5b
1630.4
Mass
Sum
100
50
0
1250
1300
1350
1400
1450
1500
1550
1600
1650
1700

Abb. 6

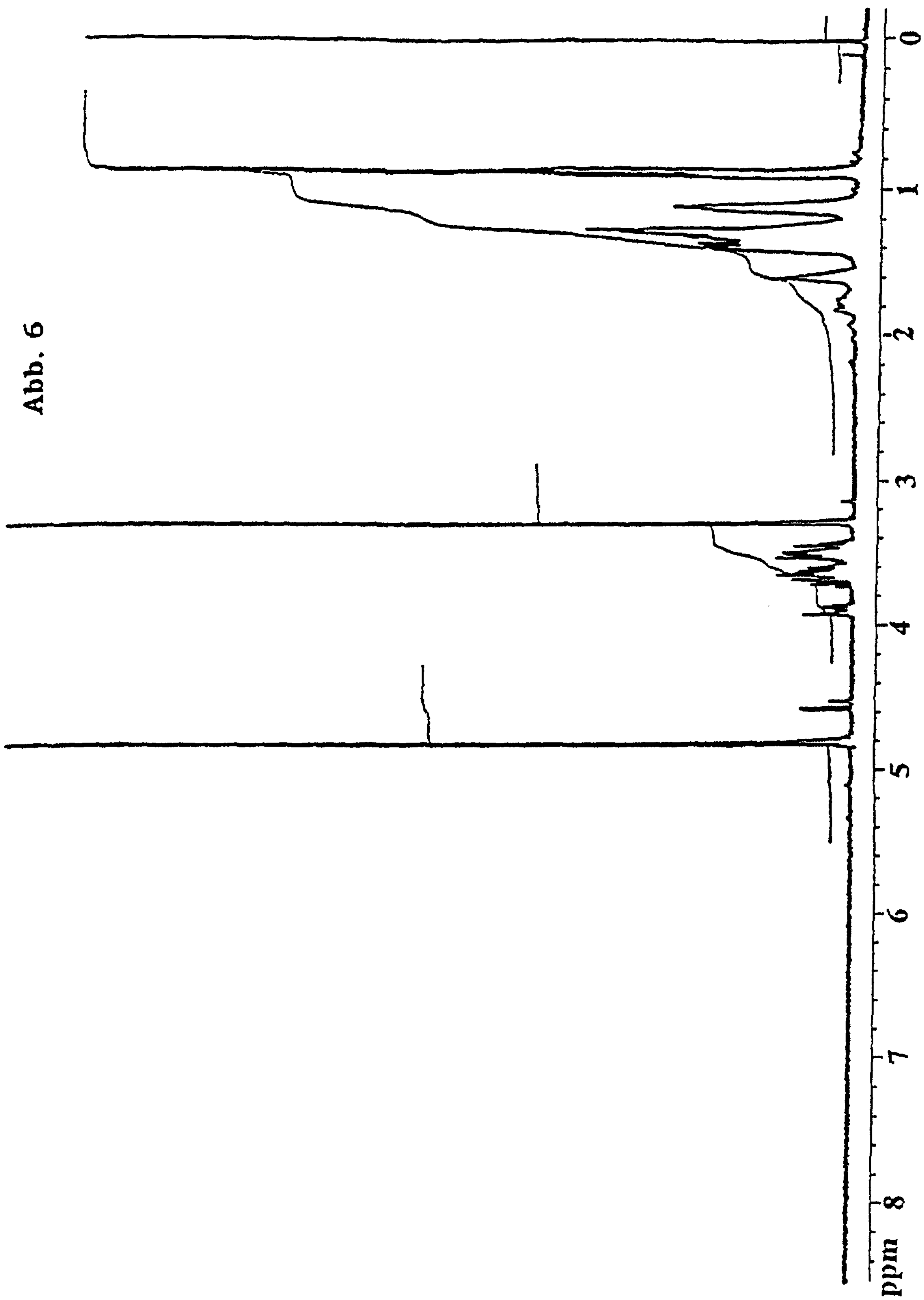

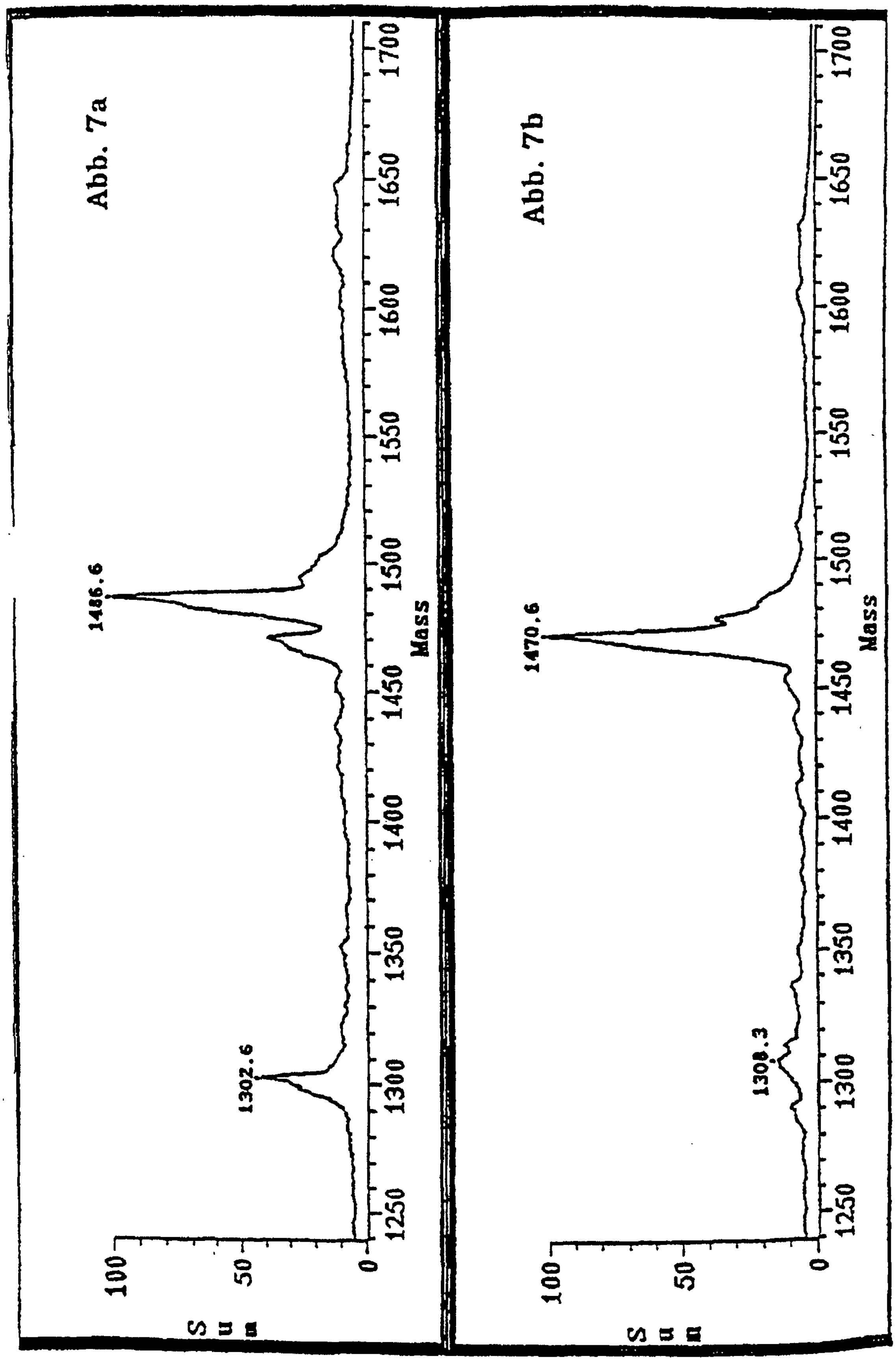
Abb. 7a
1486.6
1302.6
Mass
Sum
100
50
0
1250
1300
1350
1400
1450
1500
1550
1600
1650
1700
Abb. 7b
1470.6
1308.3
Mass
Sum
100
50
0
1250
1300
1350
1400
1450
1500
1550
1600
1650
1700

**Abb. 8**

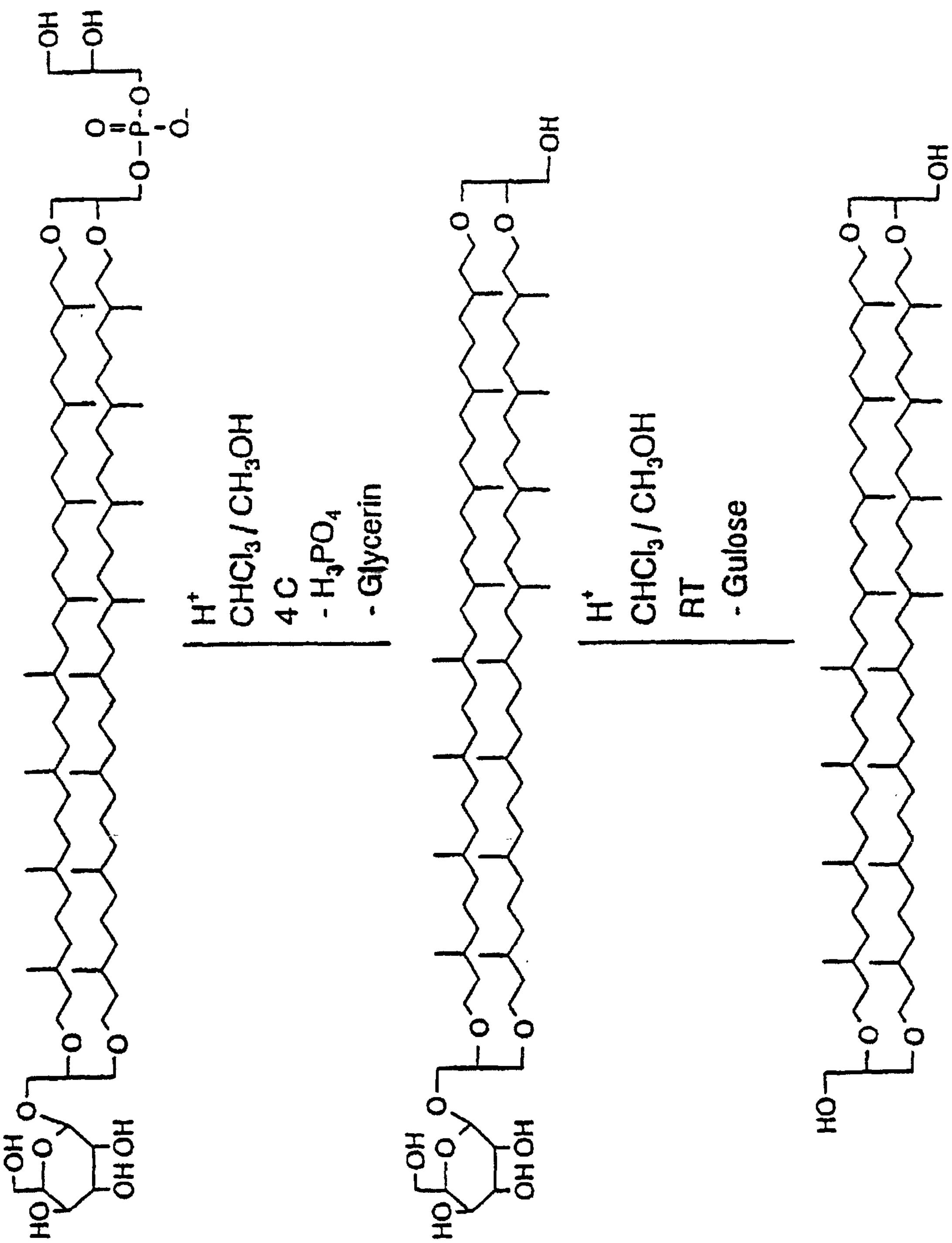

# Abb. 9a

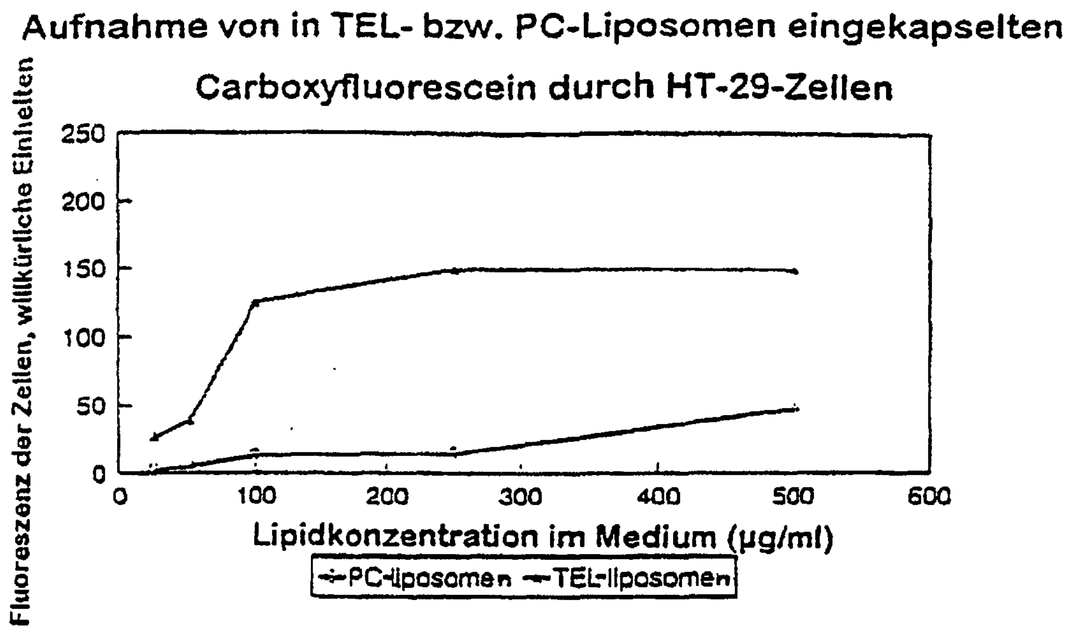
Aufnahme von in TEL- bzw. PC-Liposomen eingekapselten
Carboxyfluorescein durch HT-29-Zellen
Fluoreszenz der Zellen, willkürliche Einheiten
250
200
150
100
50
0
0
100
200
300
400
500
600
Lipidkonzentration im Medium (µg/ml)
PC-liposomen
TEL-liposomen

# Abb. 9b

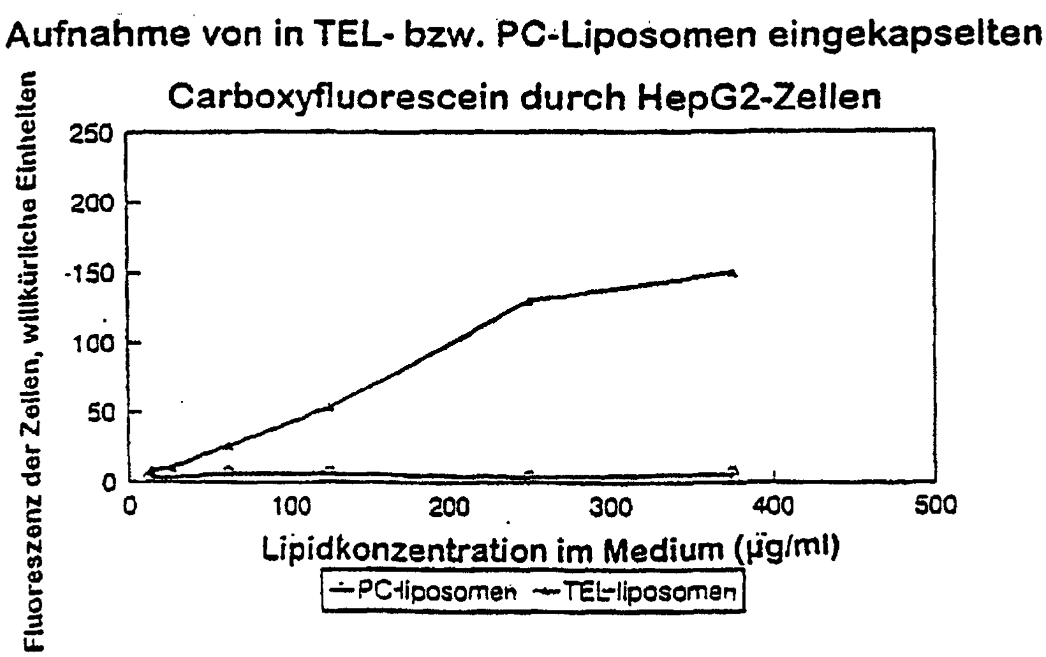
Aufnahme von in TEL- bzw. PC-Liposomen eingekapselten
Carboxyfluorescein durch HepG2-Zellen
Fluoreszenz der Zellen, willkürliche Einheiten
250
200
150
100
50
0
0
100
200
300
400
500
Lipidkonzentration im Medium (µg/ml)
PC-liposomen
TEL-liposomen

## Abb. 9c

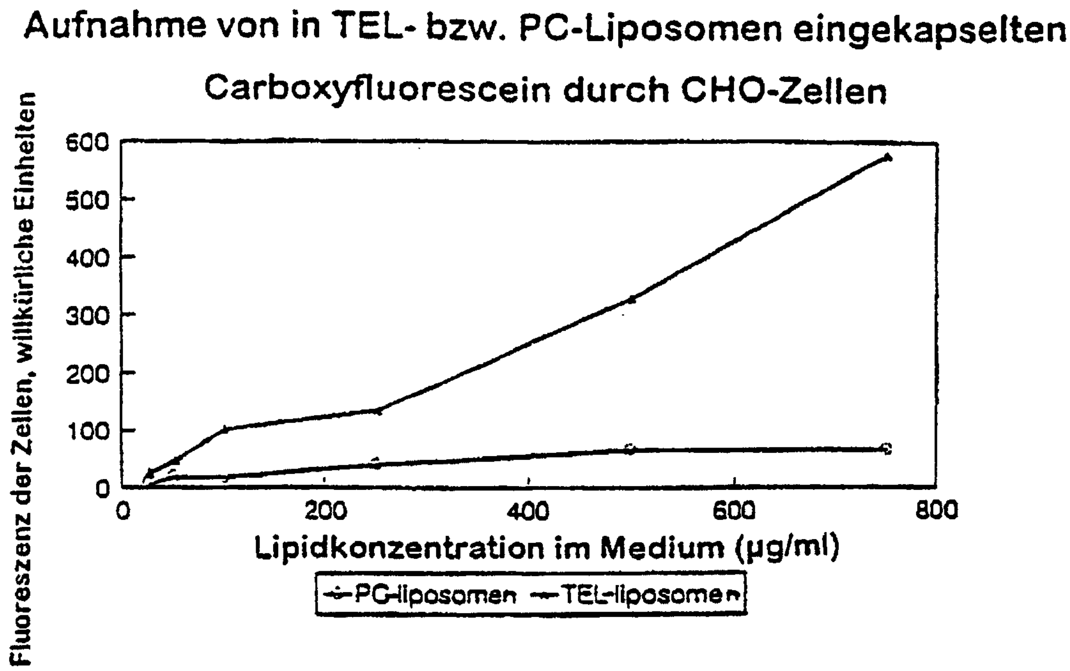
Aufnahme von in TEL- bzw. PC-Liposomen eingekapselten
Carboxyfluorescein durch CHO-Zellen
Fluoreszenz der Zellen, willkürliche Einheiten
600
500
400
300
200
100
0
0
200
400
600
800
Lipidkonzentration im Medium (µg/ml)
PC-liposomen
TEL-liposomen

## Abb. 9d

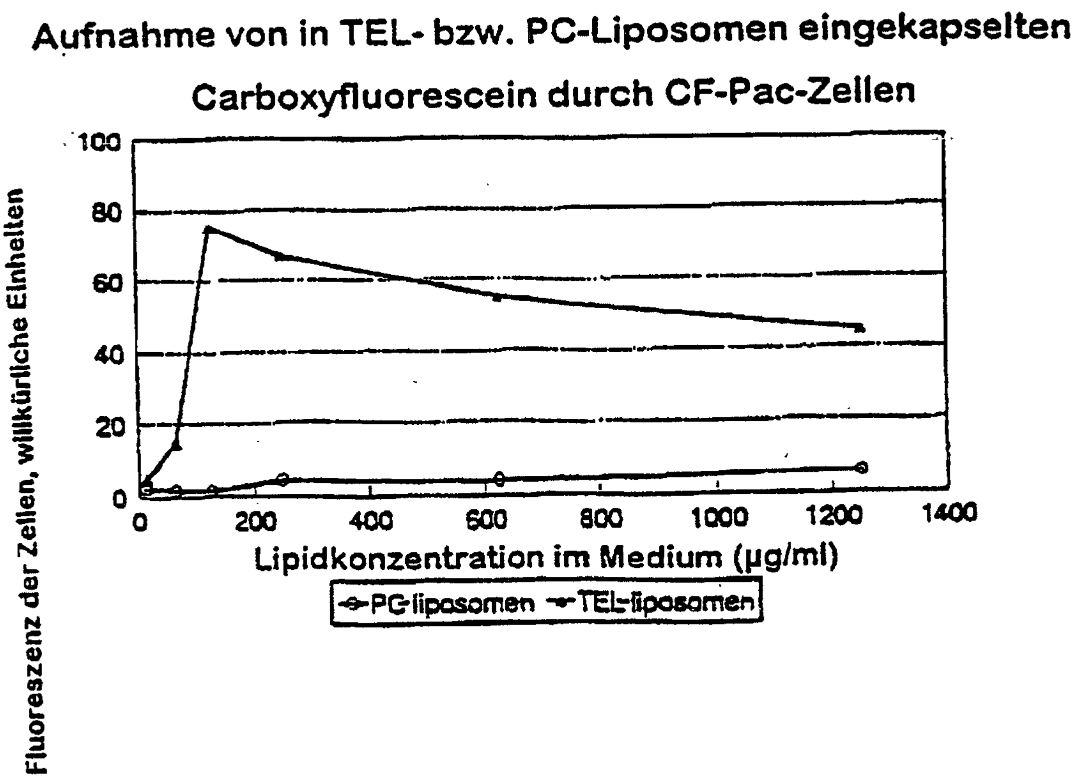
Aufnahme von in TEL- bzw. PC-Liposomen eingekapselten
Carboxyfluorescein durch CF-Pac-Zellen
Fluoreszenz der Zellen, willkürliche Einheiten
100
80
60
40
20
0
0
200
400
600
800
1000
1200
1400
Lipidkonzentration im Medium (µg/ml)
PC-liposomen
TEL-liposomen